(19) Europäisches Patentamt / European Patent Office / Office européen des brevets

(11) **EP 4 628 504 A2**

(12) **EUROPEAN PATENT APPLICATION**

(43) Date of publication:
08.10.2025 Bulletin 2025/41

(21) Application number: 25175873.6

(22) Date of filing: 04.04.2023

(51) International Patent Classification (IPC):
**C07K 14/715** (2006.01)

(52) Cooperative Patent Classification (CPC):
**A61P 35/00; A61K 40/11; A61K 40/30; A61K 40/31; A61K 40/4255; C07K 14/5443; C07K 14/7051; C07K 14/70517; C07K 14/70578; C07K 14/7155;** C07K 2319/00; C07K 2319/03; C07K 2319/33

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR

(30) Priority: 04.04.2022 GB 202204927
01.02.2023 US 202363482752 P

(62) Document number(s) of the earlier application(s) in accordance with Art. 76 EPC:
**23721778.1 / 4 504 344**

(71) Applicant: **Gammadelta Therapeutics Ltd London W2 6BD (GB)**

(72) Inventors:
• **KOVACS, Istvan**
London W2 6BD (GB)
• **SIMOES, Andre Goncalo do Espirito Santo**
London W2 6BD (GB)
• **ILLINGWORTH, Sam**
London W2 6BD (GB)

• **NUSSBAUMER, Oliver**
London W2 6BD (GB)
• **EDWARDS, Sarah**
London W2 6BD (GB)

(74) Representative: **Sagittarius IP Marlow International Parkway Marlow SL7 1YL (GB)**

Remarks:
•The complete document including Reference Table(s) and the Sequence Listing(s) can be downloaded from the EPO website
•This application was filed on 12.05.2025 as a divisional application to the application mentioned under INID code 62.
•Claims filed after the date of receipt of the divisional application (Rule 68(4) EPC).

(54) **CELLS EXPRESSING AN ANTI-MESOTHELIN CAR**

(57) Some aspects of the present disclosure are directed to engineered innate lymphoid cells comprising a heterologous targeting construct specific for mesothelin and uses thereof. Some aspects of the present disclosure are directed to polynucleotides encoding a chimeric antigen receptor (CAR) that specifically binds human mesothelin, host cells comprising the polynucleotide, and methods of treating a disease or condition in a subject using the same.

**Solid Tumor Targeting**
Driven solely by CAR

FIG. 1A

**EP 4 628 504 A2**

**Description**

**CROSS REFERENCE TO RELATED APPLICATIONS**

This PCT application claims the priority benefit of G.B. Application No. GB 2204927.4, filed on April 4, 2022, and U.S. Provisional Application No. 63/482,752, filed February 1, 2023, each of which is incorporated herein by reference in its entirety.

**REFERENCE TO SEQUENCE LISTING SUBMITTED**

**ELECTRONICALLY VIA EFS-WEB**

The content of the electronically submitted sequence listing in ASCII text file (Name: 3817_165PC01_Seqlisting_ST26; Size: 38,145 bytes; and Date of Creation: April 3, 2023), filed with the application, is incorporated herein by reference in its entirety.

**FIELD OF THE DISCLOSURE**

The invention relates to an engineered innate lymphoid cell comprising a heterologous targeting construct specific for mesothelin. The present invention also relates to pharmaceutical compositions comprising said engineered innate lymphoid cell, methods of treatment using said engineered innate lymphoid cell or pharmaceutical composition, and said engineered innate lymphoid cell or pharmaceutical composition for use in therapy. The present invention also includes a method of making the engineered innate lymphoid cell, and an isolated cell population, the population comprising a plurality of said engineered innate lymphoid cells.

**BACKGROUND**

Mesothelin, encoded by the *MSLN* gene located on human chromosome 16p.13.3, is a 40 kDa glycophosphatidylinositol (GPI) linked cell surface glycoprotein expressed by mesothelial cells. While mesothelin is present on the cell surface of normal mesothelial cells, it is also overexpressed in several human tumours including ovarian, cervical, uterine, gastric, pancreatic, and lung adenocarcinomas. Mesothelin is therefore an attractive candidate for the development of targeted cancer therapies.
There is therefore a need for therapies capable of targeting mesothelin expressing cells.

**SUMMARY OF THE DISCLOSURE**

According to the first aspect of the invention, there is provided an engineered innate lymphoid cell comprising a heterologous targeting construct specific for mesothelin.
According to a further aspect of the invention there is provided a pharmaceutical composition comprising the engineered innate lymphoid cell as described herein, in combination with one or more pharmaceutically or physiologically acceptable carriers, diluents, or excipients.
According to a further aspect of the invention there is provided a method of treating a patient in need thereof using the engineered innate lymphoid cell or the pharmaceutical composition as described herein.
According to a further aspect of the invention there is provided use of the engineered innate lymphoid cell or the pharmaceutical composition as described herein for treating a condition in a subject.
According to a further aspect of the invention there is provided the engineered innate lymphoid cell or the pharmaceutical composition as described herein for use in therapy.
According to a further aspect of the invention there is provided a method of making the engineered innate lymphoid cell as described herein.
According to a further aspect of the invention there is provided an isolated cell population, the population comprising a plurality of engineered innate lymphoid cells as described herein.
Some aspects of the present disclosure are directed to a polynucleotide comprising a nucleotide sequence encoding a chimeric antigen receptor (CAR), wherein the CAR comprises an antigen-binding domain that specifically binds human mesothelin, wherein the antigen-binding domain comprises a variable heavy (VH) domain and a variable light (VL) domain, wherein the VH comprises a VH-complementarity determining region 3 (CDR3) comprising the amino acid sequence set forth in SEQ ID NO: 3. In some aspects, the CAR further comprises a VH-CDR2 comprising the amino acid sequence set forth in SEQ ID NO: 2. In some aspects, the CAR further comprises a VH-CDR3 comprising the amino acid sequence set forth in SEQ ID NO: 3. In some aspects, the CAR further comprises a VL-CDR1 comprising the amino acid sequence set forth in SEQ ID NO: 4. In some aspects, the CAR further comprises a VL-CDR2 comprising the amino acid sequence set forth in SEQ ID NO: 5. In some aspects, the CAR further comprises a VL-CDR3 comprising the amino acid sequence set forth in SEQ ID NO: 6.

In some aspects, the CAR comprises a VH-CDR1 comprising the amino acid sequence set forth in SEQ ID NO: 1; a VH-CDR2 comprising the amino acid sequence set forth in SEQ ID NO: 2; a VH-CDR3 comprising the amino acid sequence set forth in SEQ ID NO: 3; a VL-CDR1 comprising the amino acid sequence set forth in SEQ ID NO: 4; a VL-CDR2 comprising the amino acid sequence set forth in SEQ ID NO: 5; and a VL-CDR3 comprising the amino acid sequence set forth in SEQ ID NO: 6.

In some aspects, the VH comprises an amino acid sequence having at least about 75%, at least about 80%, at least about 85%, at least about 90%, at least about 95%, at least about 96%, at least about 97%, at least about 98%, or at least about 99% sequence identity with the amino acid sequence set forth in SEQ ID NO: 7. In some aspects, the VH comprises the amino acid sequence set forth in SEQ ID NO: 7.

In some aspects, the VL comprises an amino acid sequence having at least about 75%, at least about 80%, at least about 85%, at least about 90%, at least about 95%, at least about 96%, at least about 97%, at least about 98%, or at least about 99% sequence identity with the amino acid sequence set forth in SEQ ID NO: 8. In some aspects, the VL comprises the amino acid sequence set forth in SEQ ID NO: 8.

In some aspects, the VH comprises the amino acid sequence set forth in SEQ ID NO: 7, and wherein the VL comprises the amino acid sequence set forth in SEQ ID NO: 8.

In some aspects, the CAR further comprises (i) a hinge region, (ii) a transmembrane domain, (iii) a costimulatory domain, (iv) an intracellular signaling domain, or (v) any combination thereof. In some aspects, the CAR further comprises (i) a hinge region, (ii) a transmembrane domain, (iii) a costimulatory domain, and (iv) an intracellular signaling domain.

In some aspects, the hinge region comprises a hinge region derived from CD8, CD28, or an immunoglobulin. In some aspects, the immunoglobulin is selected from the group consisting of IgA1, IgA2, IgG1, IgG2, IgG3, IgG4, IgD, IgE, IgM, and any combination thereof. In some aspects, the hinge region is derived from a CD8 hinge region. In some aspects, the hinge region comprises an amino acid sequence having at least about 75%, at least about 80%, at least about 85%, at least about 90%, at least about 95%, at least about 96%, at least about 97%, at least about 98%, or at least about 99% sequence identity with the amino acid sequence set forth in SEQ ID NO: 9. In some aspects, the hinge region comprises the amino acid sequence set forth in SEQ ID NO: 9.

In some aspects, the transmembrane domain comprises a transmembrane domain derived from CD8, KIRDS2, OX40, CD2, CD4, CD28, CD45, PD1, CD152, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2R beta, IL2R gamma, IL7R $\alpha$, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKG2D, NKG2C, CD19, or any combination thereof. In some aspects, the transmembrane domain is derived from a CD8 transmembrane domain. In some aspects, the transmembrane domain comprises an amino acid sequence having at least about 75%, at least about 80%, at least about 85%, at least about 90%, at least about 95%, at least about 96%, at least about 97%, at least about 98%, or at least about 99% sequence identity with the amino acid sequence set forth in SEQ ID NO: 10. In some aspects, the transmembrane domain comprises the amino acid sequence set forth in SEQ ID NO: 10.

In some aspects, the costimulatory domain comprises a costimulatory domain derived from a costimulatory domain of 4-1BB/CD137, interleukin-2 receptor (IL-2R), interleukin-12 receptor (IL-12R), IL-7, IL-21, IL-23, IL-15, CD2, CD3, CD4, CD7, CD8, CD27, CD28, CD30, CD40, ICOS, lymphocyte function-associated antigen-1 (LFA-1), LIGHT, NKG2C, OX40, DAP10, B7-H3, CD28 deleted for Lck binding (ICA), BTLA, GITR, HVEM, LFA-1, LIGHT, NKG2C, PD-1, TILR2, TILR4, TILR7, TILR9, Fc receptor gamma chain, Fc receptor $\varepsilon$ chain, a ligand that specifically binds with CD83, or any combination thereof. In some aspects, the costimulatory domain is derived from a 4-1BB costimulatory domain. In some aspects, the costimulatory domain comprises an amino acid sequence having at least about 75%, at least about 80%, at least about 85%, at least about 90%, at least about 95%, at least about 96%, at least about 97%, at least about 98%, or at least about 99% sequence identity with the amino acid sequence set forth in SEQ ID NO: 11. In some aspects, the costimulatory domain comprises the amino acid sequence set forth in SEQ ID NO: 11.

In some aspects, the intracellular signaling domain comprises a CD3 $\zeta$ activating domain, a CD3$\delta$ activating domain, a CD3$\varepsilon$ activating domain, a CD3$\eta$ activating domain, a CD79A activating domain, a DAP 12 activating domain, a FCER1G activating domain, a DAP10/CD28 activating domain, a ZAP70 activating domain, or any combination thereof. In some aspects, the intracellular signaling domain comprises a CD3 $\zeta$ activating domain. In some aspects, the intracellular signaling domain comprises an amino acid sequence having at least about 75%, at least about 80%, at least about 85%, at least about 90%, at least about 95%, at least about 96%, at least about 97%, at least about 98%, or at least about 99% sequence identity with the amino acid sequence set forth in SEQ ID NO: 12. In some aspects, the costimulatory domain comprises the amino acid sequence set forth in SEQ ID NO: 12.

In some aspects, the polynucleotide comprises a nucleotide sequence encoding a chimeric antigen receptor (CAR), wherein the CAR comprises (a) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; and (b)(i) a hinge region comprising an amino acid sequence having at least about 90%, at least about 95%, at least about 96%, at least about 98%, at least about 98%, or at least about 99% sequence identity to the sequence set forth in SEQ ID NO: 9; (b)(ii) a transmembrane domain comprising an amino acid sequence having at least about 90%, at least about 95%, at least about 96%, at least about 98%, at least about 98%, or at least about 99% sequence identity to the sequence set forth in SEQ ID NO: 10; (b)(iii) a costimulatory domain comprising an amino acid sequence having at least about 90%, at least about 95%, at least about 96%, at least about 98%, at least about 98%, or at least about 99% sequence identity to the sequence set forth in SEQ ID NO: 11; (b)(iv) an intracellular signaling domain comprising an amino acid sequence having at least about 90%, at least about 95%, at least about 96%, at least about 98%, at least about 98%, or at least about 99% sequence identity to the sequence set forth in SEQ ID NO: 12; or (b)(v) any combination of (b)(i) to (b)(iv).

In some aspects, the polynucleotide comprises a nucleotide sequence encoding a chimeric antigen receptor (CAR), wherein the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region comprising an amino acid sequence having at least about 90%, at least about 95%, at least about 96%, at least about 98%, at least about 98%, or at least about 99% sequence identity to the sequence set forth in SEQ ID NO: 9; (iii) a transmembrane domain comprising an amino acid sequence having at least about 90%, at least about 95%, at least about 96%, at least about 98%, at least about 98%, or at least about 99% sequence identity to the sequence set forth in SEQ ID NO: 10; (iv) a costimulatory domain comprising an amino acid sequence having at least about 90%, at least about 95%, at least about 96%, at least about 98%, at least about 98%, or at least about 99% sequence identity to the sequence set forth in SEQ ID NO: 11; and (v) an intracellular signaling domain comprising an amino acid sequence having at least about 90%, at least about 95%, at least about 96%, at least about 98%, at least about 98%, or at least about 99% sequence identity to the sequence set forth in SEQ ID NO: 12.

In some aspects, the polynucleotide comprises a nucleotide sequence encoding a chimeric antigen receptor (CAR), wherein the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region comprising an amino acid sequence having at least about 90%, at least about 95%, at least about 96%, at least about 98%, at least about 98%, or at least about 99% sequence identity to the sequence set forth in SEQ ID NO: 9; (iii) a transmembrane domain comprising an amino acid sequence having at least about 90%, at least about 95%, at least about 96%, at least about 98%, at least about 98%, or at least about 99% sequence identity to the sequence set forth in SEQ ID NO: 10; (iv) a costimulatory domain comprising an amino acid sequence having at least about 90%, at least about 95%, at least about 96%, at least about 98%, at least about 98%, or at least about 99% sequence identity to the sequence set forth in SEQ ID NO: 11; and (v) an intracellular signaling domain comprising an amino acid sequence having at least about 90%, at least about 95%, at least about 96%, at least about 98%, at least about 98%, or at least about 99% sequence identity to the sequence set forth in SEQ ID NO: 12.

In some aspects, the polynucleotide comprises a nucleotide sequence encoding a chimeric antigen receptor (CAR), wherein the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region comprising the amino acid sequence set forth in SEQ ID NO: 9; (iii) a transmembrane domain comprising the amino acid sequence set forth in SEQ ID NO: 10; (iv) a costimulatory domain comprising the amino acid sequence set forth in SEQ ID NO: 11; and (v) an intracellular signaling domain comprising the amino acid sequence set forth in SEQ ID NO: 12.

In some aspects, the polynucleotide further comprises a second nucleotide sequence encoding an armor protein. In some aspects, the armor protein comprises an IL-15Rβ polypeptide, an IL-15Rα polypeptide, an IL-2Rβ polypeptide, an IL-15 polypeptide, or any combination thereof.

In some aspects, the armor protein comprises an IL-2Rβ polypeptide. In some aspects, the armor protein comprises an amino acid sequence having at least about 70%, at least about 75%, at least about 80%, at least about 85%, at least about 90%, at least about 95%, at least about 96%, at least about 97%, at least about 98%, at least about 99% sequence identity to the amino acid sequence set forth in SEQ ID NO: 15. In some aspects, the armor protein comprises the amino acid sequence set forth in SEQ ID NO: 15.

In some aspects, the armor protein comprises an IL-2Rβ polypeptide, and wherein the armor protein does not comprise an IL-15Rα polypeptide or an IL-15 polypeptide.

In some aspects, the armor protein comprises (i) an IL-15Rα polypeptide or (ii) an IL-15Rα polypeptide tethered to an IL-15 polypeptide. In some aspects, the armor protein comprises an IL-15Rα polypeptide comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 17. In some aspects, the armor protein comprises an IL-15Rα polypeptide comprising the amino acid sequence set forth in SEQ ID NO: 17.

In some aspects, the armor protein comprises an IL-15 polypeptide. In some aspects, the armor protein comprises an IL-15 polypeptide comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 16. In some aspects, the armor protein comprises an II-15 polypeptide comprising the amino acid sequence set forth in SEQ ID NO: 16.

In some aspects, the armor protein comprises an IL-15Rα polypeptide tethered to an IL-15 polypeptide by a linker. In some aspects, the linker comprises a peptide bond. In some aspects, the linker comprises a Gly-Ser linker. In some aspects, the linker comprises an amino acid sequence selected from SEQ ID NOs: 20-25 or any combination thereof.

In some aspects, the armor protein comprises (i) an IL-2Rβ polypeptide; (ii) a cleavable linker; and (iii) an IL-15Rα polypeptide tethered to an IL-15 polypeptide. In some aspects, the cleavable linker comprises a P2A sequence. In some aspects, the cleavable linker comprises an amino acid sequence having at least about 80%, at least about 85%, at least about 90%, at least about 95%, at least about 96%, at least about 97%, at least about 98%, or at least about 99% sequence identity to the amino acid sequence set forth in SEQ ID NO: 18. In some aspects, the cleavable linker comprises the amino acid sequence set forth in SEQ ID NO: 18.

In some aspects, the polynucleotide comprises a nucleotide sequence encoding: (a) a chimeric antigen receptor (CAR), wherein the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region comprising the amino acid sequence set forth in SEQ ID NO: 9; (iii) a transmembrane domain comprising the amino acid sequence set forth in SEQ ID NO: 10; (iv) a costimulatory domain comprising the amino acid sequence set forth in SEQ ID NO: 11; and (v) an intracellular signaling domain comprising the amino acid sequence set forth in SEQ ID NO: 12; and (b) an armor protein, wherein the armor protein comprises an IL-2Rβ polypeptide comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 15.

In some aspects, the polynucleotide comprises a nucleotide sequence encoding: (a) a chimeric antigen receptor (CAR), wherein the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region comprising the amino acid sequence set forth in SEQ ID NO: 9; (iii) a transmembrane domain comprising the amino acid sequence set forth in SEQ ID NO: 10; (iv) a costimulatory domain comprising the amino acid sequence set forth in SEQ ID NO: 11; and (v) an intracellular signaling domain comprising the amino acid sequence set forth in SEQ ID NO: 12; and (b) an armor protein, wherein the armor protein comprises an IL-2Rβ polypeptide comprising the amino acid sequence set forth in SEQ ID NO: 15.

In some aspects, the polynucleotide does not comprise a nucleotide sequence encoding an IL-15Rα polypeptide or an IL-15 polypeptide.

In some aspects, the polynucleotide comprises a nucleotide sequence encoding: (a) a chimeric antigen receptor (CAR), wherein the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region comprising the amino acid sequence set forth in SEQ ID NO: 9; (iii) a transmembrane domain comprising the amino acid sequence set forth in SEQ ID NO: 10; (iv) a costimulatory domain comprising the amino acid sequence set forth in SEQ ID NO: 11; and (v) an intracellular signaling domain comprising the amino acid sequence set forth in SEQ ID NO: 12; and (b) an armor protein, wherein the armor protein comprises: (i) an IL-2Rβ polypeptide comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 15; (ii) a linker comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 18; and (iii) an IL-15Rα polypeptide tethered to an IL-15 polypeptide; wherein: (a) the IL-15Rα polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 17; and (b) the IL-15 polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 16.

In some aspects, the polynucleotide comprises a nucleotide sequence encoding: (a) a chimeric antigen receptor (CAR), wherein the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region comprising the amino acid sequence set forth in SEQ ID NO: 9; (iii) a transmembrane domain comprising the amino acid sequence set forth in SEQ ID NO: 10; (iv) a costimulatory domain comprising the amino acid sequence set forth in SEQ ID NO: 11; and (v) an intracellular signaling domain comprising the amino acid sequence set forth in SEQ ID NO: 12; and (b) an armor protein, wherein the armor protein comprises: (i) an IL-2Rβ polypeptide comprising the amino acid sequence set forth in SEQ ID NO: 15; (ii) a linker comprising the amino acid sequence set forth in SEQ ID NO: 18; and (iii) an IL-15Rα polypeptide tethered to an IL-15 polypeptide; wherein: the IL-15Rα polypeptide comprises the amino acid sequence set forth in SEQ ID NO: 17; and the IL-15 polypeptide comprises the amino acid sequence set forth in SEQ ID NO: 16.

Some aspects of the present disclosure are directed to a vector or a set of vectors comprising a polynucleotide disclosed herein. In some aspects, the vector comprises one or more promoter. In some aspects, the vector is a retroviral vector, a DNA vector, a murine leukemia virus vector, an SFG vector, a plasmid, a RNA vector, an adenoviral vector, a baculoviral vector, an Epstein Barr viral vector, a papovaviral vector, a vaccinia viral vector, a herpes simplex viral vector, an adenovirus associated vector (AAV), a lentiviral vector, or any combination thereof.

Some aspects of the present disclosure are directed to a polypeptide encoded by a polynucleotide disclosed herein.

Some aspects of the present disclosure are directed to a host cell comprising a polynucleotide disclosed herein, a vector or a set of vectors disclosed herein, or a polypeptide disclosed herein.

In some aspects, the host cell is an immune cell. In some aspects, the immune cell is an innate lymphoid cell. In some aspects, the immune cell is selected from a T cell, an NK cell, a B cell, or any combination thereof. In some aspects, the T cell is selected from a γδ T cell, an αβ T cell, or any combination thereof. In some aspects, the T cell is selected from a Vδ1 T cell and a Vδ2 T cell.

In some aspects, the host cell is obtained from a human subject. In some aspects, the host cell is obtained from a blood sample obtained from a human subject, a skin sample obtained from a human subject, a tumor sample obtained from a human subject, a gut tissue sample obtained from a human subject, or any combination thereof. In some aspects, the host cell is derived from an induced pluripotent stem cell (iPSC).

Some aspects of the present disclosure are directed to a population of cells comprising a host cell disclosed herein. In some aspects, at least about 10%, at least about 20%, at least about 30%, at least about 40%, at least about 50%, at least about 60%, at least about 70%, at least about 80%, at least about 90%, at least about 95%, at least about 96%, at least about 97%, at least about 98%, or at least about 99% of the total number of cells in the population of cells comprises the host cell.

Some aspects of the present disclosure are directed to a pharmaceutical composition, comprising a polynucleotide disclosed herein, a vector or a set of vectors disclosed herein, a polypeptide disclosed herein, a host cell disclosed herein, or a population of cells disclosed herein and a pharmaceutically acceptable excipient.

Some aspects of the present disclosure are directed to a method of treating a disease or condition in a subject in need thereof, comprising administering to the subject a polynucleotide disclosed herein, a vector or a set of vectors disclosed herein, a polypeptide disclosed herein, a host cell disclosed herein, a population of cells disclosed herein, or a pharmaceutical composition disclosed herein.

In some aspects, the disease or condition comprises a cancer. In some aspects, the cancer comprises a lung cancer, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemia, acute myeloid leukemia, chronic myeloid leukemia, acute lymphoblastic leukemia, chronic lymphocytic leukemia, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, or any combination thereof. In some aspects, the cancer comprises small cell lung cancer, non-small cell lung cancer, lung adenocarcinoma, mesothelioma, or any combination thereof.

In some aspects, the cancer is locally advanced. In some aspects, the cancer is metastatic. In some aspects, the cancer is refractory or relapsed.

In some aspects, the method further comprises administering an additional anti-cancer agent. In some aspects, the additional anti-cancer therapy comprises a chemotherapy, an immunotherapy, a radiotherapy, a surgery, or any combination thereof. In some aspects, the additional anti-cancer therapy comprises a chemotherapy.

In some aspects, the additional anti-cancer therapy comprises an immune-checkpoint inhibitor. In some aspects, the additional anti-cancer therapy comprises a PD-1 antagonist, a PD-L1 antagonist, a CTLA-4 antagonist, a LAG-3 antagonist, a GITR antagonist, or any combination thereof. In some aspects, the anti-cancer therapy comprises an antibody or antigen-binding portion thereof the specifically binds and inhibits PD-1. In some aspects, the anti-cancer therapy comprises an antibody or antigen-binding portion thereof the specifically binds and inhibits PD-L1.

## BRIEF DESCRIPTION OF THE FIGURES

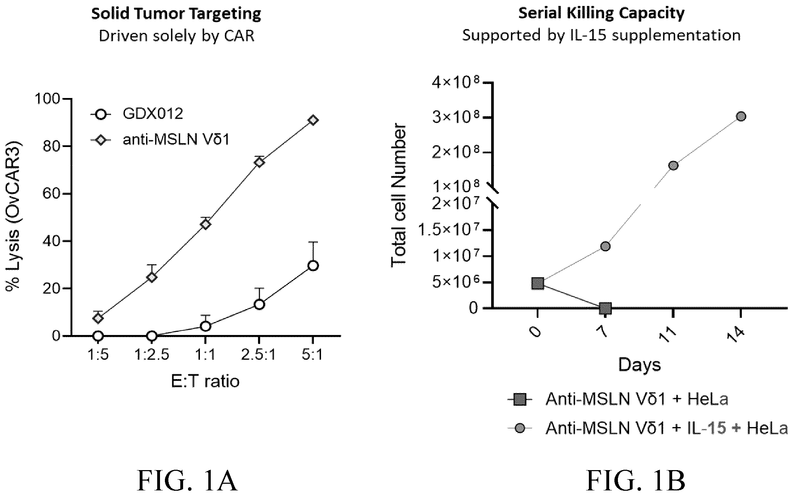

**FIGs. 1A-1B: PoC for functionality of CAR engineered cells against Meso+ve targets.** (FIG. 1A) Blood derived $\gamma\delta$ T cells transduced with the YP218 anti-mesothelin CAR construct and non-engineered blood-derived V$\delta$1 cells ("GDX012") were examined for functionality against a solid tumour target, OVCAR3 cells. Cryopreserved CAR-modified blood V$\delta$1 T-cells were thawed and immediately cultured with target cells in a 5:1, 2.5:1, 1:1, 1:1:2.5 and 1:5 E:T ratio for 18-24h in medium and without cytokine supplementation. Specific cell lysis was detected via a luciferase-based fluorescence cell counting assay. Error bars show mean $\pm$ SD of triplicate wells for each condition. These data demonstrate that genetically engineering GDX012 to express a mesothelin-CAR significantly enhanced the inherent killing capacity of V$\delta$1 T cells against OVCAR3 cells. (FIG. 1B) Blood derived $\gamma\delta$ T cells transduced with the YP218 anti-mesothelin CAR construct (n=1) were cultured in medium alone or IL-15 supplemented medium (10ng/ml) at a 2:1 E:T ratio with Hela cells. On days 7, 14 and 21, cells were harvested, and viable cell counts were performed using the NC250 cell counter. These data demonstrate that that genetically engineering GDX012 to express a mesothelin-CAR significantly enhanced the inherent killing capacity of V$\delta$1 T cells against Hela tumour targets, and that IL-15 is required in addition to CAR signaling for the survival and proliferation of mesothelin CAR-modified blood V$\delta$1 T-cells.

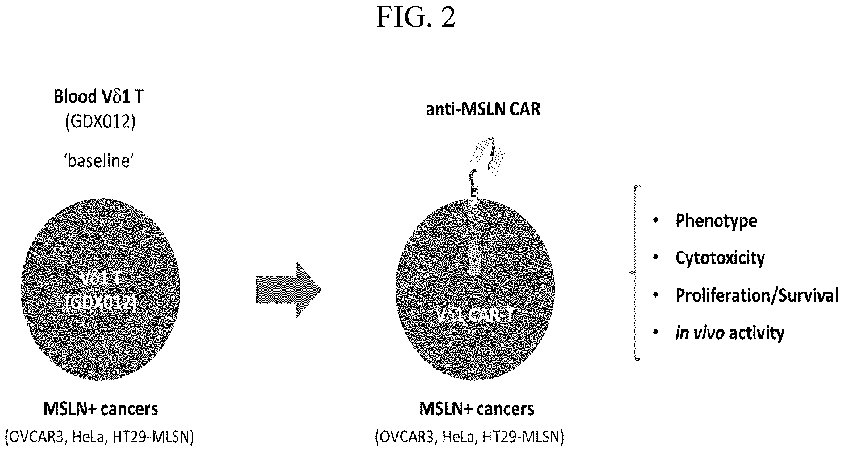

**FIG. 2:** As a proof of concept to target solid tumours, GDX012 was genetically engineered with a YP218 anti-mesothelin CAR construct. Mesothelin was chosen as a proof-of-concept solid tumour target as this protein is overexpressed across various cancer indications and mesothelin is an established target in various solid tumour models and in clinical trials. There is very low expression of mesothelin by healthy tissues making it an ideal solid tumour target. The anti-MSLN V$\delta$1 product was functionally tested to understand phenotype, cytotoxicity, proliferation and in *in vivo* activity to demonstrate solid tumour targeting and establish a framework for future gene engineering products.

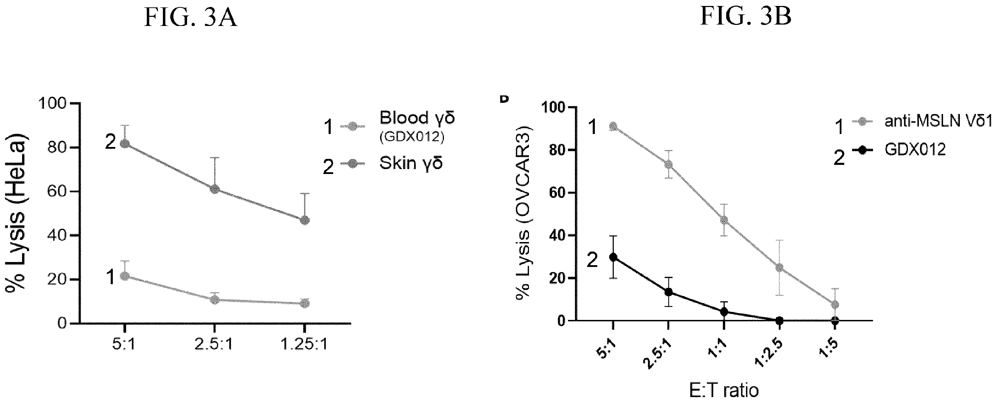

**FIGs. 3A-3B:** GDX012, unlike skin resident $\gamma\delta$ T cells, show modest inherent capacity to target solid tumour targets but can be improved through gene engineering. (FIG. 3A) GDX012 (n=3) and skin resident $\gamma\delta$ T cells (n=3) were examined for functionality against a mesothelin+target cell line, Hela cells. Cryopreserved GDX012 and skin resident $\gamma\delta$ T cells were thawed and immediately cultured with target cells in a 5:1, 2.5:1, 1.25:1, E:T ratio for 18-24h in medium and without cytokine supplementation. Specific cell lysis was detected via a luciferase-based fluorescence cell counting assay. Error bars show mean ± SD of triplicate wells for each condition. These data demonstrate that skin resident γδ T cells have inherent killing capacity against solid tumour targets, with >80% lysis of tumour targets at 5:1 E:T ratio, >60% at 2.5 E:T ratio and >50% at 1.25 E:T. Target cell lysis by GDX012 did not exceed >20% lysis across all E:T ratios profiled against Hela cells. (FIG. 3B) Blood derived γδ T cells transduced with the YP218 anti-mesothelin CAR construct and GDX012 were examined for functionality against OVCAR3 cells. Cryopreserved CAR-modified blood Vδ1 T-cells were thawed and immediately cultured with target cells in a 5:1, 2.5:1, 1.25:1, 0.625:1 and 0.3125:1 E:T ratio for 18-24h in medium and without cytokine supplementation. Specific cell lysis was detected via a luciferase-based fluorescence cell counting assay. Error bars show mean ± SD of triplicate wells for each condition. These data demonstrate that genetically engineering GDX012 to express a mesothelin-CAR significantly enhanced the inherent killing capacity of Vδ1 T cells against OVCAR3 tumour targets, with >90% lysis of tumour targets at 5:1, >75% at 2.5:1, >50% at 1.1 and >20% at 1:2.5 E:T ratio, while target cell lysis by GDX012 did not exceed 30% across all E:T ratios profiled against OVCAR3 cells.

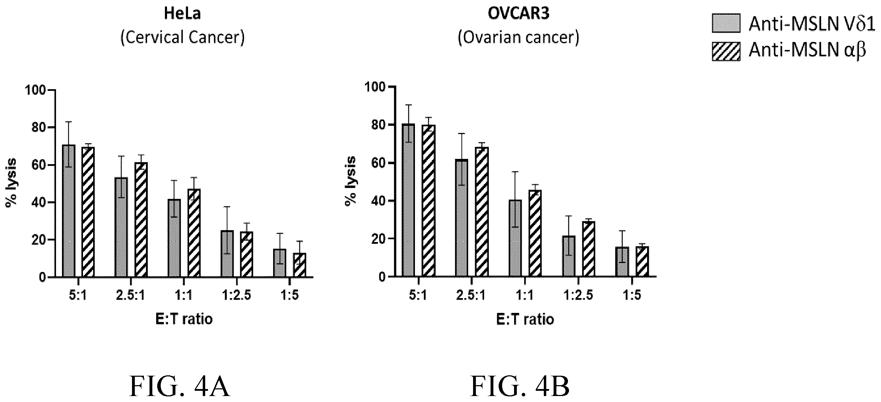

**FIGs. 4A-4B:** Mesothelin CAR-modified blood Vδ1 T-cells show robust cytotoxicity against HeLa (FIG. 4A) and OVCAR3 (FIG. 4B) cells, and this cytotoxicity was comparable to CAR-modified αβ T cells. Blood derived γδ T cells transduced with the YP218 anti-mesothelin CAR construct (n=3) and blood-derived αβ T cells transduced with the YP218 anti-mesothelin construct (n=1) were examined for functionality against Hela and OVCAR3 tumour cells. Cryopreserved CAR-modified blood Vδ1 T-cells were thawed and immediately cultured with target cells in a 5:1, 2.5:1, 1.25:1, 0.625:1 and 0.3125 E:T ratio for 18-24h in medium and without cytokine supplementation. Specific cell lysis was detected via a luciferase-based fluorescence cell counting assay. Error bars show mean ± SD of triplicate wells for each condition.

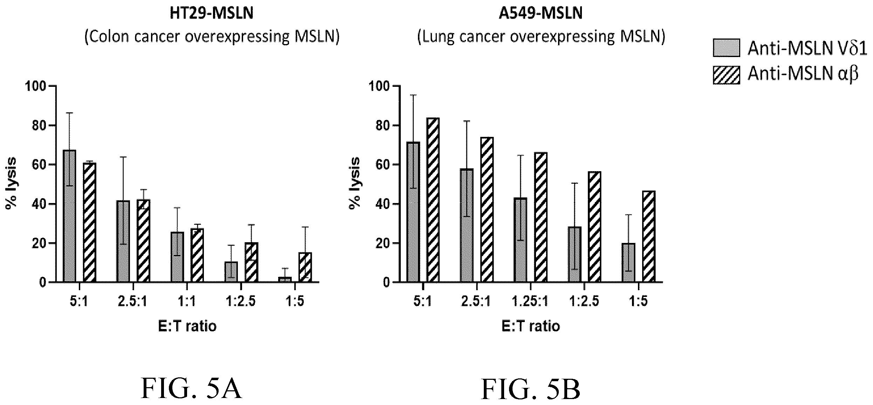

**FIGs 5A-5B:** Mesothelin CAR-modified blood Vδ1 T-cells show robust cytotoxicity against HT29 (FIG. 5A) and A549 (FIG. 5B) modified to over express mesothelin, this cytotoxicity was comparable to CAR-modified αβ T cells. Blood derived γδ T cells transduced with the YP218 anti-mesothelin CAR construct (n=2) and blood derived αβ T cells transduced with the YP218 anti-mesothelin construct (n=1) were examined for functionality against HT29.MSLN. Blood derived γδ T cells transduced with the YP218 anti-mesothelin CAR construct (n=3) and blood derived αβ T cells transduced with the YP218 anti-mesothelin construct (n=1) were tested for functionality against and A549.MSLN cells. Cryopreserved CAR-modified blood Vδ1 T-cells were thawed and immediately cultured with target cells in a 5:1, 2.5:1, 1.25:1, 0.625:1 and 0.3125 E:T ratio for 18-24h in medium and without cytokine supplementation. Specific cell lysis was detected via a luciferase-based fluorescence cell counting assay. Error bars show mean ± SD of triplicate wells for each condition.

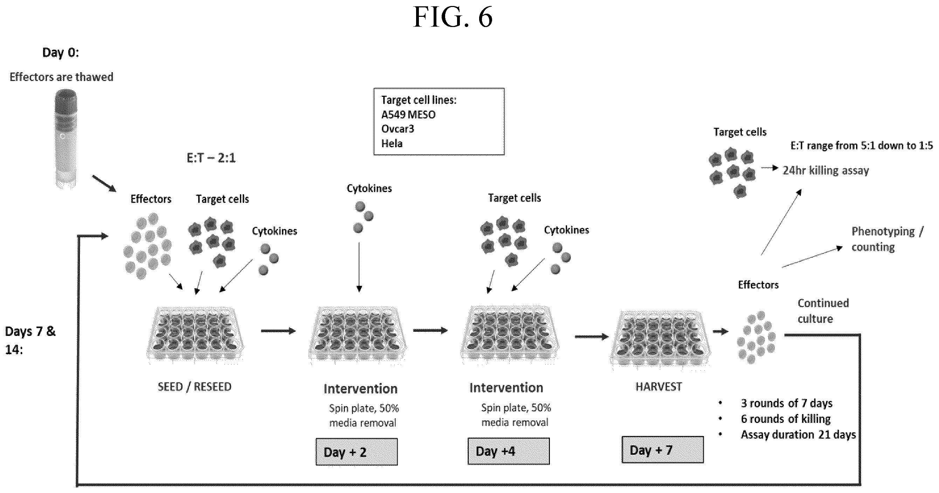

**FIG. 6: Experimental design of repeat antigen stimulation (RAS) assay against solid tumour targets.** Effector cells are thawed and co-cultured in IL-15 supplemented medium (10ng/ml) with solid tumour target cancer cell lines at an E:T ratio of 2:1. Day +2: Cultures are replenished with cytokines through a 50% media exchange. Day +4: Targets and cytokines are replenished through a 50% media exchange. Day +7: Cultures are harvested. Effectors are counted and the equivalent number of effectors seeded at D0 are reseeded in IL-15 supplemented medium (10mg/ml) with target cells as an E:T ratio of 2:1. Additional effectors are used for phenotypic analysis by flow cytometry or seeded in 24hr killing assays with target cells. Overall, effector cells are subject to 6 rounds of in-assay target cell killing and 21 days in culture with the final 24hr killing assay demonstrating a 7th round of serial killing.

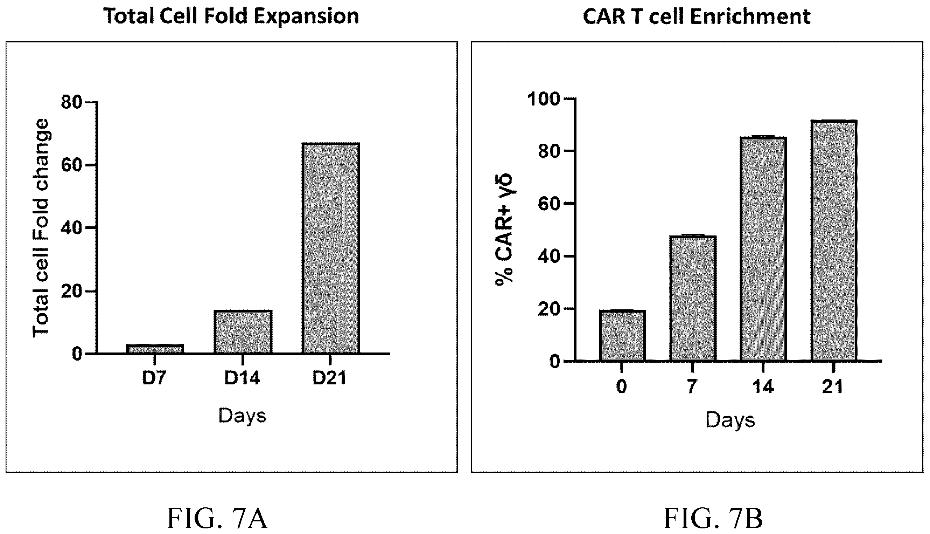

**FIGs. 7A-7B:** Mesothelin CAR-modified blood Vδ1 T-cells expand (FIG. 7A) and enrich for CAR expression (FIG. 7B) following 6 rounds of tumor challenge with Hela cells. Blood derived γδ T cells transduced with the YP218 anti-mesothelin CAR construct (n=2) were cultured in IL-15 supplemented medium (10ng/ml) at a 2:1 E:T ratio with Hela cells. On days 7, 14 and 21, cells were harvested, and viable cell counts were performed using the NC250 cell counter. The fold proliferation of effector cells at each time point was calculated by dividing the cell counts from the harvested effector cells by the number of cells seeded 7 days previously. The total or cumulative fold proliferation was calculated at each time point by multiplying the fold proliferation by the previous fold proliferation. Cells expanded 60-fold over the course of 21 days. CAR expression was measured on Vδ1 T cells on day 0, 7, 14 and 21 by flow cytometry. CAR expressing Vδ1 T cells were enriched over the course 21 days increasing from <20% expression to >90% expression at the end of the assay. Data are representative of two independent experiments.

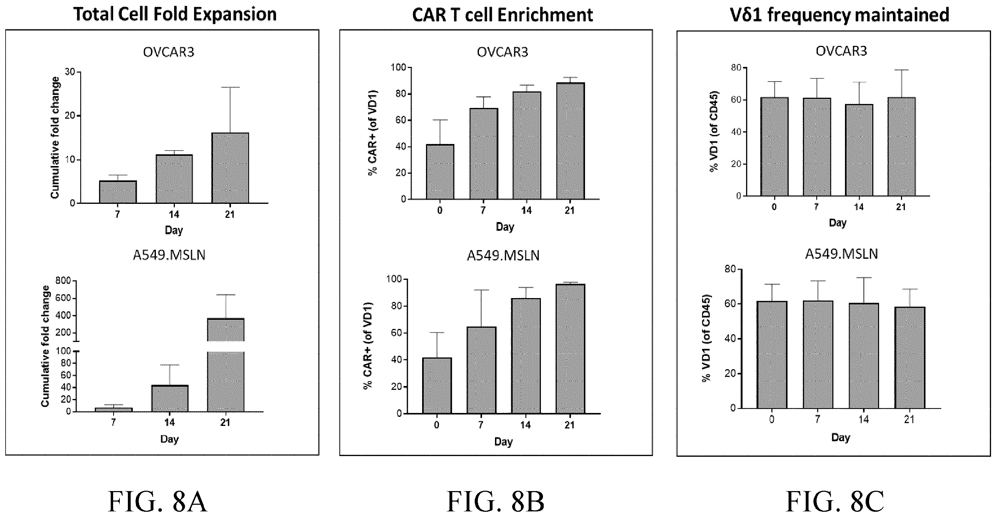

**FIGs. 8A-8C:** Mesothelin CAR-modified blood Vδ1 T-cells expand (FIG. 8A) and enrich (FIG. 8B) in response to serial killing of OVCAR3 and A549.MSLN cells in a RAS assay. Blood derived γδ T cells transduced with the YP218 anti-mesothelin CAR construct (n=3) were cultured in IL-15 supplemented medium (10ng/ml) at a 2:1 E:T ratio with OVCAR3 and A549.MSLN cells. On day 7, 14 and 21, cells were harvested, and viable cell counts were performed using the NC250 cell counter. The fold proliferation of effector cells at each time point was calculated by dividing the cell counts from the harvested effector cells by the number of cells seeded 7 days previously. The total (cumulative) fold proliferation was calculated at each time point by multiplying the fold proliferation by the previous fold proliferation. Cells cultured with OVCAR3 cells expanded 20-fold over the course of 21 days. Cells cultured with A549.MSLN cells expanded 400-fold over the course of 21 days. CAR expression was measured on Vδ1 T cells on day 0, 7, 14 and 21 by flow cytometry. CAR expressing Vδ1 T cells cultured with OVCAR3 and A549.MSLN cells were enriched over the course 21 days increasing to >95% expression at day 21. FIG. 8C shows that V$\delta$1 frequency was maintained over the course of the analysis.

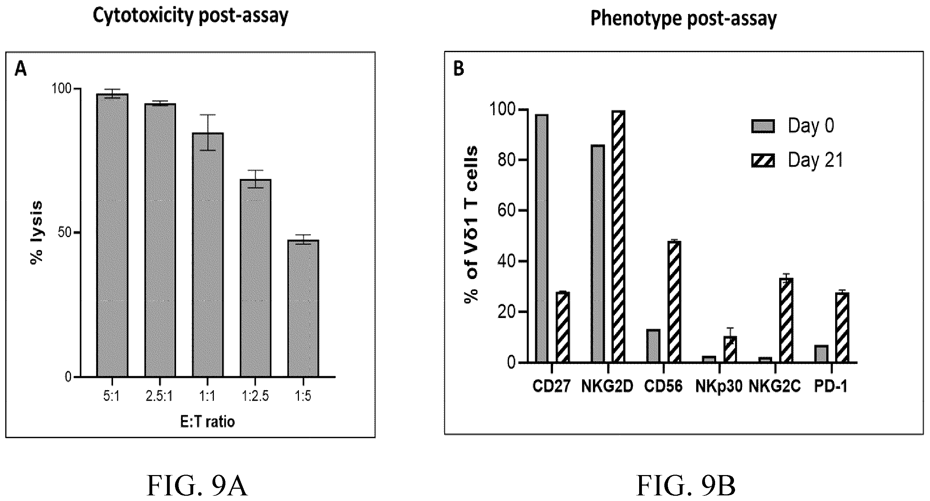

**FIGs. 9A-9B:** Mesothelin CAR-modified blood V$\delta$1 T-cells remain cytotoxic even after 21 days of RAS against Hela tumour cells. (FIG. 9A) Blood derived $\gamma\delta$ T cells transduced with the YP218 anti-mesothelin CAR construct (n=2) were cultured in a 2:1 ratio with Hela cells in IL-15 supplemented medium (10ng/ml) for 21 days. On days 7, 14 and 21, fresh target cells were harvested, and viable cell counts were obtained using the NC250 cell counter. Target cells were seeded at $2 \times 10^5$ cells/well and allowed to adhere for 4h. Effectors were harvested, counted and cultured with adherent target cells in a 5:1, 2.5:1, 1.25:1, 0.625:1 and 0.3125 E:T ratio for 18-24h in medium and without cytokine supplementation. Specific cell lysis was detected via a luciferase-based fluorescence cell counting assay and data is representative of 7 rounds of serial killing. Error bars show mean $\pm$ SD of triplicate wells for each condition. (FIG. 9B) Flow cytometric analysis of live CAR$^+$ $\gamma\delta$ T cells after day 7, 14 and 21 of RAS assay against Hela cells. Phenotypic analysis of V$\delta$1 T cells demonstrated that NKG2D, CD56, NKp30, NKG2C and PD1 expression was upregulated from day 0 to 21, while CD27, representing effector state was downregulated by V$\delta$1 T cells from day 0 to 21. Data are representative of two independent experiments.

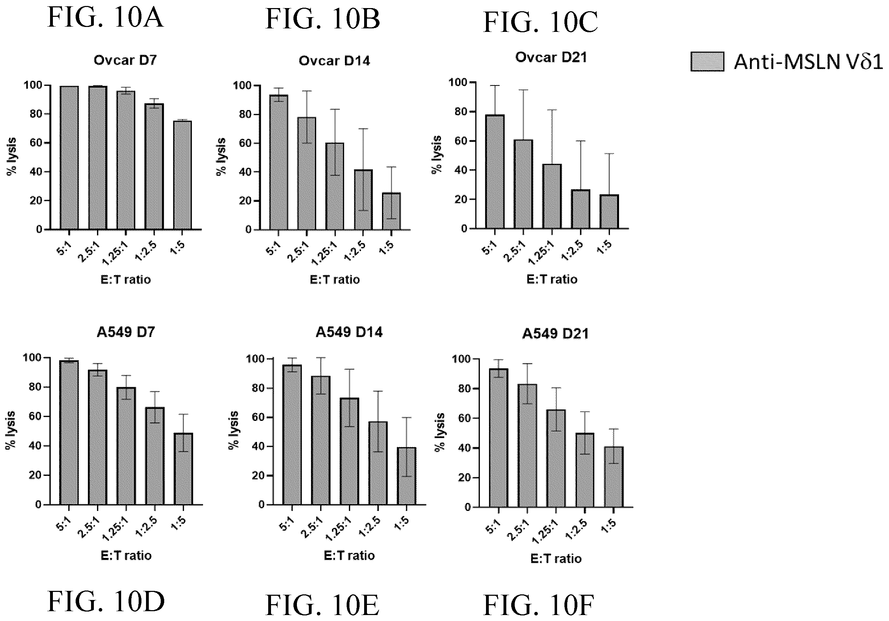

**FIGs. 10A-10F:** Mesothelin CAR-modified blood V$\delta$1 T-cells remain cytotoxic even after 21 days of RAS against OVCAR3 and A549.MSLN cells. Blood derived $\gamma\delta$ T cells transduced with the YP218 anti-mesothelin CAR construct (n=3) were cultured in a 2:1 ratio with OVCAR3 (FIGs. 10A-10C) and A549.MSLN (FIGs. 10D-10F) cells in IL-15 supplemented medium (10ng/ml) for 21 days. On day 7 (FIGs. 10A and 10D), 14 (FIGs. 10B and 10E) and 21 (FIGs. 10C and 10F), fresh target cells were harvested, and viable cell counts were obtained using the NC250 cell counter. Target cells were seeded at $2 \times 10^5$ cells/well and allowed to adhere for 4h. Effectors were harvested, counted and cultured with adherent target cells in a 5:1, 2.5:1, 1.25:1, 0.625:1 and 0.3125 E:T ratio for 18-24h in medium and without cytokine supplementation. Specific cell lysis was detected via a luciferase-based fluorescence cell counting assay. Error bars show mean $\pm$ SD of triplicate wells for each condition.

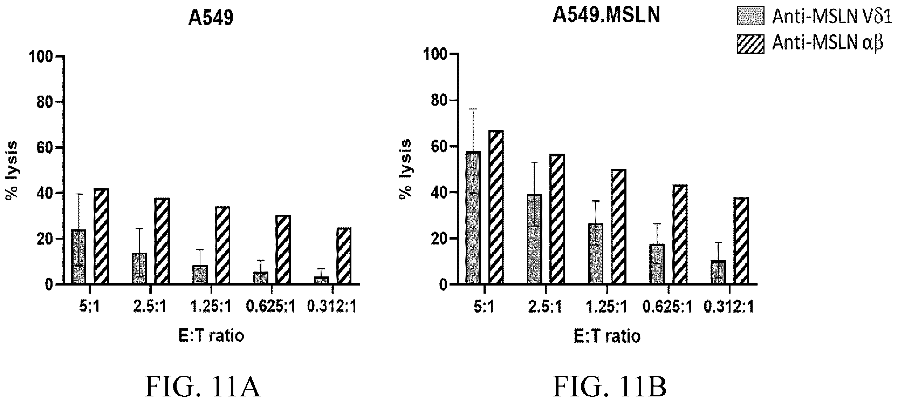

**FIGs. 11A-11B:** Mesothelin CAR-modified blood V$\delta$1 T-cells have greater cytotoxicity against MSLN expressing A549 (FIG. 11A) than unmodified A549 (FIG. 11A) tumour cells. Blood derived $\gamma\delta$ T cells transduced with the YP218 anti-mesothelin CAR construct (n=4) and blood derived $\alpha\beta$ T cells transduced with the YP218 anti-mesothelin construct (n=1) were tested for functionality against A549 tumour cells and A549 tumour cells modified to overexpress MSLN. Cryopreserved CAR-modified blood V$\delta$1 T-cells were thawed and immediately cultured with target cells in a 5:1, 2.5:1, 1.25:1, 0.625:1 and 0.3125 E:T ratio for 18-24h in medium and without cytokine supplementation. Specific cell lysis was detected via a luciferase-based fluorescence cell counting assay. Error bars show mean $\pm$ SD of triplicate wells for each condition.

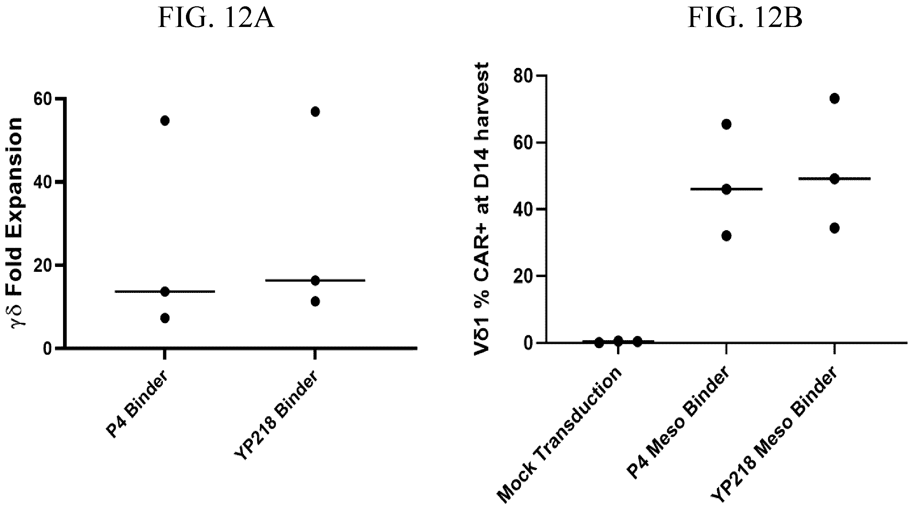

**FIGs. 12A-12B:** Skin derived Vd1+ $\gamma\delta$ T cells can be efficiently transduced with Meso-CAR. Skin resident cells were defrosted and immediately processed via MACS selection to remove $\alpha\beta$ T cells. Resultant negatively selected $\gamma\delta$ T cells were then transduced with vector encoding either P4 or YP218 anti-mesothelin CAR constructs. Transduced cells were then expanded for 14 days in the presence of IL-15 (80ng/ml) and IL21 (11.25ng/ml) before harvest and cryopreservation. The $\gamma\delta$ T cell fold rate of expansion was recorded at D14 of expansion culture (FIG. 12A). The percentage of cells positive for each mesothelin specific CAR was measured at D14 via flow cytometry (FIG. 12B).

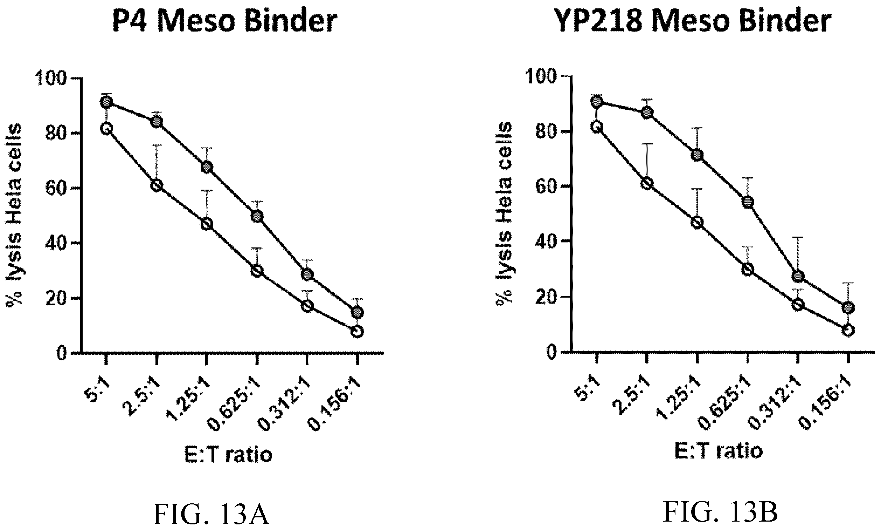

**FIGs. 13A-13B:** Skin derived $\gamma\delta$ T cells are cytotoxic against mesothelin positive target cells, HeLa cell line. Skin resident $\gamma\delta$ T cells were transduced and expanded as stated in FIGs. 12A-12B. Cryopreserved cells were then defrosted and immediately tested for functionality vs the mesothelin$^+$ Hela cell line. Cells were cultured at varying effector to target ratios for 17-20h with no cytokine supplementation. Specific cell lysis was detected for the P4 (FIG. 13A) and YP218 (FIG. 13B) binders using a CellTitreGLO cell counting assay. White circles denote untransduced skin resident $\gamma\delta$ t cells. Grey dots denote mesothelin-specific CAR transduced skin resident $\gamma\delta$ t cells.

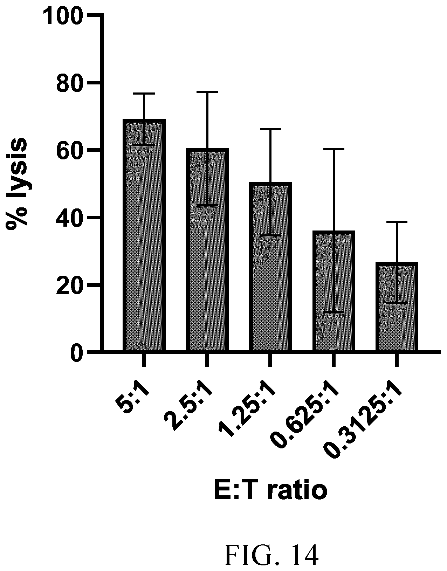

**FIG. 14:** Skin derived $\gamma\delta$ T cells are cytotoxic against mesothelin positive target cells, A459. Skin resident $\gamma\delta$ T cells transduced with the YP218 anti mesothelin CAR construct were tested for functionality versus a mesothelin$^+$ A549 target cell line. Cryopreserved YP218+ expanded skin resident $\gamma\delta$ T cells were defrosted and immediately placed in culture with target cells for 17-20h with no cytokine supplementation. Specific cell lysis was detected via a luciferase-based fluorescence cell counting assay.

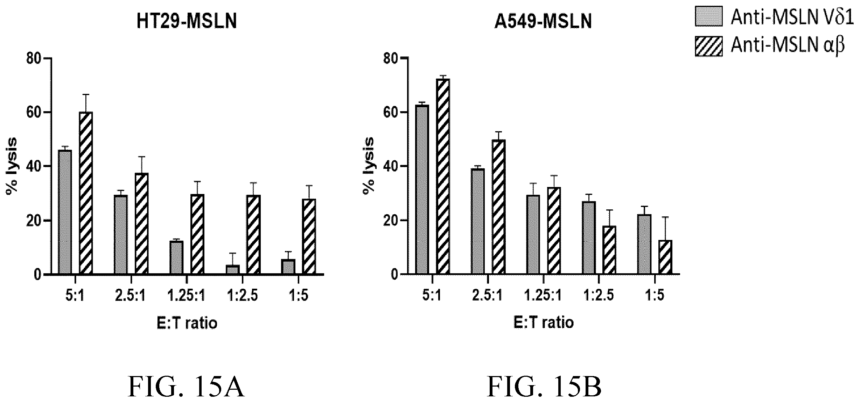

**FIGs. 15A-15B:** Mesothelin CAR-modified skin V$\delta$1 T-cells are cytotoxic against HT29.MSLN and A549.MSLN tumor cells. Skin resident $\gamma\delta$ and $\alpha\beta$ T cells transduced with the YP218 anti mesothelin CAR construct (n=1) were tested for functionality versus mesothelin$^+$ A549 target cell line. Cryopreserved YP218+ expanded skin resident $\gamma\delta$ T cells were defrosted and immediately placed in culture with target HT29.MSLN (FIG. 15A) and A549.MSLN (FIG. 15B) cells for 18-24h with no cytokine supplementation. Specific cell lysis was detected via a luciferase-based fluorescence cell counting assay. Error bars show mean $\pm$ SD of triplicate wells for each condition.

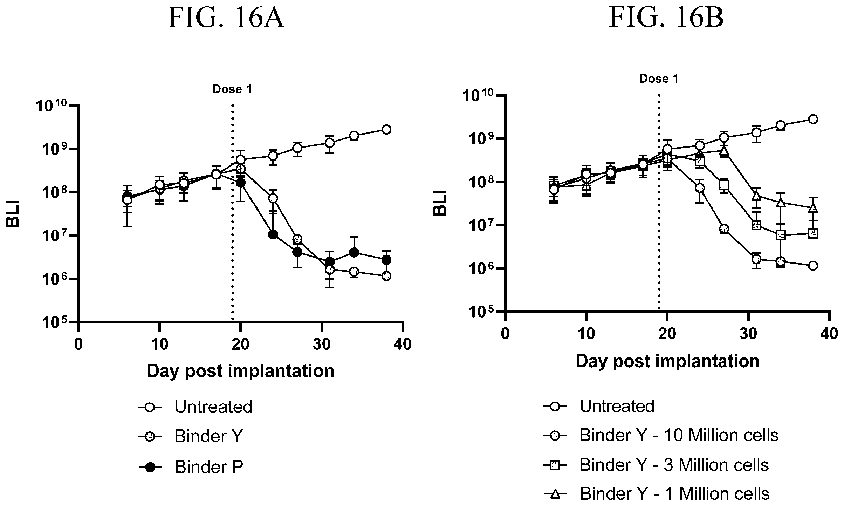

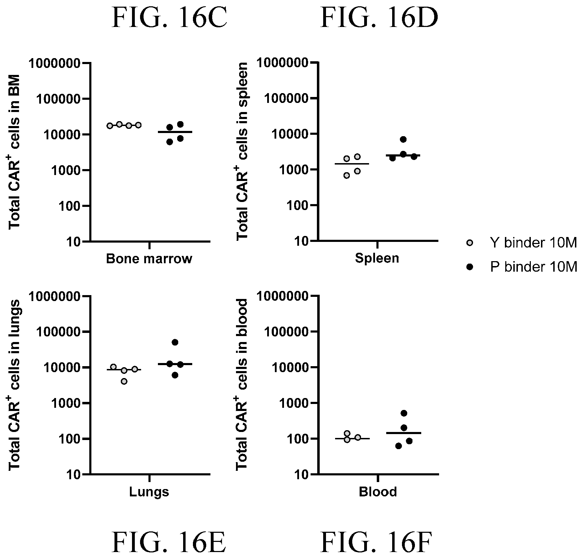

**FIGs. 16A-16F** are graphical representations illustrating that Unarmored P4 and YP218 CAR $\gamma\delta$ T cells are efficacious in a disseminated A549 tumor model in NSG mice. CARs comprised either a "Binder Y" (YP218) or "Binder P" (P4) anti-mesothelin binding domain (as indicated), a CD8 hinge region, a CD8 transmembrane domaing, a 4-1BB costimulatory domain, and a CD3$\zeta$ intracellular signaling domain. FIG. 16A provides data showing that $\gamma\delta$ T cells

transduced with either binder demonstrate effective tumor control ("Binder Y" = YP218; "Binder P" = P4). FIG. 16B provides data showing that anti-MSLN CAR expressing $\gamma\delta$ T cells were able to control tumor growth when dosed with as few as 1 million total cells ("Binder Y" = YP218; "Binder P" = P4). FIGs. 16C-16F provide data showing total the $\gamma\delta$ T cells expressing either anti-MSLN binder persisted in assess niches bone marrow (FIG. 16C), spleen (FIG. 16D), lungs (FIG. 16E), and blood (FIG. 16F) to equivalent levels ("Binder Y" = YP218; "Binder P" = P4).

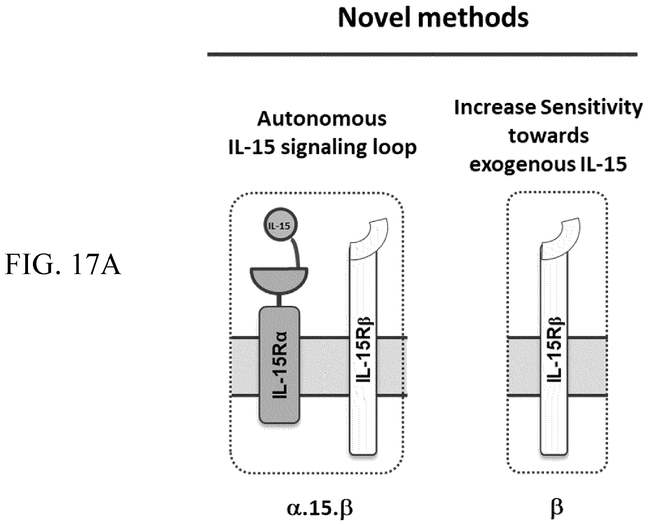

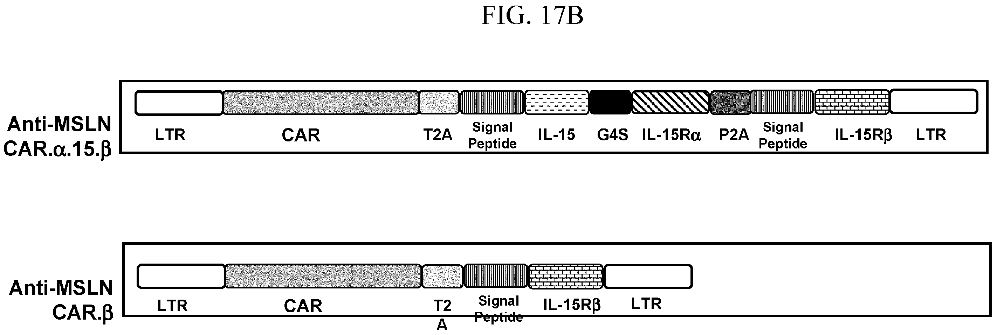

**FIGs. 17A-17B** are schematics of two novel armouring constructs (FIG. 17A) and corresponding multicistronic gammaretroviral vectors generated in which the chimeric antigen receptor (CAR) encoding sequences were fused in frame with individual IL-15 receptor chain components encoding for the wild-type or IL-15 fused (tethered) chain variants (FIG. 17B).

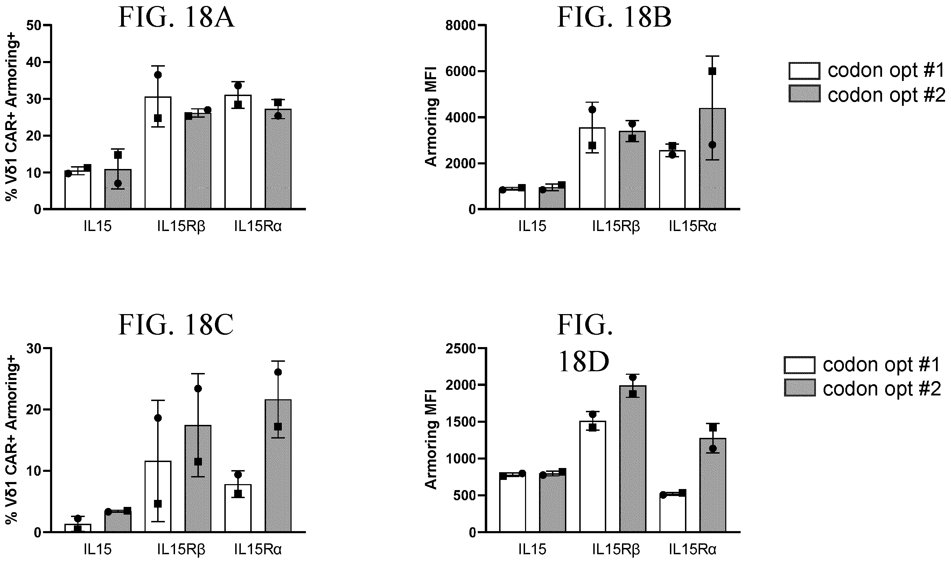

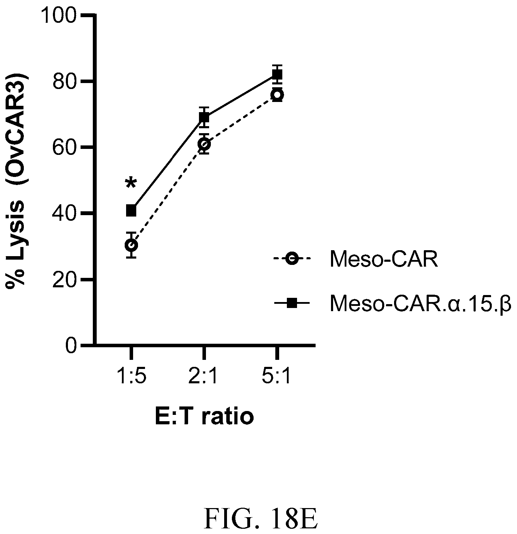

**FIGs. 18A-18D** are bar graphs illustrating the percent of V$\delta$1 CAR$^+$ Armoring$^+$ cells (FIGs. 18A and 18C) or Armoring maximum fluorescence intensity (MFI; FIGs. 18B and 18D) based on codon optimization algorithm for drug product transduced with transduced with P4 $\alpha$.15.$\beta$ codon sequences (FIGs. 18A-18B) or YP218 $\alpha$.15.$\beta$ codon sequences (FIGs. 18C-18D). FIG. 18E shows the cytotoxicity of Mesothelin-targeting CAR engineered cells unarmoured (Meso-CAR) or armoured with mbIL-15 and IL-15R$\beta$ (Meso-CAR.$\alpha$.15.$\beta$).

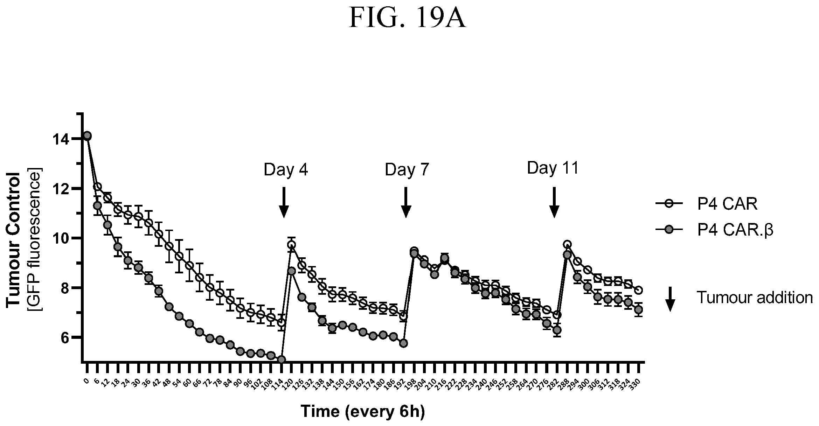

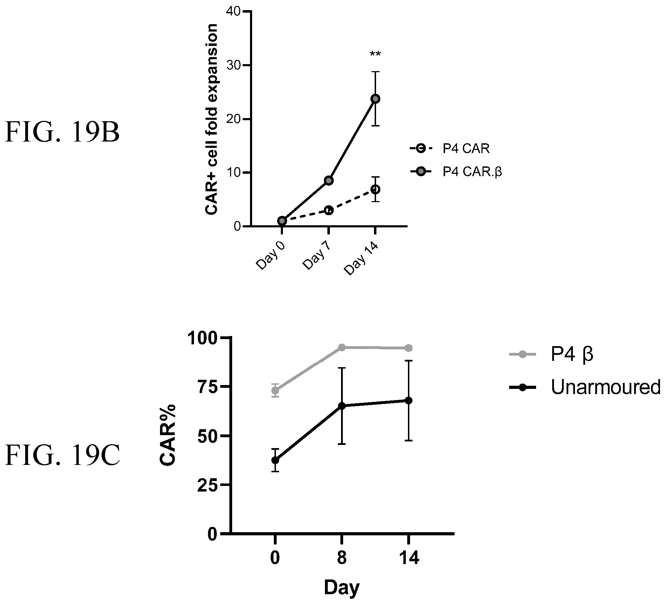

**FIGs. 19A-19C** are graphical representations of the results of a serial killing assay (FIG. 19A), fold expansion (FIG. 19B), and the percent of cells (FIG. 19C) expressing the CAR for cells transduced with an unarmoured binder P construct or a P4 $\beta$ construct.

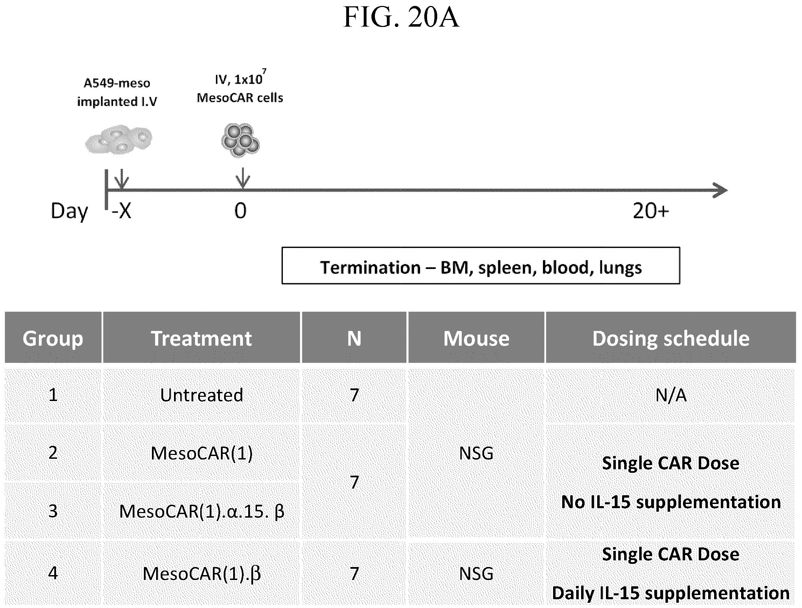

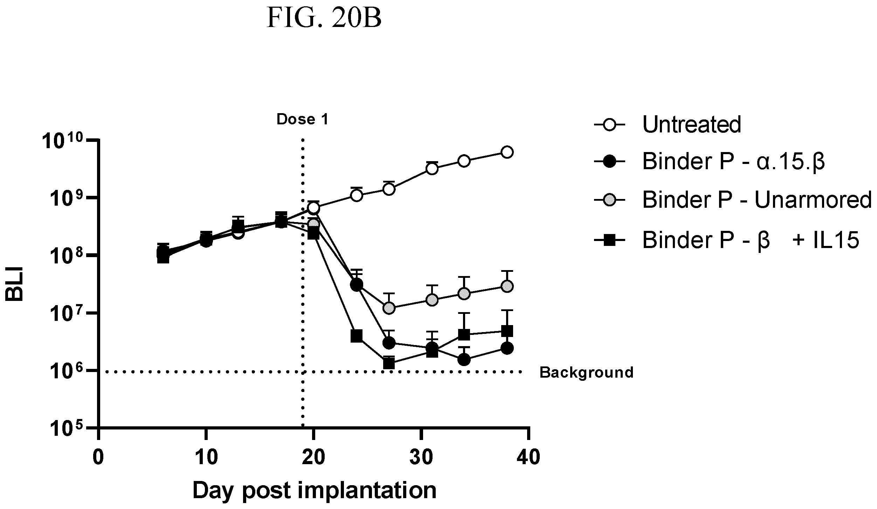

**FIGs. 20A and 20B** are a schematic diagram and results evaluating the *in vivo* performance of unarmoured and armoured with mbIL-15 and IL-15R $\beta$ MesoCAR-modified V$\delta$1 T cells (P4 CAR and P4 CAR.$\alpha$.15.$\beta$, respectively) in a disseminated A549 tumour model. A549 cell lines are engineered to express mesothelin (A549-Meso). FIG. 20A shows the schematic of the model used. FIG. 20B shows *in vivo* efficacy of the products studied.

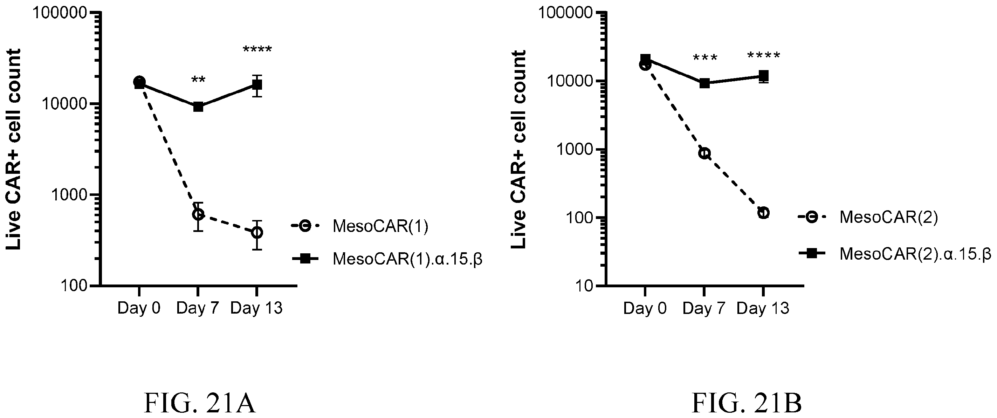

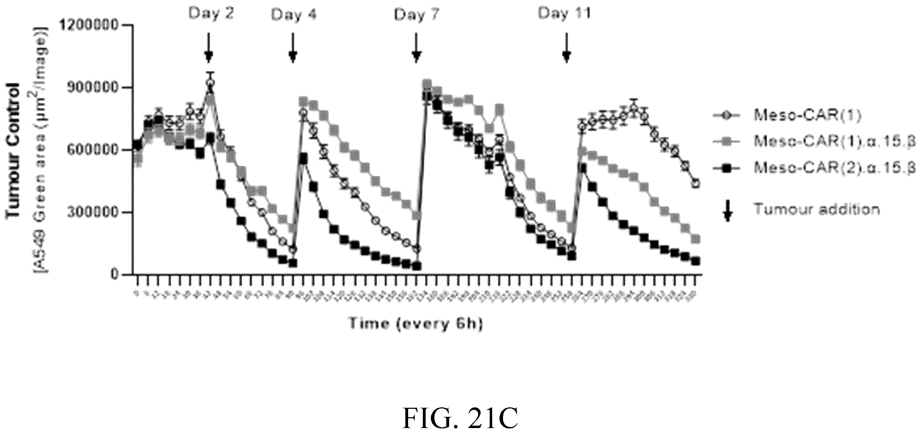

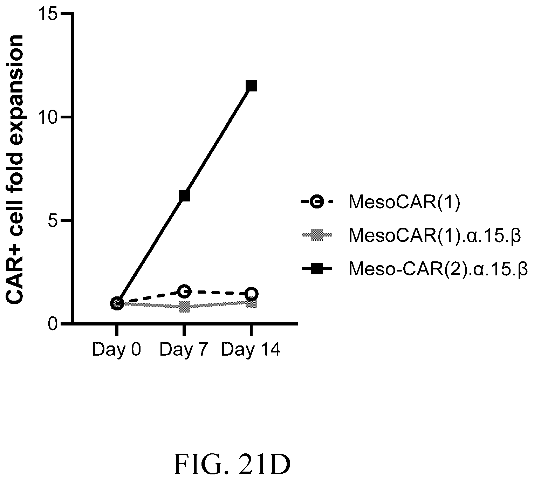

**FIGs. 21A-21D** are graphs evaluating the *in vitro* survival and effect of repeated antigen exposure on two different Mesothelin-targeting CAR(YP218(1) or P4(2))-modified V$\delta$1 T unarmoured, armoured with mbIL-15 and IL-15R$\beta$ (CAR.$\alpha$.15.$\beta$) or armoured with IL-15R$\beta$ (CAR.$\beta$) post-cryopreservation. FIGs. 21A-21B show the survival of CAR T cells of unarmoured and $\alpha$.15.$\beta$ armoured. FIG. 21C shows tumour confluence that was measured by tracking GFP fluorescence over time after treatment with different Meso-CAR T cell products. FIG. 21D shows CAR T cell fold expansion followed during the repeated antigen stimulation assay.

## DETAILED DESCRIPTION OF THE DISCLOSURE

Some aspects of the present disclosure are directed to antigen-binding moieties that specifically bind human mesothelin. Some aspects of the present disclosure are directed to polynucleotides comprising a nucleotide sequence encoding a chimeric antigen receptor (CAR), wherein the CAR comprises an antigen-binding domain that binds human mesothelin. Some aspects of the present disclosure are directed to a host cell, *e.g.,* an immune cell, *e.g.,* an innate lymphoid cell, comprising a polynucleotide disclosed herein. Additional aspects of the present disclosure are directed to methods of treating a subject in need thereof comprising administering the polynucleotide and/or the cell to the subject.

### I. Terms

In order that the present description can be more readily understood, certain terms are first defined. Additional definitions are set forth throughout the detailed description.

It is to be noted that the term "a" or "an" entity refers to one or more of that entity; for example, "a nucleotide sequence," is understood to represent one or more nucleotide sequences. As such, the terms "a" (or "an"), "one or more," and "at least one" can be used interchangeably herein.

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

It is understood that wherever aspects are described herein with the language "comprising," otherwise analogous aspects described in terms of "consisting of" and/or "consisting essentially of" are also provided. As used herein, the terms "comprise" and "include" and variations thereof (*e.g.*, "comprises," "comprising," "includes," and "including") will be understood to indicate the inclusion of a stated component, feature, element, or step or group of components, features, elements or steps but not the exclusion of any other component, feature, element, or step or group of components, features, elements, or steps. Any of the terms "comprising," "consisting essentially of," and "consisting of" may be replaced with either of the other two terms, while retaining their ordinary meanings.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

Units, prefixes, and symbols are denoted in their Système International de Unites (SI) accepted form. Numeric ranges are inclusive of the numbers defining the range. Where a range of values is recited, it is to be understood that each intervening integer value, and each fraction thereof, between the recited upper and lower limits of that range is also specifically disclosed, along with each subrange between such values. The upper and lower limits of any range can independently be included in or excluded from the range, and each range where either, neither or both limits are included is also encompassed within the disclosure. Thus, ranges recited herein are understood to be shorthand for all of the values within the range, inclusive of the recited endpoints. For example, a range of 1 to 10 is understood to include any number, combination of numbers, or sub-range from the group consisting of 1, 2, 3, 4, 5, 6, 7, 8, 9, and 10.

Where a value is explicitly recited, it is to be understood that values which are about the same quantity or amount as the recited value are also within the scope of the disclosure. Where a combination is disclosed, each subcombination of the elements of that combination is also specifically disclosed and is within the scope of the disclosure. Conversely, where different elements or groups of elements are individually disclosed, combinations thereof are also disclosed. Where any element of a disclosure is disclosed as having a plurality of alternatives, examples of that disclosure in which each alternative is excluded singly or in any combination with the other alternatives are also hereby disclosed; more than one element of a disclosure can have such exclusions, and all combinations of elements having such exclusions are hereby disclosed.

Nucleotides are referred to by their commonly accepted single-letter codes. Unless otherwise indicated, nucleotide sequences are written left to right in 5' to 3' orientation. Nucleotides are referred to herein by their commonly known one-letter symbols recommended by the IUPAC-IUB Biochemical Nomenclature Commission. Accordingly, "a" represents adenine, "c" represents cytosine, "g" represents guanine, "t" represents thymine, and "u" represents uracil.

Amino acid sequences are written left to right in amino to carboxy orientation. Amino acids are referred to herein by either their commonly known three letter symbols or by the one-letter symbols recommended by the IUPAC-IUB Biochemical Nomenclature Commission.

The term "about" refers to a value or composition that is within an acceptable error range for the particular value or composition as determined by one of ordinary skill in the art, which will depend in part on how the value or composition is measured or determined, i.e., the limitations of the measurement system. For example, "about" can mean within 1 or more than 1 standard deviation per the practice in the art. Alternatively, "about" can mean a range of up to 10%. Furthermore, particularly with respect to biological systems or processes, the term can mean up to an order of magnitude or up to 5-fold of a value. When particular values or compositions are provided in the application and claims, unless otherwise stated, the meaning of "about" should be assumed to be within an acceptable error range for that particular value or composition.

The terms "administration," "administering," and grammatical variants thereof refer to introducing a composition of the present disclosure (*e.g.*, a polynucleotide encoding a CAR or a cell expressing a CAR), into a subject via a pharmaceutically acceptable route. The introduction of a composition of the present disclosure (*e.g.*, a polynucleotide encoding a CAR or a cell expressing a CAR), into a subject is by any suitable route, including intratumorally, orally, pulmonarily, intranasally, parenterally (intravenously, intra-arterially, intramuscularly, intraperitoneally, or subcutaneously), rectally, intralymphatically, intrathecally, periocularly or topically.

Administration includes self-administration and the administration by another. A suitable route of administration allows the composition or the agent to perform its intended function. For example, if a suitable route is intravenous, the composition is administered by introducing the composition or agent into a vein of the subject.

The term "antigen" refers to a molecule that provokes an immune response. This immune response can involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA.

As used herein, the term "epitope" refers to the moieties of an antigen that specifically interact with an antigen-binding moiety, *e.g.,* a CAR or an antibody molecule. Such moieties, referred to herein as epitopic determinants, typically comprise, or are part of, elements such as amino acid side chains or sugar side chains. An epitopic determinate can be defined, *e.g.,* by methods known in the art, *e.g.,* by crystallography or by hydrogen-deuterium exchange. At least one or some of the moieties on the antibody molecule that specifically interact with an epitopic determinant are typically located in a CDR(s). Typically an epitope has a specific three dimensional structural characteristics. Typically an epitope has specific charge characteristics. Some epitopes are linear epitopes while others are conformational epitopes.

The term "autologous" refers to any material derived from the same individual to whom it is later to be re-introduced into the individual.

The term "chimeric antigen receptor" or "CAR," as used herein, refers to a recombinant polypeptide construct including an extracellular antigen binding domain, a transmembrane domain, and, optionally, an intracellular domain that propagates an activation signal that activates the cell and/or a costimulatory signal. In some embodiments, the CAR includes an optional leader sequence at the N-terminus of the CAR fusion protein. In some embodiments, the CAR lacks an intracellular (*e.g.,* signaling) domain. Expression of a CAR on the surface of a cell, *e.g.,* an immune cell, allows the cell to target and bind a particular antigen. In some aspects, the CAR is expressed by an immune cell, *e.g.,* a αβ T cell, a γδ T cell, or an NK cell. In some aspects, the antigen binding domain comprises an Fab, Fab', F(ab')2, Fd, Fv, single-chain fragment variable (scFv), single chain antibody, VHH, vNAR, nanobody (single-domain antibody), or any combination thereof. In some aspects, the transmembrane domain comprises a transmembrane domain selected from the transmembrane domain of CD8, CD4, or CD28. In some aspects, the intracellular domain comprises a costimulatory domain or a portion thereof. In some aspects, the intracellular domain comprises a costimulatory domain selected from the group consisting of the intracellular domain of a 4-1BB co-stimulatory domain, CD3z, a CD28 co-stimulatory domain, a CD27 co-stimulatory domain, and any combination thereof. A CAR can further comprise a "hinge" or "spacer" domain. Non-limiting examples of hinge/spacer domains include a CD8 hinge or an immunoglobulin hinge/spacer domain, such as an IgG1 hinge domain, and IgG2 hinge domain, an IgG3 hinge domain, or an IgG4 hinge domain.

An "intracellular signaling domain," as the term is used herein, refers to an intracellular portion of a molecule. The intracellular signaling domain can generate a signal that promotes an immune effector function of the CAR containing cell, *e.g.,* an anti-ROR1 CAR T cell described herein. Non-limiting examples of immune effector function, *e.g.,* in a CAR T cell, include cytolytic activity and helper activity, including the secretion of cytokines. In some aspects, the intracellular signal domain is the portion of the protein which transduces the effector function signal and directs the cell to perform a specialized function.

The term "complementarity determining region" or "CDR," as used herein, refers to the sequences of amino acids within antigen-binding variable regions which confer antigen specificity and binding affinity. For example, in general, there are three CDRs in each heavy chain variable region (*e.g.,* VH-CDR1, VH-CDR2, and VH-CDR3) and three CDRs in each light chain variable region (VL-CDR1, VL-CDR2, and VL-CDR3). The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, Md. ("Kabat" numbering scheme), Al-Lazikani et al., (1997) JMB 273,927-948 ("Chothia" numbering scheme), or a combination thereof. Under the Kabat numbering scheme, in some aspects, the CDR amino acid residues in the heavy chain variable domain (VH) are numbered 31-35 (VH-CDR1), 50-65 (VH-CDR2), and 95-102 (VH-CDR3); and the CDR amino acid residues in the light chain variable domain (VL) are numbered 24-34 (VL-CDR1), 50-56 (VL-CDR2), and 89-97 (VL-CDR3). Under the Chothia numbering scheme, in some aspects, the CDR amino acids in the VH are numbered 26-32 (VH-CDR1), 52-56 (VH-CDR2), and 95-102 (VH-CDR3); and the CDR amino acid residues in the VL are numbered 26-32 (VL-CDR1), 50-52 (VL-CDR2), and 91-96 (VL-CDR3). In a combined Kabat and Chothia numbering scheme, in some aspects, the CDRs correspond to the amino acid residues that are part of a Kabat CDR, a Chothia CDR, or both. For instance, in some aspects, the CDRs correspond to amino acid residues 26-35 (VH-CDR1), 50-65 (VH-CDR2), and 95-102 (VH-CDR3) in a VH, *e.g.,* a mammalian VH, *e.g.,* a human VH; and amino acid residues 24-34 (VL-CDR1), 50-56 (VL-CDR2), and 89-97 (VL-CDR3) in a VL, *e.g.,* a mammalian VL, *e.g.,* a human VL.

In some aspects, the CAR comprises a chimeric fusion protein comprising an antigen-binding domain, a transmembrane domain, and an intracellular signaling domain comprising a functional signaling domain derived from a stimulatory molecule, wherein the antigen-binding domain and the transmembrane domain are linked by a CAR hinge/spacer. In some aspects, the CAR comprises a chimeric fusion protein comprising an antigen-binding domain linked to a transmembrane domain via a CAR hinge/spacer and an intracellular signaling domain comprising a functional signaling domain derived from a costimulatory molecule and a functional signaling domain derived from a stimulatory molecule. In some aspects, the CAR comprises a chimeric fusion protein comprising an antigen-binding domain linked to a transmembrane domain via a CAR hinge/spacer and an intracellular signaling domain comprising two functional signaling domains derived from one or more costimulatory molecule(s) and a functional signaling domain derived from a stimulatory molecule. In some aspects, the CAR comprises a chimeric fusion protein comprising an antigen-binding domain linked to a transmembrane domain via a CAR hinge/spacer and an intracellular signaling domain comprising at least two functional signaling domains derived from one or more costimulatory molecule(s) and a functional signaling domain derived from a stimulatory molecule. In some aspects, the CAR comprises an optional leader sequence at the amino-terminus (N-terminus) of the CAR. In some aspects, the CAR further comprises a leader sequence at the N-terminus of the antigen-binding domain, wherein the leader sequence is optionally cleaved from the antigen-binding domain (*e.g.,* a scFv) during cellular processing and localization of the CAR to the cellular membrane.

While the present application often uses CARs to illustrate the different aspects of the disclosed subject matter, it will be apparent to a skilled artisan that the relevant disclosures provided herein can equally apply to other chimeric binding proteins. As used herein, the term "chimeric binding protein" refers to proteins that are capable of binding to one or more antigens (*e.g.,* comprising an antigen-binding moiety) and are created through the joining of two or more heterologous polynucleotides which originally coded for separate proteins or fragments of proteins or multiple fragments of the same protein connected in a non-naturally occurring orientation. Non-limiting examples of other chimeric binding proteins include a T cell receptor (TCR) (*e.g.,* engineered TCR), chimeric antibody-T cell receptor (caTCR), chimeric signaling receptor (CSR), T cell receptor mimic (TCR mimic), and combinations thereof. Accordingly, unless indicated otherwise, the term CARs, in some aspects, can encompass other types of chimeric binding proteins known in the art, *e.g.,* those described herein.

As used herein, the term "affinity" refers to a measure of the strength of the binding of an antigen or target (such as an epitope) to its cognate binding domain (such as a paratope). As used herein, the term "avidity" refers to the overall stability of the complex between a population of epitopes and paratopes (i.e., antigens and antigen binding domains).

The term "binds to the same epitope" with reference to two or more antigen-binding moieties means that the antigen-binding moieties bind to the same segment of amino acid residues. Antigen-binding moieties that "compete with another antibody for binding to a target" refer to antigen-binding moieties that inhibit (partially or completely) the binding of the other antibody to the target.

As used herein, the terms "specific binding," "selective binding," "selectively binds," and "specifically binds," refer to an antigen-binding moiety (*e.g.,* a CAR) binding to an epitope on a predetermined antigen. Typically, the antigen-binding moiety (*e.g.,* a CAR) (i) binds with an equilibrium dissociation constant (KD) of approximately less than $10^{-7}$ M, such as approximately less than $10^{-8}$ M, $10^{-9}$ M or $10^{-10}$ M or lower as determined by, *e.g.,* surface plasmon resonance (SPR) technology in a BIACORE® 2000 instrument using the predetermined antigen, *e.g.,* human mesothelin, as the target polypeptied and the CAR as the ligand, and (ii) binds to the predetermined antigen with an affinity that is at least two-fold greater than its affinity for binding to a non-specific antigen other than the predetermined antigen or a closely-related antigen. Accordingly, an antigen-binding moiety (*e.g.,* a CAR) that "specifically binds to human mesothelin" refers to an antigen-binding moiety (*e.g.,* a CAR) that binds to human mesothelin with a KD of $10^{-7}$ M or less, such as approximately less than $10^{-8}$ M, less than $10^{-9}$ M, or less than $10^{-10}$ M.

As used herein, the term "engineered $\gamma\delta$ T cell" refers to a $\gamma\delta$ T cell that expresses a transgene (i.e., a gene that has been transduced into the engineered $\gamma\delta$ T cell or a parental cell thereof).

As used herein, the term "primed $\gamma\delta$ T cell" refers to a starting population (*e.g.,* an endogenous population of $\gamma\delta$ T cells) that has been affected by a culture condition. In some instances, a primed $\gamma\delta$ T cell has a different functional viral entry receptor profile relative to its unprimed counterpart before experiencing the culture condition. In some embodiments, a population of primed $\gamma\delta$ T cells is an expanded population of $\gamma\delta$ T cells.

As used herein, an "expanded population of $\gamma\delta$ cells" refers to a population of haematopoietic cells including $\gamma\delta$ T cells that has been cultured in a condition and for a duration that has induced the expansion of $\gamma\delta$ cells, i.e., increased $\gamma\delta$ cell number. Likewise, an "expanded population of V$\delta$1 T cells," as used herein, refers to a population of haematopoietic cells including V$\delta$1 T cells that has been cultured in a condition and for a duration that has induced the expansion of V$\delta$1 T cells, i.e., increased V$\delta$1 cell number. Similarly, an "expanded population of V$\delta$2 T cells," as used herein, refers to a population of haematopoietic cells including V$\delta$2 T cells that has been cultured in a condition and for a duration that has induced the expansion of V$\delta$2 T cells, i.e., increased V$\delta$2 cell number.

As used herein, a "population" of $\gamma\delta$ T cells refers to a group of three or more $\gamma\delta$ T cells (*e.g.,* at least 10, at least $10^7$, at least $10^3$, at least $10^4$, at least $10^5$, at least $10^6$, at least $10^7$, at least $10^8$, at least $10^9$, at least $10^{10}$, at least $10^{11}$, at least $10^{12}$, or at least $10^{13}$) $\gamma\delta$ T cells (*e.g.,* engineered $\gamma\delta$ T cells). A population of a particular cell type (*e.g.,* a population of endogenous $\gamma\delta$ T cells, a population of primed $\gamma\delta$ T cells, or a population of engineered $\gamma\delta$ T cells) refers to the cells of that type and not to cells of a different type within a broader population. For example, if 10% of the cells of a starting population of $10^8$ T cells are $\gamma\delta$ T cells, the starting population of $\gamma\delta$ T cells is $10^7$.

As used herein, an "armor protein" refers to a protein encoded by a transgene that, when expressed by an immune cell, *e.g.,* an NK cell or a $\gamma\delta$ T cell (*e.g.,* a $\gamma\delta$ T cell expressing a CAR), increases persistency and/or proliferation of the immune cell, *e.g.,* NK cell or $\gamma\delta$ T cell, and/or the cytotoxicity of the immune cell, *e.g.,* NK cell or $\gamma\delta$ T cell, toward a target cell, *e.g.,* through signaling (*e.g.,* cytokine signaling) to improve, *e.g.,* cell persistence, cell viability, activation and other desired characteristics. An armor protein can be a membrane-bound protein or a soluble protein. For example, armor proteins include membrane-bound proteins, such as a membrane-bound receptor (*e.g.,* IL-15R (*e.g.,* IL-15R$\beta$ and IL-15R$\alpha$ or a fragment thereof) and/or IL-2R (*e.g.,* IL-2R$\beta$ or a fragment thereof), $\alpha\beta$ TCR, a natural cytotoxicity receptor (*e.g.,* NKp30, NKp44, or NKp46), a cytokine receptor (*e.g.,* IL-12 receptor), and/or a chemokine receptor (*e.g.,* CCR2 receptor) and/or a membrane-bound ligand or cytokine (*e.g.,* membrane-bound IL-15, membrane-bound IL-7, membrane-bound CD40L, membrane-bound 4-1BB, membrane-bound 4-1BBL, membrane bound CCL19). Additionally, or alternatively, armor proteins can be soluble proteins, such as soluble ligands or cytokines (*e.g.,* soluble IL-15, soluble IL-7, soluble IL-12, soluble CD40L, soluble 4-1BBL, and/or soluble CCL19). In some aspects, an armor protein is not antigen specific. In some aspects, the armor protein comprises an IL-2R$\beta$ polypeptide or a fragment thereof. In some aspects, the IL-2R$\beta$ polypeptide is a recombinant polypeptide. In some aspects, the IL-2R$\beta$ polypeptide is expressed without IL-2R$\alpha$ and with tethering to an IL-15. In some aspects, the IL-2R$\beta$ polypeptide is expressed without IL-2R$\alpha$ and without tethering to an IL-15.

In some aspects, an armor protein comprises two polypeptides. In some aspects, the armor protein comprises two polypeptides that are fused, *i.e.,* as a contiguous polypeptide. In some aspects, the armor protein comprises two polypeptides that associate to form a dimer. In some aspects, the armor protein comprises a dimer comprising (i) a fusion protein comprising IL-15Rα and IL-15 and (ii) IL-2Rβ.

As used herein, "IL-15" refers to native or recombinant IL-15 or a variant thereof having at least 70% (*e.g.,* at least 80%, 85%, 90%, 95%, 97%, 99%, *e.g.,* at least 85%) sequence identity over an equal length portion that acts as an agonist for one or more IL-15 receptor (IL-15R) subunits (*e.g.*, muteins, analogues, subunits, receptor complexes, fragments, isoforms, and peptidomimetics thereof). IL-15, like IL-2, is a known T-cell growth factor that can support proliferation of an IL-2-dependent cell line, CTLL-2. IL-15 was first reported by Grabstein et al. (Science 264.5161: 965-969, 1994) as a 114-amino acid mature protein. The term "IL-15," as used herein, means native or recombinant IL-15 and muteins, analogs, subunits thereof, or complexes thereof (*e.g.,* receptor complexes, *e.g.,* sushi peptides, as described in PCT Pub. No. WO 2007/046006), and each of which can stimulate proliferation of CTLL-2 cells. In the CTLL-2 proliferation assays, supernatants of cells transfected with recombinantly expressed precursor and in-frame fusions of mature forms of IL-15 can induce CTLL-2 cell proliferation. As used herein, the term "mbIL-15" refers to a membrane-bound IL-15Rα tethered to an IL-15 (*see, e.g.,* FIG. 17A).

Human IL-15 can be obtained according to the procedures described by Grabstein et al. (Science 264.5161: 965-969, 1994) or by conventional procedures such as polymerase chain reaction (PCR). A deposit of human IL-15 cDNA was made with the ATCC® on Feb. 19, 1993, and assigned accession number 69245.

The amino acid sequence of human IL-15 (Gene ID 3600) is found in Genbank under accession locator NP000576.1 GI: 10835153 (isoform 1) and NP_751915.1 GI: 26787986 (isoform 2). The murine (*Mus musculus*) IL-15 amino acid sequence (Gene ID 16168) is found in Genbank under accession locator NP_001241676.1 GI: 363000984.

IL-15 can also refer to IL-15 derived from a variety of mammalian species, including, for example, human, simian, bovine, porcine, equine, and murine. An IL-15 "mutein," as referred to herein, is a polypeptide that has an amino acid sequence different from a native mammalian IL-15 polypeptide because of one or more amino acid deletions, insertions, and/or substitutions. Variants may comprise conservatively substituted sequences, meaning that a given amino acid residue is replaced by a residue having similar physiochemical characteristics. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Other such conservative substitutions, for example, substitutions of entire regions having similar hydrophobicity characteristics, are well known. Naturally occurring IL-15 variants are also encompassed by the invention. Examples of such variants are proteins that result from alternate mRNA splicing events or from proteolytic cleavage of the IL-15 protein, wherein the IL-15 binding property is retained. Alternate splicing of mRNA may yield a truncated but biologically active IL-15 protein. Variations attributable to proteolysis include, for example, differences in the N- or C-termini upon expression in different types of host cells, due to proteolytic removal of one or more terminal amino acids from the IL-15 protein (generally from 1-10 amino acids). In some embodiments, the terminus of the protein can be modified to alter its physical properties, for example, with a chemical group such as polyethylene glycol (Yang et al. Cancer 76:687-694, 1995). In some embodiments, the terminus or interior of the protein can be modified with additional amino acids (Clark-Lewis et al. PNAS 90:3574-3577, 1993).

As used herein, "IL-2 receptor β subunit," "IL-2Rβ," "IL-15 receptor β subunit," and "IL-15Rβ," used interchangeably herein, refer to native or recombinant IL-15Rβ or a variant thereof having at least 70% (*e.g.,* at least 80%, 85%, 90%, 95%, 97%, 99%, *e.g.,* at least 85%) sequence identity over an equal length portion that acts as a receptor for one or more of IL-2 or IL-15 (*e.g.*, muteins, analogues, fragments, isoforms, and peptidomimetics thereof). IL-15Rβ includes full length and fragments thereof. IL-2Rβ, also known as IL-15Rβ, is a known T-cell growth factor receptor that can support proliferation of an IL-2-dependent cell line, CTLL-2. IL-2Rβ is a 551-amino acid mature protein. The term "IL-15Rβ," as used herein, means native or recombinant IL-2Rβ and muteins, analogs, subunits thereof, or complexes thereof.

The amino acid sequence of human IL-2Rβ (Gene ID 3560) is found in Genbank under accession locator NP_000869, NP_001333151, and NP_001333152. The murine (*Mus musculus*) IL-2Rβ amino acid sequence (Gene ID 16185) is found in Genbank under accession locator NP_032394.

IL-15Rβ can also refer to IL-15Rβ derived from a variety of mammalian species, including, for example, human, simian, bovine, porcine, equine, and murine. An IL-15Rβ "mutein" as referred to herein, is a polypeptide that has an amino acid sequence different from a native mammalian IL-15Rβ polypeptide because of one or more amino acid deletions, insertions, and/or substitutions. Variants may comprise conservatively substituted sequences, meaning that a given amino acid residue is replaced by a residue having similar physiochemical characteristics. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Other such conservative substitutions, for example, substitutions of entire regions having similar hydrophobicity characteristics, are well known. Naturally occurring IL-15Rβ variants are also encompassed by the invention. Examples of such variants are proteins that result from alternate mRNA splicing events or from proteolytic cleavage of the IL-15Rβ protein, wherein the IL-15Rβ binding property is retained. Alternate splicing of mRNA may yield a truncated but biologically active IL-15Rβ protein. Variations attributable to proteolysis include, for example, differences in the N- or C-termini upon expression in different types of host cells, due to proteolytic removal of one or more terminal amino acids from the IL-15Rβ protein (generally from 1-10 amino acids).

As used herein, "IL-15 receptor α subunit," and "IL-15Rα," refer to native or recombinant IL-15Rα or a variant thereof that acts as a receptor for IL-15 (*e.g.*, muteins, analogues, fragments, isoforms, and peptidomimetics thereof). IL-15Rα includes full length and fragments thereof. IL-15Rα, is a known T-cell growth factor receptor. IL-15Rα is a 267-amino acid mature protein. The term "IL-15Rα," as used herein, means native or recombinant IL-15Rα and muteins, analogs, subunits thereof, or complexes thereof. Fragments of IL-15Rα include soluble fragments, *e.g.*, sushi peptides, as described in PCT Pub. No. WO 2007/046006. Soluble fragments (*e.g.*, sushi peptides, such as a fragment containing residues 31-95 of IL-15Rα or a variant thereof having at least 70% (*e.g.*, at least 80%, 85%, 90%, 95%, 97%, 99%, *e.g.*, at least 85%) sequence identity over an equal length portion) also include muteins, analogues, fragments, isoforms, and peptidomimetics thereof. In some embodiments, a soluble fragment is attached to an Fc domain, *e.g.*, Fc-sushi or sushi-Fc.

The amino acid sequence of human IL-15Rα (Gene ID 3601) is found in Genbank under accession locator NP_001230468, NP_001243694, NP_002180, NP_751950, and NP_001338024. The murine (*Mus musculus*) IL-15Rα amino acid sequence (Gene ID 16169) is found in Genbank under accession locator NP_001258426.

IL-15Rα can also refer to IL-15Rα derived from a variety of mammalian species, including, for example, human, simian, bovine, porcine, equine, and murine. An IL-15Rα "mutein," as referred to herein, is a polypeptide that has an amino acid sequence different from a native mammalian IL-15Rα polypeptide because of one or more amino acid deletions, insertions, and/or substitutions. Variants may comprise conservatively substituted sequences, meaning that a given amino acid residue is replaced by a residue having similar physiochemical characteristics. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Other such conservative substitutions, for example, substitutions of entire regions having similar hydrophobicity characteristics, are well known. Naturally occurring IL-15Rα variants are also encompassed by the invention. Examples of such variants are proteins that result from alternate mRNA splicing events or from proteolytic cleavage of the IL-15Rα protein, wherein the IL-15Rα binding property is retained. Alternate splicing of mRNA may yield a truncated but biologically active IL-15Rα protein. Variations attributable to proteolysis include, for example, differences in the N- or C-termini upon expression in different types of host cells, due to proteolytic removal of one or more terminal amino acids from the IL-15Rα protein (generally from 1-10 amino acids). In some embodiments, the terminus of the protein can be modified to alter its physical properties, for example, with a chemical group such as polyethylene glycol (Yang et al. Cancer 76:687-694, 1995). In some embodiments, the terminus or interior of the protein can be modified with additional amino acids (Clark-Lewis et al. PNAS 90:3574-3577, 1993).

A "polypeptide" refers to a chain comprising at least two consecutively linked amino acid residues, with no upper limit on the length of the chain. One or more amino acid residues in the protein can contain a modification such as, but not limited to, glycosylation, phosphorylation or disulfide bond formation. A "protein" can comprise one or more polypeptides.

The term "mesothelin" or "CAK1 antigen," as used herein, refers to a glycosylphosphatidylinositol-anchored membrane polypeptide involved in cellular adhesion (*see* UNIPROT reference number Q13421). Overexpression of mesothelin has been observed in various types of cancer, making mesothelin a promising target for CAR-T therapy.

The term "nucleic acid molecule," as used herein, is intended to include DNA molecules and RNA molecules. A nucleic acid molecule can be single- stranded or double- stranded, and can be cDNA.

"Conservative amino acid substitutions" refer to substitutions of an amino acid residue with an amino acid residue having a similar side chain. Families of amino acid residues having similar side chains have been defined in the art. These families include amino acids with basic side chains (*e.g.,* lysine, arginine, histidine), acidic side chains (*e.g.,* aspartic acid, glutamic acid), uncharged polar side chains (*e.g.*, glycine, asparagine, glutamine, serine, threonine, tyrosine, cysteine, tryptophan), nonpolar side chains (*e.g.*, alanine, valine, leucine, isoleucine, proline, phenylalanine, methionine), beta-branched side chains (*e.g.*, threonine, valine, isoleucine) and aromatic side chains (*e.g.*, tyrosine, phenylalanine, tryptophan, histidine). In some aspects, a predicted nonessential amino acid residue in a mesothelin-binding moiety (*e.g.,* an anti-mesothelin CAR) is replaced with another amino acid residue from the same side chain family.

The terms "tether" or "tethered," as used herein refer to a polypeptide that, when present, joins the two or more polypeptides (*e.g.,* an IL-15Rα or fragment thereof (*e.g.,* a sushi domain) and IL-15 or a variant thereof). In general, a tether may be from about *e.g.,* 1 to 100 (*e.g.,* 5 to 50, 5 to 30, 5 to 20, *e.g.,* 1, 2, 3, 4, 5, 6, 7, 8, 9, 10, 11, 12, 13, 14, 15, 16, 17, 18, 19, 20, 21, 22, 23, 24, 25, 30, 35, 40, 45, 50, 60, 70, 80, 90, or 100) amino acids in length. In some embodiments, the tether is highly flexible and may be rich in glycine (G) and/or serine (S) residues, which may be present in the form of GS repeats. In some embodiments, a tether may link a C-terminus of a first protein to an N-terminus of a second protein. In other embodiments, a tether may attach a C-terminus of the second protein to an N-terminus of the first protein.

As used herein, the term "percent (%) identity" refers to the percentage of amino acid residues of a candidate sequence, *e.g.,* a VH, a VL, or an IL-15Rβ variant, that are identical to the amino acid residues of a reference sequence, *e.g.,* a wild-type IL-15Rβ polypeptide, after aligning the sequences and introducing gaps, if necessary, to achieve the maximum percent identity (i.e., gaps can be introduced in one or both of the candidate and reference sequences for optimal alignment and non-homologous sequences can be disregarded for comparison purposes). Alignment for purposes of determining percent identity can be achieved in various ways that are within the skill in the art, for instance, using publicly available computer software such as BLAST, ALIGN, or Megalign (DNASTAR) software. Those skilled in the art can determine appropriate parameters for measuring alignment, including any algorithms needed to achieve maximal alignment over the full length of the sequences being compared. In some embodiments, the percent amino acid sequence identity of a given candidate sequence to, with, or against a given reference sequence (which can alternatively be phrased as a given candidate sequence that has or includes a certain percent amino acid sequence identity to, with, or against

$$100 \times (\text{fraction of A/B})$$

a given reference sequence) is calculated as follows: where A is the number of amino acid residues scored as identical in the alignment of the candidate sequence and the reference sequence, and where B is the total number of amino acid residues in the reference sequence. In some embodiments where the length of the candidate sequence does not equal to the length of the reference sequence, the percent amino acid sequence identity of the candidate sequence to the reference sequence would not equal to the percent amino acid sequence identity of the reference sequence to the candidate sequence.

As used herein, a "2A peptide" refers to a class of 18-22 amino acid long peptides that share a core sequence motif of DxExNPGP, where X is any amino acid. The 2A peptide may be a foot-and-mouth disease virus 18 2A (F2A) peptide, an equine rhinitis A virus 2A (E2A) peptide, a porcine teschovirus-1 2A (P2A) peptide, or a thosea asigna virus 2A (T2A) peptide.

A "suicide gene" refers to a gene that causes a cell to kill itself. In some aspects, the suicide gene causes a cell to kill itself through apoptosis. Non-limiting examples of suicide genes include viral thymidine kinase, cytosine deaminases, intracellular antibody against antioxidative enzymes (AOEs), bacterial nitroreductase, caspase and DNase.

In one aspect, the suicide gene is viral thymidine kinase (TK). Thymidine kinase is an ATP-thymidine 5'-phosphotransferase that converts deoxythymidine intodeoxythymidien 5'-monophosphate, which is further phosphorylated to deoxythymidine diphosphate and thereafter to deoxythymidine triphosphate by viral thymidine kinase and nucleoside diphosphate kinase respectively. Deoxythymidine triphosphate is incorporated into the synthesized DNA molecule by DNA polymerase. Some dNTP analogs, such as Ganciclovir (GCV), a synthetic analogue of 2'-deoxy-guanosine, have the ability to terminate the DNA synthesis upon their incorporation into synthesized DNA. Termination of synthesis triggers the apoptotic signaling cascades. While GCV is not recognized by human thymidine kinase, it is recognized as a substrate for some viral thymidine kinase, such as Herpes Simplex Virus-1 thymidine kinase (HSV-TK). As a result, a human cell expressing HSV-TK converts GCV into GCV phosphate, which is further phosphorylated and incorporated into the synthesized DNA, leading to the termination of synthesis and apoptosis. While the variant of HSV-TK is not limited, HSV-TK is, for example, TK007 (see Preuss et al., Hum Gene Ther. 2010 Aug; 21(8): 929-41).

In some aspects, the suicide gene is cytosine deaminase. Cytosine deaminase hydrolyze cytosine to uracile with release of ammonia. In physiological conditions, the modified site is recognized by endonucleases, then the phophodiester bond in the DNA is broken, initiating repair by incorporation of a new cytosine. However, cytosine deaminase can also converts 5-fluorocytosine into 5-fluorouracil (5-FU). Therefore, upon provision of non-toxic prodrug 5-FC, cytosine deaminase converts it into highly toxic 5-FU (a suicide inhibitor of thymidylate synthetase), leading to the inhibition of cell growth and apoptosis.

The term "vector," as used herein, is intended to refer to a nucleic acid molecule capable of transporting another nucleic acid to which it has been linked. One type of vector is a "plasmid," which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" can be used interchangeably as the plasmid is the most commonly used form of vector. However, also included are other forms of expression vectors, such as viral vectors (e.g., replication defective retroviruses, adenoviruses and adeno-associated viruses), which serve equivalent functions.

The term "recombinant host cell" (or simply "host cell"), as used herein, is intended to refer to a cell that comprises a nucleic acid that is not naturally present in the cell, and can be a cell into which a recombinant expression vector has been introduced. It should be understood that such terms are intended to refer not only to the particular subject cell but to the progeny of such a cell. Because certain modifications can occur in succeeding generations due to either mutation or environmental influences, such progeny cannot, in fact, be identical to the parent cell, but are still included within the scope of the term "host cell" as used herein.

An "immune response" is as understood in the art, and generally refers to a biological response within a vertebrate against foreign agents or abnormal, *e.g.*, cancerous cells, which response protects the organism against these agents and diseases caused by them. An immune response is mediated by the action of one or more cells of the immune system (for example, a T lymphocyte, B lymphocyte, natural killer (NK) cell, macrophage, eosinophil, mast cell, dendritic cell or neutrophil) and soluble macromolecules produced by any of these cells or the liver (including antibodies, cytokines, and complement) that results in selective targeting, binding to, damage to, destruction of, and/or elimination from the vertebrate's body of invading pathogens, cells or tissues infected with pathogens, cancerous or other abnormal cells, or, in cases of autoimmunity or pathological inflammation, normal human cells or tissues. An immune reaction includes, *e.g.,* activation or inhibition of a T cell, *e.g.,* an effector T cell, a Th cell, a CD4+ cell, a CD8+ T cell, or a Treg cell, or activation or inhibition of any other cell of the immune system, *e.g.*, NK cell.

"Immunotherapy" refers to the treatment of a subject afflicted with, or at risk of contracting or suffering a recurrence of, a disease by a method comprising inducing, enhancing, suppressing or otherwise modifying the immune system or an immune response.

As used herein, the terms "treat," "treatment," or "treatment of" when used in the context of treating a disease or condition in a subject, *e.g.,* a cancer, refer to reducing disease pathology, reducing or eliminating disease symptoms, promoting increased survival rates, and/or reducing discomfort. For example, treating can refer to the ability of a therapy when administered to a subject, to reduce one or more disease symptoms, signs, or causes. Treating also refers to mitigating or decreasing one or more clinical symptom and/or inhibition or delay in the progression of the condition and/or prevention or delay of the onset of a disease or illness.

As used herein, "cancer" refers a broad group of diseases characterized by the uncontrolled growth of abnormal cells in the body. Unregulated cell division can result in the formation of malignant tumors or cells that invade neighboring tissues and can metastasize to distant parts of the body through the lymphatic system or bloodstream.

As used herein, the term an "effective amount" or a "therapeutically effective amount" of an administered therapeutic substance, such as an immune cell comprising a polynucleotide encoding a CAR, is an amount sufficient to carry out a specifically stated or intended purpose, such as treating or treatment of cancer. An "effective amount" can be determined empirically in a routine manner in relation to the stated purpose.

As used herein, the terms "subject," "individual," or "patient," refer to any subject, particularly a mammalian subject, for whom diagnosis, prognosis, or therapy is desired. Mammalian subjects include, for example, humans, non-human primates, dogs, cats, guinea pigs, rabbits, rats, mice, horses, cattle, bears, and so on.

As used herein, the terms "ug" and "uM" are used interchangeably with "$\mu$g" and "$\mu$M," respectively.

Various aspects described herein are described in further detail in the following subsections.

## II. Compositions of the Disclosure

Some aspects of the present disclosure are directed to antigen-binding moieties that specifically bind human mesothelin. Some aspects of the present disclosure are directed to polynucleotides comprising a nucleotide sequence encoding a chimeric antigen receptor (CAR), wherein the CAR comprises an antigen-binding domain that binds human mesothelin. Some aspects of the present disclosure are directed to a host cell, *e.g.,* an immune cell, *e.g.,* an innate lymphoid cell, comprising a polynucleotide disclosed herein. Additional aspects of the present disclosure are directed to methods of treating a subject in need thereof comprising administering the polynucleotide and/or the cell to the subject.

According to a first aspect of the invention, there is provided an engineered innate lymphoid cell comprising a heterologous targeting construct specific for mesothelin.

Mesothelin, encoded by the *MSLN* gene located on human chromosome 16p.13.3, is a 40 kDa glycophosphatidylinositol (GPI) linked cell surface glycoprotein expressed by mesothelial cells. The MSLN gene encodes a 71-KD precursor that is cleaved into two products at arginine 295 (Arg295): a soluble 31-KD N-terminal protein called megakaryocyte potentiating factor (MPF) and mesothelin.

In one embodiment, the innate lymphoid cell is a natural killer (NK) cell. In an alternative embodiment, the innate lymphoid cell is a gamma delta ($\gamma\delta$) T cell. In a further embodiment, the $\gamma\delta$ T cell is a Vd1+ $\gamma\delta$ cell.

References herein to "heterologous targeting construct" relates to any targeting construct which is not normally expressed by the cell.

In one embodiment, the heterologous targeting construct is a chimeric antigen receptor (CAR). CARs are composed of a single-chain variable fragment (scFv) consisting of antibody variable heavy (VH) and variable light (VL) chains, fused by a peptide linker. This antigen-binding scFv of a CAR is joined to an intracellular CD3$\zeta$ signaling domain. First generation CARs contain CD3$\zeta$ alone, while second generation chimeric antigen receptors incorporate additional signaling domains such as CD28 or 4-1BB. Third generation CARs including multiple co-stimulatory signaling modules have also been reported.

In a further embodiment, the CAR is a full-length CAR or a non-signaling CAR. References herein to "full-length chimeric antigen receptor" or "full-length CAR" or "FL CAR" relate to CARs which comprise the intracellular signaling domain. References herein to "non-signaling chimeric antigen receptor", "non-signaling CAR", "naked chimeric antigen receptor", "naked CAR" or "NKD CAR" relate to CARs which lack the intracellular CD3ζ signaling domains typically present in a CAR.

In a further embodiment, the CAR is an armoured CAR. References herein to "armoured chimeric antigen receptor" or "armoured CAR" relates to CAR cells which have been engineered to secrete cytokines, or express ligands that are known to enhance or interact with endogenous immune cells such as dendritic cells (DCs), macrophages or regulatory T-cells (Treg cells). The 'armour' can enhance CAR cytotoxicity and specificity, evade immunosuppression, avoid host rejection, and prolong CAR therapeutic half-life.

**II.A. Chimeric Antigen Receptors**

Some aspects of the present disclosure are directed to the innate lymphoid cells (*e.g.*, a Vd1$^+$ γδ cell derived from blood or skin) comprising a heterologous nucleic acid encoding a CAR, wherein the CAR comprises an antigen-binding domain that specifically binds human mesothelin. In some aspects, the innate lymphoid cell is a blood-derived Vd1+ γδ cell comprising a heterologous nucleic acid molecule encoding a CAR, wherein the CAR comprises an antigen-binding domain that specifically binds human mesothelin (*e.g.*, wherein the antigen-binding domain comprises a sequence of any of SEQ ID NOs: 1 - 8, 41-48). In some aspects, the cells further comprise a 4-1BB and CD3 zeta domain. In still a further aspect, the cells are directed to a population of γδ T cells (*e.g.*, a population of Vd1$^+$ γδ cells derived from blood or skin) comprising γδ T cells comprising a heterologous nucleic acid encoding a CAR, wherein the CAR comprises an antigen-binding domain that specifically binds human mesothelin (*e.g.*, wherein the antigen-binding domain comprises a sequence of any of SEQ ID NOs: 1 - 8, 41-48), wherein the γδ T cells further comprise a CD8 transmembrane domain. In yet a further aspect, the cells are directed to a population of γδ T cells (*e.g.*, a population of Vd1$^+$ γδ cells derived from blood or skin) comprising γδ T cells comprising a heterologous nucleic acid encoding a CAR, wherein the CAR comprises an antigen-binding domain that specifically binds human mesothelin (*e.g.*, wherein the antigen-binding domain comprises a sequence of any of SEQ ID NOs: 1 - 8, 41-48), wherein the γδ T cells further comprise (a) an IL-15R-beta or (b)(i) IL-15R-alpha tethered with an IL-15 or variant thereof and (ii) IL-15R-beta . In a further aspect, the γδ T cells described herein comprise a heterologous nucleic acid encoding a CAR, wherein the CAR comprises an antigen-binding domain that specifically binds human mesothelin (*e.g.*, wherein the antigen-binding domain comprises a sequence of any of SEQ ID NOs: 1 - 8, 41-48) and may further comprise a 4-1BB and CD3 zeta domain and/or a CD8 transmembrane domain and (a) an IL-15R-beta or (b)(i) IL-15R-alpha tethered with an IL-15 or variant thereof and (ii) IL-15R-beta. In some aspects, the IL-2Rβ polypeptide is expressed without IL-15Rα and with tethering to an IL-15. In some aspects, the IL-15Rβ polypeptide is expressed without IL-2Rα and without tethering to an IL-15.

In some aspects, the antigen-binding domain that specifically binds human mesothelin comprises a variable heavy (VH) complementarity-determining region-1 (CDR1), a VH-CDR2, a VH-CDR3, a variable light (VL) CDR-1, a VL-CDR2, and a VL-CDR3. In some aspects, the VH-CDR3 comprises the amino acid sequence set forth in SEQ ID NO: 3. In some aspects, the VH-CDR2 comprises the amino acid sequence set forth in SEQ ID NO: 2. In some aspects, the VH-CDR1 comprises the amino acid sequence set forth in SEQ ID NO: 1. In some aspects, the VL-CDR1 comprises the amino acid sequence set forth in SEQ ID NO: 4. In some aspects, the VL-CDR2 comprises the amino acid sequence set forth in SEQ ID NO: 5. In some aspects, the VL-CDR3 comprises the amino acid sequence set forth in SEQ ID NO: 6.

In some aspects, the innate lymphoid cell is a blood-derived Vd1$^+$ γδ cell comprising a heterologous nucleic acid molecule encoding a CAR, wherein the CAR comprises an antigen-binding domain that specifically binds human mesothelin, and wherein the CAR comprises a VH-CDR1 comprising the amino acid sequence set forth in SEQ ID NO: 1; a VH-CDR2 comprising the amino acid sequence set forth in SEQ ID NO: 2; a VH-CDR3 comprising the amino acid sequence set forth in SEQ ID NO: 3; a VL-CDR1 comprising the amino acid sequence set forth in SEQ ID NO: 4; a VL-CDR2 comprising the amino acid sequence set forth in SEQ ID NO: 5; and a VL-CDR3 comprising the amino acid sequence set forth in SEQ ID NO: 6.

In some aspects, the innate lymphoid cell is a blood-derived Vd1$^+$ γδ cell comprising a heterologous nucleic acid molecule encoding a CAR, wherein the CAR comprises an antigen-binding domain that specifically binds human mesothelin, and wherein the CAR comprises (i) a VH comprising an amino acid sequence having at least about 75%, at least about 80%, at least about 85%, at least about 90%, at least about 95%, at least about 96%, at least about 97%, at least about 98%, or at least about 99% sequence identity with the amino acid sequence set forth in SEQ ID NO: 7, (ii) a VL comprising an amino acid sequence having at least about 75%, at least about 80%, at least about 85%, at least about 90%, at least about 95%, at least about 96%, at least about 97%, at least about 98%, or at least about 99% sequence identity with the amino acid sequence set forth in SEQ ID NO: 8, or (iii) both (i) and (ii).

In some aspects, the innate lymphoid cell is a blood-derived Vd1$^+$ γδ cell comprising a heterologous nucleic acid molecule encoding a CAR, wherein the CAR comprises an antigen-binding domain that specifically binds human mesothelin, and wherein the CAR comprises (i) a VH comprising the amino acid sequence set forth in SEQ ID NO: 7, (ii) a VL comprising the amino acid sequence set forth in SEQ ID NO: 8, or (iii) both (i) and (ii).

In some aspects, the antigen-binding domain that specifically binds human mesothelin comprises the P4 antigen-binding domain (Table 1).

Table 1: Antigen-Binding Domain Amino Acid Sequences

| P4 | |
|---|---|
| HC-CDR1 | GDSVSSNSAT (SEQ ID NO: 1) |
| HC-CDR2 | TYYRSKWYN (SEQ ID NO: 2) |
| HC-CDR3 | ARGMMTYYYGMDV (SEQ ID NO: 3) |
| LC-CDR1 | SGINVGPYR (SEQ ID NO: 4) |
| LC-CDR2 | YKSDSDK (SEQ ID NO: 5) |
| LC-CDR3 | MIWHSSAAV (SEQ ID NO: 6) |
| VH | QVQLQQSGPGLVTPSQTLSLTCAISGDSVSSNSATWNWIRQSPSRGLEWLGRTYYRSKWYND YAVSVKSRMSINPDTSKNQFSLQLNSVTPEDTAVYYCARGMMTYYYGMDVWGQGTTVTVSSG ILGS (SEQ ID NO: 7) |
| VL | QPVLTQSSSLSASPGASASLTCTLRSGINVGPYRIYWYQQKPGSPPQYLLNYKSDSDKQQGS GVPSRFSGSKDASANAGVLLISGLRSEDEADYYCMIWHSSAAVFGGGTQLTVLS (SEQ ID NO: 8) |
| YP218 | |
| HC-CDR1 | FYFYAC (SEQ ID NO: 41) |
| HC-CDR2 | CIYTAGSGSTYYASWAKG (SEQ ID NO: 42) |
| HC-CDR3 | TANTRSTYYLNLW (SEQ ID NO: 43) |
| LC-CDR1 | ASQRISSYLS (SEQ ID NO: 44) |
| LC-CDR2 | GASTLAS (SEQ ID NO: 45) |
| LC-CDR3 | SYAYFDSNNWHAF (SEQ ID NO: 46) |
| VH | MEVQLVESGGGLVQPGGSLRLSCAASGFDLGFYFYACWVRQAPGKGLEWVSCIYTAGSGSTY YASWAKGRFTISRDNSKNTLYLQMNSLRAEDTAVYYCARSTANTRSTYYLNLWGQGTLVTV (SEQ ID NO: 47) |
| VL | DIQMTQSPSSLSASVGDRVTITCQASQRISSYLSWYQQKPGKVPKLLIYGASTLASGVPSRF SGSGSGTDFTLTISSLQPEDVATYYCQSYAYFDSNNWHAFGGGTKVEI (SEQ ID NO: 48) |

In some aspects, the innate lymphoid cell is a blood-derived Vd1$^+$ γδ cell comprising a heterologous nucleic acid molecule encoding a CAR, wherein the CAR comprises an antigen-binding domain that specifically binds human mesothelin, and wherein the CAR comprises a VH-CDR1 comprising the amino acid sequence set forth in SEQ ID NO: 41; a VH-CDR2 comprising the amino acid sequence set forth in SEQ ID NO: 42; a VH-CDR3 comprising the amino acid sequence set forth in SEQ ID NO: 43; a VL-CDR1 comprising the amino acid sequence set forth in SEQ ID NO: 44; a VL-CDR2 comprising the amino acid sequence set forth in SEQ ID NO: 45; and a VL-CDR3 comprising the amino acid sequence set forth in SEQ ID NO: 46.

In some aspects, the innate lymphoid cell is a blood-derived Vd1$^+$ γδ cell comprising a heterologous nucleic acid molecule encoding a CAR, wherein the CAR comprises an antigen-binding domain that specifically binds human mesothelin, and wherein the CAR comprises (i) a VH comprising an amino acid sequence having at least about 75%, at least about 80%, at least about 85%, at least about 90%, at least about 95%, at least about 96%, at least about 97%, at least about 98%, or at least about 99% sequence identity with the amino acid sequence set forth in SEQ ID NO: 47, (ii) a VL comprising an amino acid sequence having at least about 75%, at least about 80%, at least about 85%, at least about 90%, at least about 95%, at least about 96%, at least about 97%, at least about 98%, or at least about 99% sequence identity with the amino acid sequence set forth in SEQ ID NO: 48, or (iii) both (i) and (ii).

In some aspects, the innate lymphoid cell is a blood-derived Vd1$^+$ $\gamma\delta$ cell comprising a heterologous nucleic acid molecule encoding a CAR, wherein the CAR comprises an antigen-binding domain that specifically binds human mesothelin, and wherein the CAR comprises (i) a VH comprising the amino acid sequence set forth in SEQ ID NO: 47, (ii) a VL comprising the amino acid sequence set forth in SEQ ID NO: 48, or (iii) both (i) and (ii).

### II.A.1. Hinge Region

In some aspects, the CAR further comprises a hinge region between the antigen-binding domain that specifically binds mesothelin and the transmembrane domain. In some aspects, the hinge is derived from an immunoglobulin (*e.g.*, derived from hinge regions or loop regions). In certain aspects, these hinges comprise, *e.g.*, IgA1, IgA2, IgG1, IgG2, IgG3, IgG4, IgD, IgE, or IgM hinge regions, fragments thereof (alone or capped by additional sequences, *e.g.*, CH1 or CH2 regions sequences), or combinations of fragments from IgA1, IgA2, IgG1, IgG2, IgG3, IgG4, IgD, IgE, or IgM hinge regions. In some aspects, the hinges comprise, *e.g.*, IgA1, IgA2, IgG1, IgG2, IgG3, IgG4, IgD, IgE, or IgM constant domain loop regions, fragments thereof (alone or capped by additional sequences, *e.g.*, from adjacent β-strands), or combinations of fragments from IgA1, IgA2, IgG1, IgG2, IgG3, IgG4, IgD, IgE, or IgM loop regions. In some aspects, the hinge of the present disclosure comprises hinge region derived sequences, loop region derived sequences, or combinations thereof.

In some aspects, the hinge region is selected from a CD8 hinge, a CD28 hinge, and an immunoglobulin hinge. In some aspects, the hinge comprises a CD8 hinge. In some aspects, the CAR comprises a hinge comprising the amino acid sequence set forth in SEQ ID NO: 9 (Table 2).

Table 2: CAR Sequences

| Element | Amino Acid Sequence |
|---|---|
| Hinge Region (CD8) | TTTPAPRPPTPAPTIASQPLSLRPEACRPAAGGAVHTRGL (SEQ ID NO: 9) |
| TM Domain (CD8) | DFACDIYIWAPLAGTCGVLLLSLVITLYC (SEQ ID NO: 10) |
| Costim. Region (4-1BB) | KRGRKKLLYIFKQPFMRPVQTTQEEDGCSCRFPEEEEGGCEL (SEQ ID NO: 11) |
| Intracellular signaling | RVKFSRSADAPAYQQGQNQLYNELNLGRREEYDVLDKRRGRDPEMG GKPRRKNPQEGLYNELQKDKMAEAYSEIGMKGERRRGKGHDGLYQ GLSTATKDTYDALHMQALPPR (SEQ ID NO: 12) |
| domain (CD3z) | |

Accordingly, in some aspects, the present disclosure provides a CAR (or a polynucleotide encoding a CAR), wherein the CAR comprises (i) an antigen-binding domain that specifically binds mesothelin (*e.g.*, anti-mesothelin scFv), (ii) a hinge region comprising the hinge region of CD8, (iii) a transmembrane domain, and (iv) an intracellular domain. In some aspects, the present disclosure provides a CAR (or a polynucleotide encoding a CAR), wherein the CAR comprises (i) an antigen-binding domain that specifically binds mesothelin (*e.g.*, anti-mesothelin scFv), (ii) a hinge region comprising the amino acid sequence set forth in SEQ ID NO: 9, (iii) a transmembrane domain, and (iv) an intracellular domain.

### II.A.2. Transmembrane Domain

In some aspects, the CAR further comprises a transmembrane domain. The transmembrane domain can be derived either from a natural or from a recombinant source. Where the source is natural, the domain can be derived from any membrane-bound or transmembrane protein. In some aspects, the transmembrane domain is capable of signaling to the intracellular domain(s) whenever the CAR of the present disclosure has bound to a target.

In some aspects, a transmembrane domain can include at least the transmembrane region(s) of, *e.g.,* CD8, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM(SLAMF1, CD150, IPO-3), BLAME (SLAMF8), SELPLG (CD162), LTBR, PAG/Cbp, NKG2D, NKG2C, or CD19.

In some aspects, the TM domain is derived from CD8, CD2, CD4, CD28, CD45, PD1, CD152, or any combination thereof. In some aspects, the TM domain is derived from CD8.

In some aspects, the TM domain comprises an amino acid sequence having at least about 60%, at least about 70%, at least about 80%, at least about 90%, at least about 95%, at least about 96%, at least about 97%, at least about 98%, or at least about 99% sequence identity to SEQ ID NO: 10. In certain aspects, the TM domain comprises the amino acid sequence set forth in SEQ ID NO: 10.

### II.A.3. Costimulatory Domain

In some aspects, the CAR further comprises a costimulatory domain. In some aspects, the costimulatory domain comprises a costimulatory domain of 4-1BB/CD137, interleukin-2 receptor (IL-2R), interleukin-12 receptor (IL-12R), IL-7, IL-21, IL-23, IL-15, CD2, CD3, CD4, CD7, CD8, CD27, CD28, CD30, CD40, ICOS, lymphocyte function-associated antigen-1 (LFA-1), LIGHT, NKG2C, OX40, DAP10, B7-H3, CD28 deleted for Lck binding (ICA), BTLA, GITR, HVEM, LFA-1, LIGHT, NKG2C, PD-1, TILR2, TILR4, TILR7, TILR9, Fc receptor gamma chain, Fc receptor $\varepsilon$ chain, a ligand that specifically binds with CD83, or any combination thereof. In some aspects, the CAR comprises a 4-1BB costimulatory domain.

In some aspects, the costimulatory domain comprises an amino acid sequence having at least about 60%, at least about 70%, at least about 80%, at least about 90%, at least about 95%, at least about 96%, at least about 97%, at least about 98%, or at least about 99% sequence identity to SEQ ID NO: 11. In certain aspects, the TM domain comprises the amino acid sequence set forth in SEQ ID NO: 11.

### II.A.4. Intracellular Signaling Domain

In some aspects, the CAR further comprises an intracellular signaling domain. In some aspects, the intracellular signaling domain comprises a CD3 $\zeta$ activating domain, a CD3$\delta$ activating domain, a CD3$\varepsilon$ activating domain, a CD3$\eta$ activating domain, a CD79A activating domain, a DAP 12 activating domain, a FCER1G activating domain, a DAP10/CD28 activating domain, a ZAP70 activating domain, or any combination thereof. In some aspects, the intracellular signaling domain comprises a CD3 $\zeta$ activating domain.

In some aspects, the intracellular signaling domain comprises an amino acid sequence having at least about 70%, at least about 75%, at least about 80%, at least about 85%, at least about 90%, at least about 95%, at least about 96%, at least about 97%, at least about 98%, or at least about 99% sequence identity to SEQ ID NO: 12. In certain aspects, the intracellular signaling domain comprises the sequence set forth in SEQ ID NO: 12.

In some aspects, the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a transmembrane domain; and (iii) an intracellular signaling domain. In some aspects, the CAR further comprises a costimulatory domain. In some aspects, the CAR further comprises a hinge region.

In some aspects, the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a transmembrane domain; and (iii) an intracellular signaling domain comprising a CD3 $\zeta$ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a transmembrane domain comprising an amino acid sequence having at least about 70%, at least about 75%, at least about 80%, at least about 85%, at least about 90%, at least about 95%, at least about 96%, at least about 97%, at least about 98%, or at least about 99% sequence identity to SEQ ID NO: 10; and (iii) an intracellular signaling domain comprising a CD3 $\zeta$ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a transmembrane domain comprising the amino acid sequence set forth in SEQ ID NO: 10; and (iii) an intracellular signaling domain comprising a CD3 $\zeta$ activating domain. In some aspects, the CAR further comprises a costimulatory domain. In some aspects, the CAR further comprises a hinge region.

In some aspects, the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a transmembrane domain; (iii) a costimulatory domain; and (iv) an intracellular signaling domain comprising a CD3 $\zeta$ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a transmembrane domain; (iii) a costimulatory domain comprising an amino acid sequence having at least about 70%, at least about 75%, at least about 80%, at least about 85%, at least about 90%, at least about 95%, at least about 96%, at least about 97%, at least about 98%, or at least about 99% sequence identity to SEQ ID NO: 11; and (iv) an intracellular signaling domain comprising a CD3 $\zeta$ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a transmembrane domain; (iii) a costimulatory domain comprising the amino acid sequence set forth in SEQ ID NO: 11; and (iv) an intracellular signaling domain comprising a CD3 $\zeta$ activating domain. In some aspects, the CAR further comprises a hinge region.

In some aspects, the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region; (iii) a transmembrane domain; (iv) a costimulatory domain; and (v) an intracellular signaling domain comprising a CD3 $\zeta$ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region comprising an amino acid sequence having at least about 70%, at least about 75%, at least about 80%, at least about 85%, at least about 90%, at least about 95%, at least about 96%, at least about 97%, at least about 98%, or at least about 99% sequence identity to SEQ ID NO: 9; (iii) a transmembrane domain; (iv) a costimulatory domain; and (v) an intracellular signaling domain comprising a CD3 $\zeta$ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region comprising the amino acid sequence set forth in SEQ ID NO: 9; (iii) a transmembrane domain; (iv) a costimulatory domain; and (v) an intracellular signaling domain comprising a CD3 $\zeta$ activating domain.

In some aspects, the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a CD8 hinge region; (iii) a CD8 transmembrane domain; (iv) a 4-1BB costimulatory domain; and (v) an intracellular signaling domain comprising a CD3 ζ activating domain.

In some aspects, the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region comprising an amino acid sequence having at least about 90%, at least about 95%, at least about 96%, at least about 98%, at least about 98%, or at least about 99% sequence identity to the sequence set forth in SEQ ID NO: 9; (iii) a transmembrane domain comprising an amino acid sequence having at least about 90%, at least about 95%, at least about 96%, at least about 98%, at least about 98%, or at least about 99% sequence identity to the sequence set forth in SEQ ID NO: 10; (iv) a costimulatory domain comprising an amino acid sequence having at least about 90%, at least about 95%, at least about 96%, at least about 98%, at least about 98%, or at least about 99% sequence identity to the sequence set forth in SEQ ID NO: 11; and (v) an intracellular signaling domain comprising an amino acid sequence having at least about 90%, at least about 95%, at least about 96%, at least about 98%, at least about 98%, or at least about 99% sequence identity to the sequence set forth in SEQ ID NO: 12.

In some aspects, the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region comprising the amino acid sequence set forth in SEQ ID NO: 9; (iii) a transmembrane domain comprising the amino acid sequence set forth in SEQ ID NO: 10; (iv) a costimulatory domain comprising the amino acid sequence set forth in SEQ ID NO: 11; and (v) an intracellular signaling domain comprising the amino acid sequence set forth in SEQ ID NO: 12.

In some aspects, the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a CD8 hinge region; (iii) a CD8 transmembrane domain; (iv) a 4-1BB costimulatory domain; and (v) an intracellular signaling domain comprising a CD3 ζ activating domain.

In some aspects, the CAR comprises (i) an antigen-binding domain that specifically binds human mesothelin, wherein the antigen-binding domain comprises a VH comprising an amino acid sequence having at least about 90%, at least about 95%, at least about 96%, at least about 98%, at least about 98%, or at least about 99% sequence identity to the amino acid sequence set forth in SEQ ID NO: 7, and a VL comprising an amino acid sequence having at least about 90%, at least about 95%, at least about 96%, at least about 98%, at least about 98%, or at least about 99% sequence identity to the amino acid sequence set forth in SEQ ID NO: 8; (ii) a hinge region comprising an amino acid sequence having at least about 90%, at least about 95%, at least about 96%, at least about 98%, at least about 98%, or at least about 99% sequence identity to SEQ ID NO: 9; (iii) a transmembrane domain comprising an amino acid sequence having at least about 90%, at least about 95%, at least about 96%, at least about 98%, at least about 98%, or at least about 99% sequence identity to the sequence set forth in SEQ ID NO: 10; (iv) a costimulatory domain comprising an amino acid sequence having at least about 90%, at least about 95%, at least about 96%, at least about 98%, at least about 98%, or at least about 99% sequence identity to the sequence set forth in SEQ ID NO: 11; and (v) an intracellular signaling domain comprising an amino acid sequence having at least about 90%, at least about 95%, at least about 96%, at least about 98%, at least about 98%, or at least about 99% sequence identity to the sequence set forth in SEQ ID NO: 12.

In some aspects, the CAR comprises (i) an antigen-binding domain that specifically binds human mesothelin, wherein the CAR comprises a VH comprising the amino acid sequence set forth in SEQ ID NO: 7 and a VL comprising the amino acid sequence set forth in SEQ ID NO: 8; (ii) a hinge region comprising the amino acid sequence set forth in SEQ ID NO: 9; (iii) a transmembrane domain comprising the amino acid sequence set forth in SEQ ID NO: 10; (iv) a costimulatory domain comprising the amino acid sequence set forth in SEQ ID NO: 11; and (v) an intracellular signaling domain comprising the amino acid sequence set forth in SEQ ID NO: 12.

In some aspects, the CAR comprises (i) an antigen-binding domain that specifically binds human mesothelin, wherein the antigen-binding domain comprises a VH comprising the amino acid sequence set forth in SEQ ID NO: 7 and a VL comprising the amino acid sequence set forth in SEQ ID NO: 8; (ii) a CD8 hinge region; (iii) a CD8 transmembrane domain; (iv) a 4-1BB costimulatory domain; and (v) an intracellular signaling domain comprising a CD3 ζ activating domain.

In some aspects, the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a transmembrane domain; and (iii) an intracellular signaling domain. In some aspects, the CAR further comprises a costimulatory domain. In some aspects, the CAR further comprises a hinge region.

In some aspects, the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a transmembrane domain; and (iii) an intracellular signaling domain comprising a CD3 $\zeta$ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a transmembrane domain comprising an amino acid sequence having at least about 70%, at least about 75%, at least about 80%, at least about 85%, at least about 90%, at least about 95%, at least about 96%, at least about 97%, at least about 98%, or at least about 99% sequence identity to SEQ ID NO: 10; and (iii) an intracellular signaling domain comprising a CD3 $\zeta$ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a transmembrane domain comprising the amino acid sequence set forth in SEQ ID NO: 10; and (iii) an intracellular signaling domain comprising a CD3 $\zeta$ activating domain. In some aspects, the CAR further comprises a costimulatory domain. In some aspects, the CAR further comprises a hinge region.

In some aspects, the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a transmembrane domain; (iii) a costimulatory domain; and (iv) an intracellular signaling domain comprising a CD3 $\zeta$ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a transmembrane domain; (iii) a costimulatory domain comprising an amino acid sequence having at least about 70%, at least about 75%, at least about 80%, at least about 85%, at least about 90%, at least about 95%, at least about 96%, at least about 97%, at least about 98%, or at least about 99% sequence identity to SEQ ID NO: 11; and (iv) an intracellular signaling domain comprising a CD3 $\zeta$ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a transmembrane domain; (iii) a costimulatory domain comprising the amino acid sequence set forth in SEQ ID NO: 11; and (iv) an intracellular signaling domain comprising a CD3 $\zeta$ activating domain. In some aspects, the CAR further comprises a hinge region.

In some aspects, the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a hinge region; (iii) a transmembrane domain; (iv) a costimulatory domain; and (v) an intracellular signaling domain comprising a CD3 ζ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a hinge region comprising an amino acid sequence having at least about 70%, at least about 75%, at least about 80%, at least about 85%, at least about 90%, at least about 95%, at least about 96%, at least about 97%, at least about 98%, or at least about 99% sequence identity to SEQ ID NO: 9; (iii) a transmembrane domain; (iv) a costimulatory domain; and (v) an intracellular signaling domain comprising a CD3 ζ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a hinge region comprising the amino acid sequence set forth in SEQ ID NO: 9; (iii) a transmembrane domain; (iv) a costimulatory domain; and (v) an intracellular signaling domain comprising a CD3 ζ activating domain.

In some aspects, the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a CD8 hinge region; (iii) a CD8 transmembrane domain; (iv) a 4-1BB costimulatory domain; and (v) an intracellular signaling domain comprising a CD3 ζ activating domain.

In some aspects, the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a hinge region comprising an amino acid sequence having at least about 90%, at least about 95%, at least about 96%, at least about 98%, at least about 98%, or at least about 99% sequence identity to the sequence set forth in SEQ ID NO: 9; (iii) a transmembrane domain comprising an amino acid sequence having at least about 90%, at least about 95%, at least about 96%, at least about 98%, at least about 98%, or at least about 99% sequence identity to the sequence set forth in SEQ ID NO: 10; (iv) a costimulatory domain comprising an amino acid sequence having at least about 90%, at least about 95%, at least about 96%, at least about 98%, at least about 98%, or at least about 99% sequence identity to the sequence set forth in SEQ ID NO: 11; and (v) an intracellular signaling domain comprising an amino acid sequence having at least about 90%, at least about 95%, at least about 96%, at least about 98%, at least about 98%, or at least about 99% sequence identity to the sequence set forth in SEQ ID NO: 12.

In some aspects, the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a hinge region comprising the amino acid sequence set forth in SEQ ID NO: 9; (iii) a transmembrane domain comprising the amino acid sequence set forth in SEQ ID NO: 10; (iv) a costimulatory domain comprising the amino acid sequence set forth in SEQ ID NO: 11; and (v) an intracellular signaling domain comprising the amino acid sequence set forth in SEQ ID NO: 12.

In some aspects, the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a CD8 hinge region; (iii) a CD8 transmembrane domain; (iv) a 4-1BB costimulatory domain; and (v) an intracellular signaling domain comprising a CD3 ζ activating domain.

In some aspects, the CAR comprises (i) an antigen-binding domain that specifically binds human mesothelin, wherein the antigen-binding domain comprises a VH comprising an amino acid sequence having at least about 90%, at least about 95%, at least about 96%, at least about 98%, at least about 98%, or at least about 99% sequence identity to the amino acid sequence set forth in SEQ ID NO: 47, and a VL comprising an amino acid sequence having at least about 90%, at least about 95%, at least about 96%, at least about 98%, at least about 98%, or at least about 99% sequence identity to the amino acid sequence set forth in SEQ ID NO: 48; (ii) a hinge region comprising an amino acid sequence having at least about 90%, at least about 95%, at least about 96%, at least about 98%, at least about 98%, or at least about 99% sequence identity to SEQ ID NO: 9; (iii) a transmembrane domain comprising an amino acid sequence having at least about 90%, at least about 95%, at least about 96%, at least about 98%, at least about 98%, or at least about 99% sequence identity to the sequence set forth in SEQ ID NO: 10; (iv) a costimulatory domain comprising an amino acid sequence having at least about 90%, at least about 95%, at least about 96%, at least about 98%, at least about 98%, or at least about 99% sequence identity to the sequence set forth in SEQ ID NO: 11; and (v) an intracellular signaling domain comprising an amino acid sequence having at least about 90%, at least about 95%, at least about 96%, at least about 98%, at least about 98%, or at least about 99% sequence identity to the sequence set forth in SEQ ID NO: 12.

In some aspects, the CAR comprises (i) an antigen-binding domain that specifically binds human mesothelin, wherein the CAR comprises a VH comprising the amino acid sequence set forth in SEQ ID NO: 47 and a VL comprising the amino acid sequence set forth in SEQ ID NO: 48; (ii) a hinge region comprising the amino acid sequence set forth in SEQ ID NO: 9; (iii) a transmembrane domain comprising the amino acid sequence set forth in SEQ ID NO: 10; (iv) a costimulatory domain comprising the amino acid sequence set forth in SEQ ID NO: 11; and (v) an intracellular signaling domain comprising the amino acid sequence set forth in SEQ ID NO: 12.

In some aspects, the CAR comprises (i) an antigen-binding domain that specifically binds human mesothelin, wherein the antigen-binding domain comprises a VH comprising the amino acid sequence set forth in SEQ ID NO: 47 and a VL comprising the amino acid sequence set forth in SEQ ID NO: 48; (ii) a CD8 hinge region; (iii) a CD8 transmembrane domain; (iv) a 4-1BB costimulatory domain; and (v) an intracellular signaling domain comprising a CD3 $\zeta$ activating domain.

### II.A.4. Armoring

In some aspects, a modified immune cell disclosed herein (e.g., an NK cell or a $\gamma\delta$ T cell expressing a CAR of the present disclosure) further expresses an armor protein. In some aspects, the armor protein increases the persistence of the modified immune cell, relative to a similarly modified immune cell that does not express the armor protein. In some aspects, the armor protein increases the proliferation of the modified immune cell, relative to a similarly modified immune cell that does not express the armor protein. In some aspects, the armor protein increases the cytotoxicity of the modified immune cell, relative to a similarly modified immune cell that does not express the armor protein.

In some aspects, the armor protein comprises a membrane-bound polypeptide. In some aspects, the armor proteins comprises a membrane-bound receptor. In some aspects, the membrane-bound receptor comprises IL-15R (e.g., IL-15R$\beta$ and IL-15R$\alpha$). In some aspects, the membrane-bound receptor comprises IL-2R (e.g., IL-2R$\beta$). In some aspects, the membrane-bound receptor comprises $\alpha\beta$ TCR. In some aspects, the membrane-bound receptor comprises a natural cytotoxicity receptor (e.g., NKp30, NKp44, or NKp46). In some aspects, the membrane-bound receptor comprises a cytokine receptor (e.g., IL-12 receptor). In some aspects, the membrane-bound receptor comprises a chemokine receptor (e.g., CCR2 receptor). In some aspects, the armor protein comprises a membrane-bound ligand or cytokine. In some aspects, the membrane-bound ligand or cytokine comprises membrane-bound IL-15, membrane-bound IL-7, membrane-bound CD40L, membrane-bound 4-1BB, membrane-bound 4-1BBL, membrane bound CCL19, or any combination thereof.

In some aspects, the armor protein comprises a soluble polypeptide. In some aspects, the soluble protein comprises a soluble ligand. In some aspects, the soluble protein comprises a cytokine. In some aspects, the armor protein comprises soluble IL-15, soluble IL-7, soluble IL-12, soluble CD40L, soluble 4-1BBL, soluble CCL19, or any combination thereof.

In some aspects, the armor protein comprises a membrane-bound polypeptide and a soluble polypeptide.

In some aspects, the armor protein comprises an IL-2R$\beta$ polypeptide. In some aspects, the armor protein comprises an amino acid sequence having at least about 70%, at least about 75%, at least about 80%, at least about 85%, at least about 90%, at least about 95%, at least about 96%, at least about 97%, at least about 98%, at least about 99% sequence identity to the amino acid sequence set forth in SEQ ID NO: 15 (Table 3). In some aspects, the armor protein comprises the amino acid sequence set forth in SEQ ID NO: 15. In some aspects, the armor protein further comprises a signal peptide, wherein the signal peptide is cleaved post translation. Any signal peptide can be used. In some aspects, the armor protein comprises an IL-2R$\beta$ polypeptide, and the armor protein does not comprise an IL-15R$\alpha$ polypeptide or a construct comprising an IL-15R$\alpha$ polypeptide tethered to an IL-15 polypeptide.

Table 3: Armoring Proteins

| Armoring Protein | Sequence |
|---|---|
| IL-2Rβ | AVNGTSQFTCFYNSRANISCVWSQDGALQDTSCQVHAWPDRRRWNQTCE LLPVSQASWACNLILGAPDSQKLTTVDIVTLRVLCREGVRWRVMAIQDF KPFENLRLMAPISLQVVHVETHRCNISWEISQASHYFERHLEFEARTLS PGHTWEEAPLLTLKQKQEWICLETLTPDTQYEFQVRVKPLQGEFTTWSP WSQPLAFRTKPAALGKDTIPWLGHLLVGLSGAFGFIILVYLLINCRNTG PWLKKVLKCNTPDPSKFFSQLSSEHGGDVQKWLSSPFPSSSFSPGGLAP EISPLEVLERDKVTQLLLQQDKVPEPASLSSNHSLTSCFTNQGYFFFHL PDALEIEACQVYFTYDPYSEEDPDEGVAGAPTGSSPQPLQPLSGEDDAY CTFPSRDDLLLFSPSLLGGPSPPSTAPGGSGAGEERMPPSLQERVPRDW DPQPLGPPTPGVPDLVDFQPPPELVLREAGEEVPDAGPREGVSFPWSRP PGQGEFRALNARLPLNTDAYLSLQELQGQDPTHLV (SEQ ID NO: 15) |
| IL-15 | NWVNVISDLKKIEDLIQSMHIDATLYTESDVHPSCKVTAMKCFLLELQV ISLESGDASIHDTVENLIILANNSLSSNGNVTESGCKECEELEEKNIKE FLQSFVHIVQMFINTS (SEQ ID NO: 16) |
| IL-15Rα | ITCPPPMSVEHADIWVKSYSLYSRERYICNSGFKRKAGTSSLTECVLNK ATNVAHWTTPSLKCIRDPALVHQRPAPPSTVTTAGVTPQPESLSPSGKE PAASSPSSNNTAATTAAIVPGSQLMPSKSPSTGTTEISSHESSHGTPSQ TTAKNWELTASASHQPPGVYPQGHSDTTVAISTSTVLLCGLSAVSLLAC YLKSRQTPPLASVEMEAMEALPVTWGTSSRDEDLENCSHHL (SEQ ID NO: 17) |
| P2A Linker | GSGATNFSLLKQAGDVEENPGP (SEQ ID NO: 18) |
| Gly-Ser Linker | GGGGS (SEQ ID NO: 19) |
| Gly-Ser Linker | GGGGSGGGGS (SEQ ID NO: 20) |
| Gly-Ser Linker | GGGGSGGGGSGGGGS (SEQ ID NO: 21) |
| Gly-Ser Linker | GGGGSGGGGSGGGGSGGGGS (SEQ ID NO: 22) |
| Gly-Ser Linker | GGGGSGGGGSGGGGSGGGGSGGGS (SEQ ID NO: 23) |
| Gly-Ser Linker | GGGGSGGGGSGGGGSGGGS (SEQ ID NO: 24) |
| Gly-Ser Linker | GGGGSGGGGSGGGS (SEQ ID NO: 25) |

In some aspects, the armor protein comprises an IL-15Rα polypeptide. In some aspects, the armor protein comprises a construct comprising an IL-15Rα polypeptide tethered to an IL-15 polypeptide. In some aspects, the armor protein comprises (i) an IL-2Rβ polypeptide and (ii) an IL-15Rα polypeptide tethered to an IL-15 polypeptide. In some aspects, the armor protein comprises (i) an IL-2Rβ polypeptide and (ii) a construct comprising an IL-15Rα polypeptide tethered to an IL-15 polypeptide.

In some aspects, the IL-15Rα polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 17. In some aspects, the IL-15Rα polypeptide comprises the amino acid sequence set forth in SEQ ID NO: 17. In some aspects, the IL-15Rα polypeptide comprises a signal peptide sequence.

In some aspects the IL-15 polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 16. In some aspects, the IL-15 polypeptide comprises the amino acid sequence set forth in SEQ ID NO: 16. In some aspects, the IL-15 polypeptide comprises a signal peptide sequence.

In some aspects, the IL-15Rα polypeptide is tethered to the IL-15 polypeptide by a linker. In some aspects, the linker comprises one or more peptide bonds. In some aspects, the linker comprises a peptide linker. In some aspects, the peptide linker is a flexible linker. In some aspects, the linker is a rigid linker. In some aspects, the linker is a cleavable linker. In some aspects, the linker is a Gly-Ser linker. In some aspects, the linker comprises one or more repeats of the sequence GGGS, GGGS (SEQ ID NO: 19), or a combination thereof. In some aspects, the IL-15Rα polypeptide is tethered to the IL-15 polypeptide by a linker comprising the amino acid sequence set forth in SEQ ID NO: 20. In some aspects, the IL-15Rα polypeptide is tethered to the IL-15 polypeptide by a linker comprising the amino acid sequence set forth in SEQ ID NO: 21. In some aspects, the IL-15Rα polypeptide is tethered to the IL-15 polypeptide by a linker comprising the amino acid sequence set forth in SEQ ID NO: 22. In some aspects, the IL-15Rα polypeptide is tethered to the IL-15 polypeptide by a linker comprising the amino acid sequence set forth in SEQ ID NO: 23. In some aspects, the IL-15Rα polypeptide is tethered to the IL-15 polypeptide by a linker comprising the amino acid sequence set forth in SEQ ID NO: 24. In some aspects, the IL-15Rα polypeptide is tethered to the IL-15 polypeptide by a linker comprising the amino acid sequence set forth in SEQ ID NO: 25.

In some aspects, the construct comprising the IL-15Rα polypeptide is tethered to the IL-15 polypeptide is arranged from N-terminal to C-terminal according to the following order: (i) N-terminus, (ii) the IL-15 polypeptide, (iii) a peptide linker disclosed herein *(e.g.,* a polypeptide comprising GGGS, GGGS (SEQ ID NO: 19), or a combination thereof; (iv) the IL-15Rα polypeptide; (v) C-terminus. In some aspects, the construct comprising the IL-15Rα polypeptide is tethered to the IL-15 polypeptide is arranged from N-terminal to C-terminal according to the following order: (i) N-terminus, (ii) an IL-15 polypeptide comprising the amino acid sequence set forth in SEQ ID NO: 16, (iii) a peptide linker disclosed herein *(e.g.,* a polypeptide comprising GGGS, GGGS (SEQ ID NO: 19), or a combination thereof; (iv) an IL-15Rα polypeptide comprising the amino acid sequence set forth in SEQ ID NO: 17; (v) C-terminus.

In some aspects, the armor protein is expressed as a single polypeptide. In some aspects, the armor protein is expressed as a single polypeptide comprising (i) an IL-2Rβ polypeptide; (ii) a linker; and (iii) an IL-15Rα polypeptide tethered to an IL-15 polypeptide. In some aspects, the armor protein is expressed as a single polypeptide comprising (i) an IL-2Rβ polypeptide; (ii) a cleavable linker; and (iii) an IL-15Rα polypeptide tethered to an IL-15 polypeptide. In some aspects, the cleavable linker comprises a P2A sequence. In some aspects the linker comprises an amino acid sequence having at least about 80%, at least about 85%, at least about 90%, at least about 95%, at least about 96%, at least about 97%, at least about 98%, or at least about 99% sequence identity to the amino acid sequence set forth in SEQ ID NO: 18. In some aspects, the armor protein is expressed as a single polypeptide comprising (i) an IL-2Rβ polypeptide; (ii) a linker comprising the amino acid sequence set forth in SEQ ID NO: 18; and (iii) an IL-15Rα polypeptide tethered to an IL-15 polypeptide.

In some aspects, the armor protein is expressed as a single polypeptide comprising (i) an IL-2Rβ polypeptide comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 15; (ii) a linker comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 18; and (iii) an IL-15Rα polypeptide tethered to an IL-15 polypeptide; wherein the IL-15Rα polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 17; and wherein the IL-15 polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 16.

In some aspects, the armor protein is expressed as a single polypeptide comprising (i) an IL-2Rβ polypeptide comprising the amino acid sequence set forth in SEQ ID NO: 15; (ii) a linker comprising the amino acid sequence set forth in SEQ ID NO: 18; and (iii) an IL-15Rα polypeptide tethered to an IL-15 polypeptide; wherein the IL-15Rα polypeptide comprises the amino acid sequence set forth in SEQ ID NO: 17; and wherein the IL-15 polypeptide comprises the amino acid sequence set forth in SEQ ID NO: 16.

**II.B. Nucleic Acid Molecules**

Some aspects of the present disclosure are directed to a polynucleotide or a set of polynucleotides encoding a CAR disclosed herein, *i.e.,* a CAR that specifically binds mesothelin. In some aspects, the polynucleotide or the set of polynucleotides encodes a CAR, wherein the CAR comprises an antigen-binding domain that specifically binds human mesothelin, and wherein the CAR comprises a VH-CDR1 comprising the amino acid sequence set forth in SEQ ID NO: 1; a VH-CDR2 comprising the amino acid sequence set forth in SEQ ID NO: 2; a VH-CDR3 comprising the amino acid sequence set forth in SEQ ID NO: 3; a VL-CDR1 comprising the amino acid sequence set forth in SEQ ID NO: 4; a VL-CDR2 comprising the amino acid sequence set forth in SEQ ID NO: 5; and a VL-CDR3 comprising the amino acid sequence set forth in SEQ ID NO: 6.

In some aspects, the polynucleotide or the set of polynucleotides encodes a CAR, wherein the CAR comprises an antigen-binding domain that specifically binds human mesothelin, and wherein the CAR comprises a VH comprising an amino acid sequence having at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 7. In some aspects, the polynucleotide or the set of polynucleotides encodes a CAR, wherein the CAR comprises an antigen-binding domain that specifically binds human mesothelin, and wherein the CAR comprises a VH comprising the amino acid sequence set forth in SEQ ID NO: 7.

In some aspects, the polynucleotide or the set of polynucleotides encodes a CAR, wherein the CAR comprises an antigen-binding domain that specifically binds human mesothelin, and wherein the CAR comprises a VL comprising an amino acid sequence having at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 8. In some aspects, the polynucleotide or the set of polynucleotides encodes a CAR, wherein the CAR comprises an antigen-binding domain that specifically binds human mesothelin, and wherein the CAR comprises a VL comprising the amino acid sequence set forth in SEQ ID NO: 8.

In some aspects, the polynucleotide or the set of polynucleotides encodes a CAR, wherein the CAR comprises (i) an antigen-binding domain that specifically binds human mesothelin, and wherein the CAR comprises a VH comprising an amino acid sequence having at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 7; and (ii) a VL comprising an amino acid sequence having at least about 75%, at least about 80%, at least about 85%, at least about 90%, at least about 95%, at least about 96%, at least about 97%, at least about 98%, or at least about 99% sequence identity with the amino acid sequence set forth in SEQ ID NO: 8.

In some aspects, the polynucleotide or the set of polynucleotides encodes a CAR, wherein the CAR comprises an antigen-binding domain that specifically binds human mesothelin, and wherein the CAR comprises (i) a VH comprising the amino acid sequence set forth in SEQ ID NO: 7 and (ii) a VL comprising the amino acid sequence set forth in SEQ ID NO: 8.

Some aspects of the present disclosure are directed to a polynucleotide or a set of polynucleotides encoding a CAR disclosed herein, *i.e.,* a CAR that specifically binds mesothelin. In some aspects, the polynucleotide or the set of polynucleotides encodes a CAR, wherein the CAR comprises an antigen-binding domain that specifically binds human mesothelin, and wherein the CAR comprises a VH-CDR1 comprising the amino acid sequence set forth in SEQ ID NO: 41; a VH-CDR2 comprising the amino acid sequence set forth in SEQ ID NO: 42; a VH-CDR3 comprising the amino acid sequence set forth in SEQ ID NO: 43; a VL-CDR1 comprising the amino acid sequence set forth in SEQ ID NO: 44; a VL-CDR2 comprising the amino acid sequence set forth in SEQ ID NO: 45; and a VL-CDR3 comprising the amino acid sequence set forth in SEQ ID NO: 46.

In some aspects, the polynucleotide or the set of polynucleotides encodes a CAR, wherein the CAR comprises an antigen-binding domain that specifically binds human mesothelin, and wherein the CAR comprises a VH comprising an amino acid sequence having at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 47. In some aspects, the polynucleotide or the set of polynucleotides encodes a CAR, wherein the CAR comprises an antigen-binding domain that specifically binds human mesothelin, and wherein the CAR comprises a VH comprising the amino acid sequence set forth in SEQ ID NO: 47.

In some aspects, the polynucleotide or the set of polynucleotides encodes a CAR, wherein the CAR comprises an antigen-binding domain that specifically binds human mesothelin, and wherein the CAR comprises a VL comprising an amino acid sequence having at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 48. In some aspects, the polynucleotide or the set of polynucleotides encodes a CAR, wherein the CAR comprises an antigen-binding domain that specifically binds human mesothelin, and wherein the CAR comprises a VL comprising the amino acid sequence set forth in SEQ ID NO: 48.

In some aspects, the polynucleotide or the set of polynucleotides encodes a CAR, wherein the CAR comprises (i) an antigen-binding domain that specifically binds human mesothelin, and wherein the CAR comprises a VH comprising an amino acid sequence having at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 47; and (ii) a VL comprising an amino acid sequence having at least about 75%, at least about 80%, at least about 85%, at least about 90%, at least about 95%, at least about 96%, at least about 97%, at least about 98%, or at least about 99% sequence identity with the amino acid sequence set forth in SEQ ID NO: 48.

In some aspects, the polynucleotide or the set of polynucleotides encodes a CAR, wherein the CAR comprises an antigen-binding domain that specifically binds human mesothelin, and wherein the CAR comprises (i) a VH comprising the amino acid sequence set forth in SEQ ID NO: 47 and (ii) a VL comprising the amino acid sequence set forth in SEQ ID NO: 48.

In some aspects, the polynucleotide or the set of polynucleotides encodes a CAR, wherein the CAR comprises (i) an antigen-binding domain that specifically binds mesothelin, as disclosed herein; (ii) a transmembrane domain disclosed herein; and (iii) an intracellular signaling domain disclosed herein. In some aspects, the polynucleotide or the set of polynucleotides encodes a CAR, wherein the CAR comprises (i) an antigen-binding domain that specifically binds mesothelin, as disclosed herein; (ii) a CD8 transmembrane domain; and (iii) an intracellular signaling domain disclosed herein. In some aspects, the polynucleotide or the set of polynucleotides encodes a CAR, wherein the CAR comprises (i) an antigen-binding domain that specifically binds mesothelin, as disclosed herein; (ii) a transmembrane domain disclosed herein; and (iii) a CD3ζ intracellular signaling domain. In some aspects, the polynucleotide or the set of polynucleotides encodes a CAR, wherein the CAR comprises (i) an antigen-binding domain that specifically binds mesothelin, as disclosed herein; (ii) a CD8 transmembrane domain; and (iii) a CD3ζ intracellular signaling domain.

In some aspects, the polynucleotide or the set of polynucleotides encodes a CAR, wherein the CAR comprises (i) an antigen-binding domain disclosed herein; (ii) a transmembrane domain comprising an amino acid sequence having at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 10; and (iii) an intracellular signaling domain disclosed herein. In some aspects, the polynucleotide or the set of polynucleotides encodes a CAR, wherein the CAR comprises (i) an antigen-binding domain disclosed herein; (ii) a transmembrane domain disclosed herein; and (iii) an intracellular signaling domain comprising an amino acid sequence having at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 12. In some aspects, the polynucleotide or the set of polynucleotides encodes a CAR, wherein the CAR comprises (i) an antigen-binding domain disclosed herein; (ii) a transmembrane domain comprising an amino acid sequence having at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 10; and (iii) an intracellular signaling domain comprising an amino acid sequence having at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 12.

In some aspects, the polynucleotide or the set of polynucleotides encodes a CAR, wherein the CAR further comprises a hinge region disclosed herein. In some aspects, the hinge region comprises a CD8 hinge region. In some aspects, the hinge region comprises an amino acid sequence having at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 9. In some aspects, the hinge region comprises the amino acid sequence set forth in SEQ ID NO: 9.

In some aspects, the polynucleotide or the set of polynucleotides encodes a CAR, wherein the CAR further comprises a costimulatory region disclosed herein. In some aspects, the costimulatory region comprises a 4-1BB costimulatory region. In some aspects, the costimulatory region comprises an amino acid sequence having at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 11. In some aspects, the costimulatory region comprises the amino acid sequence set forth in SEQ ID NO: 11.

In some aspects, the polynucleotide or the set of polynucleotides encodes a CAR, wherein the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a transmembrane domain; (iii) a costimulatory domain; and (iv) an intracellular signaling domain comprising a CD3 ζ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a transmembrane domain; (iii) a costimulatory domain comprising an amino acid sequence having at least about 70%, at least about 75%, at least about 80%, at least about 85%, at least about 90%, at least about 95%, at least about 96%, at least about 97%, at least about 98%, or at least about 99% sequence identity to SEQ ID NO: 11; and (iv) an intracellular signaling domain comprising a CD3 ζ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a transmembrane domain; (iii) a costimulatory domain comprising the amino acid sequence set forth in SEQ ID NO: 11; and (iv) an intracellular signaling domain comprising a CD3 $\zeta$ activating domain. In some aspects, the CAR further comprises a hinge region.

In some aspects, the polynucleotide or the set of polynucleotides encodes a CAR, wherein the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region; (iii) a transmembrane domain; (iv) a costimulatory domain; and (v) an intracellular signaling domain comprising a CD3 $\zeta$ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region comprising an amino acid sequence having at least about 70%, at least about 75%, at least about 80%, at least about 85%, at least about 90%, at least about 95%, at least about 96%, at least about 97%, at least about 98%, or at least about 99% sequence identity to SEQ ID NO: 9; (iii) a transmembrane domain; (iv) a costimulatory domain; and (v) an intracellular signaling domain comprising a CD3 $\zeta$ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region comprising the amino acid sequence set forth in SEQ ID NO: 9; (iii) a transmembrane domain; (iv) a costimulatory domain; and (v) an intracellular signaling domain comprising a CD3 $\zeta$ activating domain.

In some aspects, the polynucleotide or the set of polynucleotides encodes a CAR, wherein the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a CD8 hinge region; (iii) a CD8 transmembrane domain; (iv) a 4-1BB costimulatory domain; and (v) an intracellular signaling domain comprising a CD3 $\zeta$ activating domain.

In some aspects, the polynucleotide or the set of polynucleotides encodes a CAR, wherein the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region comprising an amino acid sequence having at least about 90%, at least about 95%, at least about 96%, at least about 98%, at least about 98%, or at least about 99% sequence identity to the sequence set forth in SEQ ID NO: 9; (iii) a transmembrane domain comprising an amino acid sequence having at least about 90%, at least about 95%, at least about 96%, at least about 98%, at least about 98%, or at least about 99% sequence identity to the sequence set forth in SEQ ID NO: 10; (iv) a costimulatory domain comprising an amino acid sequence having at least about 90%, at least about 95%, at least about 96%, at least about 98%, at least about 98%, or at least about 99% sequence identity to the sequence set forth in SEQ ID NO: 11; and (v) an intracellular signaling domain comprising an amino acid sequence having at least about 90%, at least about 95%, at least about 96%, at least about 98%, at least about 98%, or at least about 99% sequence identity to the sequence set forth in SEQ ID NO: 12.

In some aspects, the polynucleotide or the set of polynucleotides encodes a CAR, wherein the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region comprising the amino acid sequence set forth in SEQ ID NO: 9; (iii) a transmembrane domain comprising the amino acid sequence set forth in SEQ ID NO: 10; (iv) a costimulatory domain comprising the amino acid sequence set forth in SEQ ID NO: 11; and (v) an intracellular signaling domain comprising the amino acid sequence set forth in SEQ ID NO: 12.

In some aspects, the polynucleotide of the set of polynucleotides encodes a CAR, wherein the CAR comprises (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a CD8 hinge region; (iii) a CD8 transmembrane domain; (iv) a 4-1BB costimulatory domain; and (v) an intracellular signaling domain comprising a CD3 $\zeta$ activating domain.

In some aspects, the polynucleotide of the set of polynucleotides encodes a CAR, wherein the CAR comprises (i) an antigen-binding domain that specifically binds human mesothelin, wherein the antigen-binding domain comprises a VH comprising an amino acid sequence having at least about 90%, at least about 95%, at least about 96%, at least about 98%, at least about 98%, or at least about 99% sequence identity to the amino acid sequence set forth in SEQ ID NO: 7, and a VL comprising an amino acid sequence having at least about 90%, at least about 95%, at least about 96%, at least about 98%, at least about 98%, or at least about 99% sequence identity to the amino acid sequence set forth in SEQ ID NO: 8; (ii) a hinge region comprising an amino acid sequence having at least about 90%, at least about 95%, at least about 96%, at least about 98%, at least about 98%, or at least about 99% sequence identity to SEQ ID NO: 9; (iii) a transmembrane domain comprising an amino acid sequence having at least about 90%, at least about 95%, at least about 96%, at least about 98%, at least about 98%, or at least about 99% sequence identity to the sequence set forth in SEQ ID NO: 10; (iv) a costimulatory domain comprising an amino acid sequence having at least about 90%, at least about 95%, at least about 96%, at least about 98%, at least about 98%, or at least about 99% sequence identity to the sequence set forth in SEQ ID NO: 11; and (v) an intracellular signaling domain comprising an amino acid sequence having at least about 90%, at least about 95%, at least about 96%, at least about 98%, at least about 98%, or at least about 99% sequence identity to the sequence set forth in SEQ ID NO: 12.

In some aspects, the polynucleotide of the set of polynucleotides encodes a CAR, wherein the CAR comprises (i) an antigen-binding domain that specifically binds human mesothelin, wherein the CAR comprises a VH comprising the amino acid sequence set forth in SEQ ID NO: 7 and a VL comprising the amino acid sequence set forth in SEQ ID NO: 8; (ii) a hinge region comprising the amino acid sequence set forth in SEQ ID NO: 9; (iii) a transmembrane domain comprising the amino acid sequence set forth in SEQ ID NO: 10; (iv) a costimulatory domain comprising the amino acid sequence set forth in SEQ ID NO: 11; and (v) an intracellular signaling domain comprising the amino acid sequence set forth in SEQ ID NO: 12.

In some aspects, the polynucleotide of the set of polynucleotides encodes a CAR, wherein the CAR comprises (i) an antigen-binding domain that specifically binds human mesothelin, wherein the antigen-binding domain comprises a VH comprising the amino acid sequence set forth in SEQ ID NO: 7 and a VL comprising the amino acid sequence set forth in SEQ ID NO: 8; (ii) a CD8 hinge region; (iii) a CD8 transmembrane domain; (iv) a 4-1BB costimulatory domain; and (v) an intracellular signaling domain comprising a CD3 $\zeta$ activating domain.

In some aspects, the polynucleotide or the set of polynucleotides encodes a CAR, wherein the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a transmembrane domain; (iii) a costimulatory domain; and (iv) an intracellular signaling domain comprising a CD3 $\zeta$ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a transmembrane domain; (iii) a costimulatory domain comprising an amino acid sequence having at least about 70%, at least about 75%, at least about 80%, at least about 85%, at least about 90%, at least about 95%, at least about 96%, at least about 97%, at least about 98%, or at least about 99% sequence identity to SEQ ID NO: 11; and (iv) an intracellular signaling domain comprising a CD3 $\zeta$ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a transmembrane domain; (iii) a costimulatory domain comprising the amino acid sequence set forth in SEQ ID NO: 11; and (iv) an intracellular signaling domain comprising a CD3 $\zeta$ activating domain. In some aspects, the CAR further comprises a hinge region.

In some aspects, the polynucleotide or the set of polynucleotides encodes a CAR, wherein the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a hinge region; (iii) a transmembrane domain; (iv) a costimulatory domain; and (v) an intracellular signaling domain comprising a CD3 $\zeta$ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a hinge region comprising an amino acid sequence having at least about 70%, at least about 75%, at least about 80%, at least about 85%, at least about 90%, at least about 95%, at least about 96%, at least about 97%, at least about 98%, or at least about 99% sequence identity to SEQ ID NO: 9; (iii) a transmembrane domain; (iv) a costimulatory domain; and (v) an intracellular signaling domain comprising a CD3 $\zeta$ activating domain. In some aspects, the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a hinge region comprising the amino acid sequence set forth in SEQ ID NO: 9; (iii) a transmembrane domain; (iv) a costimulatory domain; and (v) an intracellular signaling domain comprising a CD3 $\zeta$ activating domain.

In some aspects, the polynucleotide or the set of polynucleotides encodes a CAR, wherein the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a CD8 hinge region; (iii) a CD8 transmembrane domain; (iv) a 4-1BB costimulatory domain; and (v) an intracellular signaling domain comprising a CD3 $\zeta$ activating domain.

In some aspects, the polynucleotide or the set of polynucleotides encodes a CAR, wherein the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a hinge region comprising an amino acid sequence having at least about 90%, at least about 95%, at least about 96%, at least about 98%, at least about 98%, or at least about 99% sequence identity to the sequence set forth in SEQ ID NO: 9; (iii) a transmembrane domain comprising an amino acid sequence having at least about 90%, at least about 95%, at least about 96%, at least about 98%, at least about 98%, or at least about 99% sequence identity to the sequence set forth in SEQ ID NO: 10; (iv) a costimulatory domain comprising an amino acid sequence having at least about 90%, at least about 95%, at least about 96%, at least about 98%, at least about 98%, or at least about 99% sequence identity to the sequence set forth in SEQ ID NO: 11; and (v) an intracellular signaling domain comprising an amino acid sequence having at least about 90%, at least about 95%, at least about 96%, at least about 98%, at least about 98%, or at least about 99% sequence identity to the sequence set forth in SEQ ID NO: 12.

In some aspects, the polynucleotide or the set of polynucleotides encodes a CAR, wherein the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a hinge region comprising the amino acid sequence set forth in SEQ ID NO: 9; (iii) a transmembrane domain comprising the amino acid sequence set forth in SEQ ID NO: 10; (iv) a costimulatory domain comprising the amino acid sequence set forth in SEQ ID NO: 11; and (v) an intracellular signaling domain comprising the amino acid sequence set forth in SEQ ID NO: 12.

In some aspects, the polynucleotide of the set of polynucleotides encodes a CAR, wherein the CAR comprises (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a CD8 hinge region; (iii) a CD8 transmembrane domain; (iv) a 4-1BB costimulatory domain; and (v) an intracellular signaling domain comprising a CD3 $\zeta$ activating domain.

In some aspects, the polynucleotide of the set of polynucleotides encodes a CAR, wherein the CAR comprises (i) an antigen-binding domain that specifically binds human mesothelin, wherein the antigen-binding domain comprises a VH comprising an amino acid sequence having at least about 90%, at least about 95%, at least about 96%, at least about 98%, at least about 98%, or at least about 99% sequence identity to the amino acid sequence set forth in SEQ ID NO: 47, and a VL comprising an amino acid sequence having at least about 90%, at least about 95%, at least about 96%, at least about 98%, at least about 98%, or at least about 99% sequence identity to the amino acid sequence set forth in SEQ ID NO: 48; (ii) a hinge region comprising an amino acid sequence having at least about 90%, at least about 95%, at least about 96%, at least about 98%, at least about 98%, or at least about 99% sequence identity to SEQ ID NO: 9; (iii) a transmembrane domain comprising an amino acid sequence having at least about 90%, at least about 95%, at least about 96%, at least about 98%, at least about 98%, or at least about 99% sequence identity to the sequence set forth in SEQ ID NO: 10; (iv) a costimulatory domain comprising an amino acid sequence having at least about 90%, at least about 95%, at least about 96%, at least about 98%, at least about 98%, or at least about 99% sequence identity to the sequence set forth in SEQ ID NO: 11; and (v) an intracellular signaling domain comprising an amino acid sequence having at least about 90%, at least about 95%, at least about 96%, at least about 98%, at least about 98%, or at least about 99% sequence identity to the sequence set forth in SEQ ID NO: 12.

In some aspects, the polynucleotide of the set of polynucleotides encodes a CAR, wherein the CAR comprises (i) an antigen-binding domain that specifically binds human mesothelin, wherein the CAR comprises a VH comprising the amino acid sequence set forth in SEQ ID NO: 47 and a VL comprising the amino acid sequence set forth in SEQ ID NO: 48; (ii) a hinge region comprising the amino acid sequence set forth in SEQ ID NO: 9; (iii) a transmembrane domain comprising the amino acid sequence set forth in SEQ ID NO: 10; (iv) a costimulatory domain comprising the amino acid sequence set forth in SEQ ID NO: 11; and (v) an intracellular signaling domain comprising the amino acid sequence set forth in SEQ ID NO: 12.

In some aspects, the polynucleotide of the set of polynucleotides encodes a CAR, wherein the CAR comprises (i) an antigen-binding domain that specifically binds human mesothelin, wherein the antigen-binding domain comprises a VH comprising the amino acid sequence set forth in SEQ ID NO: 47 and a VL comprising the amino acid sequence set forth in SEQ ID NO: 48; (ii) a CD8 hinge region; (iii) a CD8 transmembrane domain; (iv) a 4-1BB costimulatory domain; and (v) an intracellular signaling domain comprising a CD3 $\zeta$ activating domain.

Some aspects of the present disclosure are directed to a polynucleotide or a set of polynucleotides encoding an armor protein, *i.e.,* an armor protein disclosed herein. In some aspects, the polynucleotide or the set of polynucleotides encodes an armor protein, wherein the armor protein comprises an IL-2R$\beta$ polypeptide (e.g., a recombinant IL-2R$\beta$ polypeptide) (e.g., wherein the armor comprises a recombinant IL-2R$\beta$ polypeptide and polynucleotide or set of polynucleotides does not express IL-15R$\alpha$ polypeptide tethered to an IL-15 polypeptide). In some aspects, the polynucleotide or the set of polynucleotides encodes an armor protein, which comprises an amino acid sequence having at least about 70%, at least about 75%, at least about 80%, at least about 85%, at least about 90%, at least about 95%, at least about 96%, at least about 97%, at least about 98%, at least about 99% sequence identity to the amino acid sequence set forth in SEQ ID NO: 15. In some aspects, the polynucleotide or the set of polynucleotides encodes an armor protein that comprises the amino acid sequence set forth in SEQ ID NO: 15. In some aspects, the polynucleotide or the set of polynucleotides encodes an armor protein, wherein the armor protein comprises (i) a signal peptide and (ii) an IL-2R$\beta$ polypeptide, wherein the signal peptide is cleaved post translation. In some aspects, the armor protein comprises an IL-2R$\beta$ polypeptide, and the armor protein does not comprise an IL-15R$\alpha$ polypeptide or a construct comprising an IL-15R$\alpha$ polypeptide tethered to an IL-15 polypeptide.

In some aspects, the polynucleotide or the set of polynucleotides encodes (i) an IL-2R$\beta$ polypeptide, disclosed herein, and (ii) does not encode an IL-15R$\alpha$ polypeptide or a construct comprising an IL-15R$\alpha$ polypeptide tethered to an IL-15 polypeptide.

In some aspects, the polynucleotide or the set of polynucleotides encodes (i) an IL-2R$\beta$ polypeptide, disclosed herein, and (ii) an IL-15R$\alpha$ polypeptide disclosed herein or a construct comprising an IL-15R$\alpha$ polypeptide tethered to an IL-15 polypeptide disclosed herein.

In some aspects, the polynucleotide or the set of polynucleotides encodes an armor protein comprising an IL-15R$\alpha$ polypeptide. In some aspects, the polynucleotide or the set of polynucleotides encodes an armor protein, comprising an IL-15R$\alpha$ polypeptide tethered to an IL-15 polypeptide. In some aspects, the polynucleotide or the set of polynucleotides encodes an armor protein comprising (i) an IL-2R$\beta$ polypeptide and (ii) an IL-15R$\alpha$ polypeptide tethered to an IL-15 polypeptide.

In some aspects, the polynucleotide or the set of polynucleotides encodes an armor protein comprising an IL-15Rα polypeptide, wherein the IL-15Rα polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 17. In some aspects, the IL-15Rα polypeptide comprises the amino acid sequence set forth in SEQ ID NO: 17. In some aspects, the polynucleotide or the set of polynucleotides encodes a signal peptide immediately upstream of the sequence encoding the IL-15Rα polypeptide.

In some aspects, the polynucleotide or the set of polynucleotides encodes an armor protein comprising an IL-15 polypeptide, wherein the IL-15 polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 16. In some aspects, the IL-15 polypeptide comprises the amino acid sequence set forth in SEQ ID NO: 16. In some aspects, the polynucleotide or the set of polynucleotides encodes a signal peptide immediately upstream of the sequence encoding the IL-15 polypeptide.

In some aspects, a single polynucleotide of the disclosure encodes an IL-15Rα polypeptide tethered to an IL-15 polypeptide by a linker. In some aspects, the linker comprises one or more peptide bonds. In some aspects, the linker is a Gly-Ser linker. In some aspects, the linker comprises one or more repeats of the sequence GGGS, GGGS (SEQ ID NO: 19), or a combination thereof. In some aspects, a single polynucleotide of the disclosure encodes an IL-15Rα polypeptide tethered to an IL-15 polypeptide by a linker, wherein the linker comprises an amino acid sequence selected from SEQ ID NOs: 20-25.

In some aspects, a single polynucleotide of the disclosure encodes (i) an IL-2Rβ polypeptide; (ii) a linker; and (iii) an IL-15Rα polypeptide tethered to an IL-15 polypeptide. In some aspects, the single polynucleotide encodes (i) an IL-2Rβ polypeptide; (ii) a cleavable linker; and (iii) an IL-15Rα polypeptide tethered to an IL-15 polypeptide. In some aspects, the cleavable linker comprises a P2A sequence. In some aspects the linker comprises an amino acid sequence having at least about 80%, at least about 85%, at least about 90%, at least about 95%, at least about 96%, at least about 97%, at least about 98%, or at least about 99% sequence identity to the amino acid sequence set forth in SEQ ID NO: 18. In some aspects, the a single polynucleotide of the disclosure encodes (i) an IL-2Rβ polypeptide; (ii) a linker comprising the amino acid sequence set forth in SEQ ID NO: 18; and (iii) an IL-15Rα polypeptide tethered to an IL-15 polypeptide.

In some aspects, the single polynucleotide of the disclosure encodes (i) an IL-2Rβ polypeptide comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 15; (ii) a linker comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 18; and (iii) an IL-15Rα polypeptide tethered to an IL-15 polypeptide; wherein the IL-15Rα polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 17; and wherein the IL-15 polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 16.

In some aspects, the single polynucleotide of the disclosure encodes (i) an IL-2Rβ polypeptide comprising the amino acid sequence set forth in SEQ ID NO: 15; (ii) a linker comprising the amino acid sequence set forth in SEQ ID NO: 18; and (iii) an IL-15Rα polypeptide tethered to an IL-15 polypeptide; wherein the IL-15Rα polypeptide comprises the amino acid sequence set forth in SEQ ID NO: 17; and wherein the IL-15 polypeptide comprises the amino acid sequence set forth in SEQ ID NO: 16.

In some aspects, the (i) CAR and (ii) armor protein are both encoded by a single polynucleotide. In some aspects, (i) the nucleic acid sequence of the single polynucleotide encoding the CAR and (ii) the nucleic acid sequence of the single polynucleotide encoding the armor protein are separated by an IRES. In some aspects, (i) the nucleic acid sequence of the single polynucleotide encoding the CAR and (ii) the nucleic acid sequence of the single polynucleotide encoding the armor protein are expressed under the control of the same promoter sequence. In some aspects, (i) the nucleic acid sequence of the single polynucleotide encoding the CAR is expressed under the control of a first promoter, and (ii) the nucleic acid sequence of the single polynucleotide encoding the armor protein is expressed under the control of a second promoter. In some aspects, the first promoter and the second promoter are the same. In some aspects, the first promoter and the second promoter are different.

Some aspects of the present disclosure are directed to a polynucleotide encoding (A) a CAR, comprising (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region comprising an amino acid sequence having at least about 90%, at least about 95%, at least about 96%, at least about 98%, at least about 98%, or at least about 99% sequence identity to the sequence set forth in SEQ ID NO: 9; (iii) a transmembrane domain comprising an amino acid sequence having at least about 90%, at least about 95%, at least about 96%, at least about 98%, at least about 98%, or at least about 99% sequence identity to the sequence set forth in SEQ ID NO: 10; (iv) a costimulatory domain comprising an amino acid sequence having at least about 90%, at least about 95%, at least about 96%, at least about 98%, at least about 98%, or at least about 99% sequence identity to the sequence set forth in SEQ ID NO: 11; and (v) an intracellular signaling domain comprising an amino acid sequence having at least about 90%, at least about 95%, at least about 96%, at least about 98%, at least about 98%, or at least about 99% sequence identity to the sequence set forth in SEQ ID NO: 12; and (B) an armor protein, comprising (i) an IL-2Rβ polypeptide comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 15; (ii) a linker comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 18; and (iii) an IL-15Rα polypeptide tethered to an IL-15 polypeptide; wherein the IL-15Rα polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 17; and wherein the IL-15 polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 16.

Some aspects of the present disclosure are directed to a polynucleotide encoding (A) a CAR, comprising (i) an antigen-binding domain that specifically binds human mesothelin, wherein the antigen-binding domain comprises a VH comprising an amino acid sequence having at least about 90%, at least about 95%, at least about 96%, at least about 98%, at least about 98%, or at least about 99% sequence identity to the amino acid sequence set forth in SEQ ID NO: 7 and a VL comprising an amino acid sequence having at least about 90%, at least about 95%, at least about 96%, at least about 98%, at least about 98%, or at least about 99% sequence identity to the amino acid sequence set forth in SEQ ID NO: 8; (ii) a hinge region comprising an amino acid sequence having at least about 90%, at least about 95%, at least about 96%, at least about 98%, at least about 98%, or at least about 99% sequence identity to the sequence set forth in SEQ ID NO: 9; (iii) a transmembrane domain comprising an amino acid sequence having at least about 90%, at least about 95%, at least about 96%, at least about 98%, at least about 98%, or at least about 99% sequence identity to the sequence set forth in SEQ ID NO: 10; (iv) a costimulatory domain comprising an amino acid sequence having at least about 90%, at least about 95%, at least about 96%, at least about 98%, at least about 98%, or at least about 99% sequence identity to the sequence set forth in SEQ ID NO: 11; and (v) an intracellular signaling domain comprising an amino acid sequence having at least about 90%, at least about 95%, at least about 96%, at least about 98%, at least about 98%, or at least about 99% sequence identity to the sequence set forth in SEQ ID NO: 12; and (B) an armor protein, comprising (i) an IL-2Rβ polypeptide comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 15; (ii) a linker comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 18; and (iii) an IL-15Rα polypeptide tethered to an IL-15 polypeptide; wherein the IL-15Rα polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 17; and wherein the IL-15 polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 16.

Some aspects of the present disclosure are directed to a polynucleotide encoding (A) a CAR, comprising (i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; (ii) a hinge region comprising amino acid sequence set forth in SEQ ID NO: 9; (iii) a transmembrane domain comprising the amino acid sequence set forth in SEQ ID NO: 10; (iv) a costimulatory domain comprising the amino acid sequence set forth in SEQ ID NO: 11; and (v) an intracellular signaling domain comprising the amino acid sequence set forth in SEQ ID NO: 12; and (B) an armor protein, comprising (i) an IL-2Rβ polypeptide comprising the amino acid sequence set forth in SEQ ID NO: 15; (ii) a linker comprising the amino acid sequence set forth in SEQ ID NO: 18; and (iii) an IL-15Rα polypeptide tethered to an IL-15 polypeptide; wherein the IL-15Rα polypeptide comprises the amino acid sequence set forth in SEQ ID NO: 17; and wherein the IL-15 polypeptide comprises the amino acid sequence set forth in SEQ ID NO: 16.

Some aspects of the present disclosure are directed to a polynucleotide encoding (A) a CAR, comprising (i) an antigen-binding domain that specifically binds human mesothelin, wherein the antigen-binding domain comprises a VH comprising the amino acid sequence set forth in SEQ ID NO: 7 and a VL comprising the amino acid sequence set forth in SEQ ID NO: 8; (ii) a hinge region comprising amino acid sequence set forth in SEQ ID NO: 9; (iii) a transmembrane domain comprising the amino acid sequence set forth in SEQ ID NO: 10; (iv) a costimulatory domain comprising the amino acid sequence set forth in SEQ ID NO: 11; and (v) an intracellular signaling domain comprising the amino acid sequence set forth in SEQ ID NO: 12; and (B) an armor protein, comprising (i) an IL-2Rβ polypeptide comprising the amino acid sequence set forth in SEQ ID NO: 15; (ii) a linker comprising the amino acid sequence set forth in SEQ ID NO: 18; and (iii) an IL-15Rα polypeptide tethered to an IL-15 polypeptide; wherein the IL-15Rα polypeptide comprises the amino acid sequence set forth in SEQ ID NO: 17; and wherein the IL-15 polypeptide comprises the amino acid sequence set forth in SEQ ID NO: 16.

Some aspects of the present disclosure are directed to a polynucleotide encoding (A) a CAR, comprising (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a hinge region comprising an amino acid sequence having at least about 90%, at least about 95%, at least about 96%, at least about 98%, at least about 98%, or at least about 99% sequence identity to the sequence set forth in SEQ ID NO: 9; (iii) a transmembrane domain comprising an amino acid sequence having at least about 90%, at least about 95%, at least about 96%, at least about 98%, at least about 98%, or at least about 99% sequence identity to the sequence set forth in SEQ ID NO: 10; (iv) a costimulatory domain comprising an amino acid sequence having at least about 90%, at least about 95%, at least about 96%, at least about 98%, at least about 98%, or at least about 99% sequence identity to the sequence set forth in SEQ ID NO: 11; and (v) an intracellular signaling domain comprising an amino acid sequence having at least about 90%, at least about 95%, at least about 96%, at least about 98%, at least about 98%, or at least about 99% sequence identity to the sequence set forth in SEQ ID NO: 12; and (B) an armor protein, comprising (i) an IL-2Rβ polypeptide comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 15; (ii) a linker comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 18; and (iii) an IL-15Rα polypeptide tethered to an IL-15 polypeptide; wherein the IL-15Rα polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 17; and wherein the IL-15 polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 16.

Some aspects of the present disclosure are directed to a polynucleotide encoding (A) a CAR, comprising (i) an antigen-binding domain that specifically binds human mesothelin, wherein the antigen-binding domain comprises a VH comprising an amino acid sequence having at least about 90%, at least about 95%, at least about 96%, at least about 98%, at least about 98%, or at least about 99% sequence identity to the amino acid sequence set forth in SEQ ID NO: 47 and a VL comprising an amino acid sequence having at least about 90%, at least about 95%, at least about 96%, at least about 98%, at least about 98%, or at least about 99% sequence identity to the amino acid sequence set forth in SEQ ID NO: 48; (ii) a hinge region comprising an amino acid sequence having at least about 90%, at least about 95%, at least about 96%, at least about 98%, at least about 98%, or at least about 99% sequence identity to the sequence set forth in SEQ ID NO: 9; (iii) a transmembrane domain comprising an amino acid sequence having at least about 90%, at least about 95%, at least about 96%, at least about 98%, at least about 98%, or at least about 99% sequence identity to the sequence set forth in SEQ ID NO: 10; (iv) a costimulatory domain comprising an amino acid sequence having at least about 90%, at least about 95%, at least about 96%, at least about 98%, at least about 98%, or at least about 99% sequence identity to the sequence set forth in SEQ ID NO: 11; and (v) an intracellular signaling domain comprising an amino acid sequence having at least about 90%, at least about 95%, at least about 96%, at least about 98%, at least about 98%, or at least about 99% sequence identity to the sequence set forth in SEQ ID NO: 12; and (B) an armor protein, comprising (i) an IL-2Rβ polypeptide comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 15; (ii) a linker comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 18; and (iii) an IL-15Rα polypeptide tethered to an IL-15 polypeptide; wherein the IL-15Rα polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 17; and wherein the IL-15 polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 16.

Some aspects of the present disclosure are directed to a polynucleotide encoding (A) a CAR, comprising (i) an antigen-binding domain that specifically binds human mesothelin, wherein the antigen-binding domain comprises a VH-CDR1 comprising the amino acid sequence set forth in SEQ ID NO: 41, a VH-CDR2 comprising the amino acid sequence set forth in SEQ ID NO: 42, a VH-CDR3 comprising the amino acid sequence set forth in SEQ ID NO: 43, a VL-CDR1 comprising the amino acid sequence set forth in SEQ ID NO: 44, a VL-CDR2 comprising the amino acid sequence set forth in SEQ ID NO: 45, and a VL-CDR3 comprising the amino acid sequence set forth in SEQ ID NO: 46; (ii) a hinge region comprising amino acid sequence set forth in SEQ ID NO: 9; (iii) a transmembrane domain comprising the amino acid sequence set forth in SEQ ID NO: 10; (iv) a costimulatory domain comprising the amino acid sequence set forth in SEQ ID NO: 11; and (v) an intracellular signaling domain comprising the amino acid sequence set forth in SEQ ID NO: 12; and (B) an armor protein, comprising (i) an IL-2Rβ polypeptide comprising the amino acid sequence set forth in SEQ ID NO: 15; (ii) a linker comprising the amino acid sequence set forth in SEQ ID NO: 18; and (iii) an IL-15Rα polypeptide tethered to an IL-15 polypeptide; wherein the IL-15Rα polypeptide comprises the amino acid sequence set forth in SEQ ID NO: 17; and wherein the IL-15 polypeptide comprises the amino acid sequence set forth in SEQ ID NO: 16.

Some aspects of the present disclosure are directed to a polynucleotide encoding (A) a CAR, comprising (i) an antigen-binding domain that specifically binds human mesothelin, wherein the antigen-binding domain comprises a VH comprising the amino acid sequence set forth in SEQ ID NO: 47 and a VL comprising the amino acid sequence set forth in SEQ ID NO: 48; (ii) a hinge region comprising amino acid sequence set forth in SEQ ID NO: 9; (iii) a transmembrane domain comprising the amino acid sequence set forth in SEQ ID NO: 10; (iv) a costimulatory domain comprising the amino acid sequence set forth in SEQ ID NO: 11; and (v) an intracellular signaling domain comprising the amino acid sequence set forth in SEQ ID NO: 12; and (B) an armor protein, comprising (i) an IL-2Rβ polypeptide comprising the amino acid sequence set forth in SEQ ID NO: 15; (ii) a linker comprising the amino acid sequence set forth in SEQ ID NO: 18; and (iii) an IL-15Rα polypeptide tethered to an IL-15 polypeptide; wherein the IL-15Rα polypeptide comprises the amino acid sequence set forth in SEQ ID NO: 17; and wherein the IL-15 polypeptide comprises the amino acid sequence set forth in SEQ ID NO: 16.

In some aspects, the polynucleotide or the set of polynucleotides further comprises a suicide gene disclosed herein. In some aspects, the suicide gene encodes viral thymidine kinase, cytosine deaminases, intracellular antibody against antioxidative enzymes (AOEs), bacterial nitroreductase, caspase and Dnase.

Some aspects of the present disclosure are directed to a vector comprising a polynucleotide or set of polynucleotides disclosed herein. In some aspects, the present disclosure is directed to a vector or a set of vectors comprising a polynucleotide encoding a CAR, as described herein. In some aspects, the set of vectors comprises a first vector and a second vector, wherein the first vector comprises a nucleic acid sequence encoding a CAR disclosed herein, and the second vector comprises a nucleic acid sequence encoding an armoring protein disclosed herein. In some aspects, the present disclosure is directed to a vector comprising (i) a nucleic acid sequence encoding a CAR disclosed herein and (ii) a nucleic acid sequence encoding an armor protein disclosed herein.

Any vector can be suitable for the present disclosure. In some aspects, the vector is a viral vector. In some aspects, the vector is a retroviral vector, a DNA vector, a murine leukemia virus vector, an SFG vector, a plasmid, a RNA vector, an adenoviral vector, a baculoviral vector, an Epstein Barr viral vector, a papovaviral vector, a vaccinia viral vector, a herpes simplex viral vector, an adenovirus associated vector (AAV), a lentiviral vector, or any combination thereof.

### II.C. Engineered Cells

Some aspects of the present disclosure are directed to an engineered cell comprising (i) a polynucleotide encoding a CAR disclosed herein, (ii) a CAR disclosed herein, (iii) a polynucleotide encoding an armor protein disclosed herein, (iv) an armor protein disclosed herein or (v) any combination of (i)-(iv). In some aspects, the engineered cell is an immune cell. In some aspects, the engineered cell is an innate lymphoid cell. In some aspects, the engineered cell is selected from a T cell, an NK cell, a B cell, or any combination thereof. In some aspects, the T cell is a $\gamma\delta$ T cell. In some aspects, the engineered cell is a V$\delta$1 T cell. In some aspects, the engineered cell is a V$\delta$2 T cell. In some aspects, the T cell is an $\alpha\beta$ T cell. In some aspects, the engineered cell is a tumor infiltrating lymphocyte (TIL).

The engineered cell can be obtained from any source prior to engineering the cell. In some aspects, the cell prior to engineering, *e.g.*, the innate lymphoid cell, is obtained from a human subject. In some aspects, the cell prior to engineering, *e.g.*, the innate lymphoid cell, is obtained from a healthy human subject. In some aspects, the cell prior to engineering, *e.g.*, the innate lymphoid cell, is obtained from a human subject afflicted with a disease or condition. In some aspects, the disease or condition involved activation of an immune response in the subject. In some aspects, the disease or condition comprises a cancer. In some aspects, the cell prior to engineering, *e.g.*, the innate lymphoid cell, is obtained from a tumor sample from a human subject. In some aspects, the cell prior to engineering, *e.g.*, the innate lymphoid cell, is obtained from peripheral blood from a human subject. In some aspects, the cell prior to engineering, *e.g.*, the innate lymphoid cell, is obtained from a skin sample obtained from a human subject. In some aspects, the cell prior to engineering, *e.g.*, the innate lymphoid cell, is obtained from a gut tissue sample obtained from the subject.

In some aspects, the engineered cell comprises a T cell, wherein the T cell comprises a polynucleotide encoding a CAR disclosed herein, *e.g.,* an anti-mesothelin CAR. In some aspects, the engineered cell comprises a T cell, wherein the T cell comprises (i) a polynucleotide encoding a CAR disclosed herein, *e.g.,* an anti-mesothelin CAR; and (ii) a polynucleotide encoding an armor protein disclosed herein.

In some aspects, the engineered cell comprises a $\gamma\delta$ T cell, wherein the $\gamma\delta$ T cell comprises a polynucleotide encoding a CAR disclosed herein, *e.g.,* an anti-mesothelin CAR. In some aspects, the engineered cell comprises a $\gamma\delta$ T cell, wherein the $\gamma\delta$ T cell comprises (i) a polynucleotide encoding a CAR disclosed herein, *e.g.,* an anti-mesothelin CAR; and (ii) a polynucleotide encoding an armor protein disclosed herein.

In some aspects, the engineered cell comprises a V$\delta$1 T cell, wherein the V$\delta$1 T cell comprises a polynucleotide encoding a CAR disclosed herein, *e.g.,* an anti-mesothelin CAR. In some aspects, the engineered cell comprises a V$\delta$1 T cell, wherein the V$\delta$1 T cell comprises (i) a polynucleotide encoding a CAR disclosed herein, *e.g.,* an anti-mesothelin CAR; and (ii) a polynucleotide encoding an armor protein disclosed herein.

In some aspects, the engineered cell comprises a V$\delta$2 T cell, wherein the V$\delta$2 T cell comprises a polynucleotide encoding a CAR disclosed herein, *e.g.,* an anti-mesothelin CAR. In some aspects, the engineered cell comprises a V$\delta$2 T cell, wherein the V$\delta$2 T cell comprises (i) a polynucleotide encoding a CAR disclosed herein, *e.g.,* an anti-mesothelin CAR; and (ii) a polynucleotide encoding an armor protein disclosed herein.

In some aspects, the engineered cell comprises a NK cell, wherein the NK cell comprises a polynucleotide encoding a CAR disclosed herein, *e.g.,* an anti-mesothelin CAR. In some aspects, the engineered cell comprises a NK cell, wherein the NK cell comprises (i) a polynucleotide encoding a CAR disclosed herein, *e.g.,* an anti-mesothelin CAR; and (ii) a polynucleotide encoding an armor protein disclosed herein.

In some aspects, the engineered cell is derived from an induced pluripotent stem cell (iPSC). In some aspects, the iPSC is differentiated into a cell that expresses one or more markers of an innate lymphoid cell, *e.g.*, a T cell *(e.g.,* a $\gamma\delta$ T cell) or an NK cell. In some aspects, the iPSC is transfected with (i) a polynucleotide encoding a CAR disclosed herein, *e.g.,* an anti-mesothelin CAR; and/or (ii) a polynucleotide encoding an armor protein disclosed herein prior to differentiation into a cell that expresses one or more markers of an innate lymphoid cell, *e.g.*, a T cell *(e.g.,* a $\gamma\delta$ T cell) or an NK cell. In some aspects, the iPSC is transfected with (i) a polynucleotide encoding a CAR disclosed herein, *e.g.,* an anti-mesothelin CAR; and/or (ii) a polynucleotide encoding an armor protein disclosed herein after differentiation into a cell that expresses one or more markers of an innate lymphoid cell, *e.g.*, a T cell *(e.g.,* a $\gamma\delta$ T cell) or an NK cell. In some aspects, the iPSC is transfected with (i) a polynucleotide encoding a CAR disclosed herein, *e.g.,* an anti-mesothelin CAR; and/or (ii) a polynucleotide encoding an armor protein disclosed herein during differentiation into a cell that expresses one or more markers of an innate lymphoid cell, *e.g.,* a T cell *(e.g.,* a $\gamma\delta$ T cell) or an NK cell.

According to a further aspect of the invention there is provided an isolated cell population, the population comprising a plurality of engineered innate lymphoid cells as described herein. Some aspects of the present disclosure are directed to a population of cells comprising a plurality of engineered innate lymphoid cells as described herein.

In some embodiments, engineered innate lymphoid cells may represent greater than 10%, greater than 20%, greater than 30%, greater than 40%, greater than 50%, greater than 60%, greater than 70%, greater than 80% or greater than 90% of the total number of cells in the isolated cell population. In some aspects, at least about 10%, at least about 20%, at least about 30%, at least about 40%, at least about 50%, at least about 60%, at least about 70%, at least about 80%, at least about 90%, at least about 95%, at least about 96%, at least about 97%, at least about 98%, or at least about 99% of the cells in the population of cells are engineered innate lymphoid cells, as disclosed herein.

## II.D. Pharmaceutical Compositions

According to a further aspect of the invention there is provided a pharmaceutical composition comprising the engineered innate lymphoid cell as described herein, in combination with one or more pharmaceutically or physiologically acceptable carriers, diluents, or excipients. Such compositions may include buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (*e.g.,* aluminium hydroxide); and preservatives. Cryopreservation solutions which may be used in the pharmaceutical compositions of the invention include, for example, DMSO. Compositions can be formulated, *e.g.,* for intravenous administration.

## III. Methods of the Disclosure

According to a further aspect of the invention there is provided a method of treating a patient in need thereof using the engineered innate lymphoid cell or the pharmaceutical composition as described herein. Some aspects of the present disclosure are directed to methods of treating a disease or condition in a subject in need thereof, comprising administering to the subject engineered innate lymphoid cell disclosed herein.

According to a further aspect of the invention there is provided use of the engineered innate lymphoid cell or the pharmaceutical composition as described herein for treating a condition in a subject.

According to a further aspect of the invention there is provided the engineered innate lymphoid cell or the pharmaceutical composition as described herein for use in therapy.

The engineered innate lymphoid cell or the pharmaceutical composition may be useful in the treatment of cancer. "Cancer," as used herein, refers to the abnormal growth or division of cells. Generally, the growth and/or life span of a cancer cell exceeds, and is not coordinated with, that of the normal cells and tissues around it. Cancers may be benign, pre-malignant or malignant. Cancer occurs in a variety of cells and tissues, including the oral cavity (e.g. mouth, tongue, pharynx, etc.), digestive system (e.g. oesophagus, stomach, small intestine, colon, rectum, liver, bile duct, gall bladder, pancreas, etc.), respiratory system (e.g. larynx, lung, bronchus, etc.), bones, joints, skin (e.g. basal cell, squamous cell, meningioma, etc.), breast, genital system, (*e.g.* uterus, ovary, prostate, testis, etc.), urinary system (*e.g.* bladder, kidney, ureter, etc.), eye, nervous system (*e.g.* brain, etc.), endocrine system (*e.g.* thyroid, etc.), and haematopoietic system (e.g. lymphoma, myeloma, leukaemia, acute lymphocytic leukaemia, chronic lymphocytic leukaemia, acute myeloid leukaemia, chronic myeloid leukaemia, etc.).

In some aspects, the disease or condition comprises a cancer, *e.g.,* the subject is afflicted with a cancer. In some aspects, the cancer comprises a solid tumor type of cancer. In some aspects, the cancer comprises a lung cancer (*e.g.,* small cell lung cancer (SCLC), non-small cell lung cancer (NSCLC)), lung adenocarcinoma, mesothelioma, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemia, acute myeloid leukemia, chronic myeloid leukemia, acute lymphoblastic leukemia, chronic lymphocytic leukemia, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, or any combination thereof. In some aspects, the cancer is locally advanced. In some aspects, the cancer is metastatic. In some aspects, the cancer is refractory. In some aspects, the cancer is relapsed. In some aspects, the cancer is refractory or relapsed following one or more prior anti-cancer therapy. In some aspects, the one or more prior anti-cancer therapy comprises a standard of care therapy.

In some aspects, the compositions disclosed herein are administered in combination with an additional anti-cancer therapy. In some aspects, the additional anti-cancer therapy comprises a chemotherapy, an immunotherapy, a radiotherapy, a surgery, or any combination thereof. In some aspects, the additional anti-cancer therapy comprises a chemotherapy. In some aspects, the additional anti-cancer therapy comprises an immune-checkpoint inhibitor. In some aspects, the additional anti-cancer therapy comprises a PD-1 antagonist, a PD-L1 antagonist, a CTLA-4 antagonist, a LAG-3 antagonist, a GITR antagonist, or any combination thereof. In some aspects, the anti-cancer therapy comprises an antibody or antigen-binding portion thereof the specifically binds and inhibits PD-1. In some aspects, the anti-cancer therapy comprises an antibody or antigen-binding portion thereof the specifically binds and inhibits PD-L1.

In some aspects, the method further comprises pretreating the subject prior to administering the population of immune cells. In some aspects, the subject is administered a chemotherapy prior to administering the population of immune cells. In some aspects, the subject is administered an immuno-depleting chemotherapy prior to administering the population of immune cells. In some aspects, the immuno-depleting chemotherapy comprises cyclophosphamide, fludarabine, or both.

In some aspects, the method comprises administering to the subject (i) an engineered innate lymphoid cell disclosed herein and (ii) a cytokine. In some aspects, the cytokine comprises IL-2, an analog thereof, a variant thereof, or a fragment thereof.

In some aspects, the cells of the present disclosure are administered to a subject at a dose of at least about $1 \times 10^6$ cells, at least about $2 \times 10^6$ cells, at least about $3 \times 10^6$ cells, at least about $4 \times 10^6$ cells, at least about $5 \times 10^6$ cells, $1 \times 10^7$ cells, at least about $2 \times 10^7$ cells, at least about $3 \times 10^7$ cells, at least about $4 \times 10^7$ cells, at least about $5 \times 10^7$ cells, $1 \times 10^8$ cells, at least about $2 \times 10^8$ cells, at least about $3 \times 10^8$ cells, at least about $4 \times 10^8$ cells, at least about $5 \times 10^8$ cells, $1 \times 10^9$ cells, at least about $2 \times 10^9$ cells, at least about $3 \times 10^9$ cells, at least about $4 \times 10^9$ cells, or at least about $5 \times 10^9$ cells.

The practice of the present disclosure will employ, unless otherwise indicated, conventional techniques of cell biology, cell culture, molecular biology, transgenic biology, microbiology, recombinant DNA, and immunology, which are within the skill of the art. Such techniques are explained fully in the literature. *See,* for example, Sambrook et al., ed. (1989) Molecular Cloning A Laboratory Manual (2nd ed.; Cold Spring Harbor Laboratory Press); Sambrook et al., ed. (1992) Molecular Cloning: A Laboratory Manual, (Cold Springs Harbor Laboratory, NY); D. N. Glover ed., (1985) DNA Cloning, Volumes I and II; Gait, ed. (1984) Oligonucleotide Synthesis; Mullis et al. U.S. Pat. No. 4,683,195; Hames and Higgins, eds. (1984) Nucleic Acid Hybridization; Hames and Higgins, eds. (1984) Transcription And Translation; Freshney (1987) Culture Of Animal Cells (Alan R. Liss, Inc.); Immobilized Cells And Enzymes (IRL Press) (1986); Perbal (1984) A Practical Guide To Molecular Cloning; the treatise, Methods In Enzymology (Academic Press, Inc., N.Y.); Miller and Calos eds. (1987) Gene Transfer Vectors For Mammalian Cells, (Cold Spring Harbor Laboratory); Wu et al., eds., Methods In Enzymology, Vols. 154 and 155; Mayer and Walker, eds. (1987) Immunochemical Methods In Cell And Molecular Biology (Academic Press, London); Weir and Blackwell, eds., (1986) Handbook Of Experimental Immunology, Volumes I-IV; Manipulating the Mouse Embryo, Cold Spring Harbor Laboratory Press, Cold Spring Harbor, N.Y., (1986)); Crooke, Antisense drug Technology: Principles, Strategies and Applications, 2nd Ed. CRC Press (2007) and in Ausubel et al. (1989) Current Protocols in Molecular Biology (John Wiley and Sons, Baltimore, Md.).

All of the references cited above, as well as all references cited herein, are incorporated herein by reference in their entireties.

The following examples are offered by way of illustration and not by way of limitation.

## EXAMPLES

## Materials and Methods

### Cell culture

#### *Retroviral transduction*

Expanding $\gamma\delta$ T-cells were transduced with retroviral vectors in Retronectin coated (20 mg/mL) cell expansion bags (PermaLife bags, OriGen Biomedical) at defined multiplicity of infection (MOI). MOI refers to the number of infectious particles (measured by flow cytometry) that were added per cell during transduction. Viral vectors were diluted in CTS OpTmizer medium. Transduction efficiency was determined using flow cytometry after three days post-transduction at regular intervals.

### Flow Cytometry

Immunophenotyping was performed using a BD FACSLyricTM flow cytometry instruments. Dead cells were excluded using LIVE/DEADTM Fixable Aqua Dead Cell Stain kit (InvitrogenFlow data is gated on live, single, Pan $\gamma\delta$+, V$\delta$1+, CAR+ events.

### Transduction with gammaretroviral vector encoding anti-MSLN chimeric antigen receptor

Cells were expanded and transduced with a $\gamma$-retroviral vector encoding for Mesothelin (MSLN) targeting chimeric antigen receptor constructs in the presence of RetroNectin (20 $\mu$g/mL). Viral vector was mixed with immune cells diluted in CTS OpTmizer overnight at 37°C. Transduction efficiency was determined by flow cytometry four to nine days post-transduction.

### Thawing Cryopreserved Cell Products

Frozen cryovials were thawed in a 37°C water bath and added to pre-warmed OpTmizer + 2.5% allogeneic plasma. Cells were spun down at 300g for 7 minutes, counted and viability assessed, and then resuspended at 2x106 cells per mL for phenotyping and downstream assays.

Survival Assay

Cells were cultured in 96 well plates for 13 days in the absence of IL-15, with duplicate wells from each condition being harvested on days 3, 7, 10 and 13. Surviving Pan- γδ+, CAR+ cells were quantified by flow cytometry using a BD FACS Lyric.

Repeated Antigen Stimulation Assay

Drug product was thawed and co-cultured with A549-MLSN-fluc-gfp cells (in the absence or presence of 1ng/mL of IL-15) at an E:T ratio of 2:1 in 24 well plates (300,000 effectors to 150,000 targets) in an Incucyte to track GFP fluorescence over time, as a surrogate for tumor cell lysis. On days 2 and 9 of culture cells were fed with fresh media plus or minus 1ng/mL of IL-15, replacing 50% of the well volume by careful aspiration after centrifugation. On days 4 and 11 of culture cells were fed with fresh media containing targets (150,000 per well) plus or minus 1ng/mL of IL-15, replacing 50% of the well volume by careful aspiration after centrifugation. On day 7 all wells were harvested, counted and re-seeded at an E:T of 2:1 as on day 0.

*In vivo* studies

Study 1 (FIG. 16): . NSG mice aged 6-8 weeks were implanted with 1x106 A549-MSLN-fluc-GFP cells via intravenous (IV) injection. On day 19 post-implantation, mice were injected with vehicle (n=7, Cryostor CS5, group 1), YP218 γδ CAR T cells at 10 million, 3 million or 1 million total cells per mouse (n=7, groups 2-4), or P4 γδ CAR T cells at 10 million total cells per mouse. From day 19 mice were additionally dosed daily with 1ug human IL-15 via IP injection. Mice were imaged twice per week using an IVIS camera following the intraperitoneal (IP) injection of 150 mg/kg d-Luciferin.

Study 2 (FIG. 20): NSG mice aged 6-8 weeks were implanted with 1x106 A549-MSLN-fluc-GFP cells via intravenous (IV) injection. On day 19 post-implantation, mice were injected with vehicle (n=7, Cryostor CS5, group 1), P4 unarmored γδ CAR T cells at 10 million total cells per mouse (n=7, group 2), P4 α.15.β γδ CAR T cells at 10 million total cells per mouse (n=7, group 3) or P4 β γδ CAR T cells at 10 million total cells per mouse (n=7, group 4). Mice were imaged twice per week using an IVIS camera following the intraperitoneal (IP) injection of 150 mg/kg d-Luciferin. Mice in group 4 were dosed with 1μg IL-15 daily via IP injection.

**EXAMPLE 1**

Blood derived γδ T cells transduced with the YP218 anti-mesothelin CAR construct and non-engineered blood-derived Vδ1 cells ("GDX012") were examined for functionality against a solid tumour target, OVCAR3 cells. Cryopreserved CAR-modified blood Vδ1 T-cells were thawed and immediately cultured with target cells in a 5:1, 2.5:1, 1:1, 1:1:2.5 and 1:5 E:T ratio for 18-24h in medium and without cytokine supplementation. Specific cell lysis was detected via a luciferase-based fluorescence cell counting assay (FIG. 1A). These data demonstrate that genetically engineering GDX012 to express a mesothelin-CAR significantly enhanced the inherent killing capacity of Vδ1 T cells against OVCAR3 cells.

Blood derived γδ T cells transduced with the YP218 anti-mesothelin CAR construct (n=1) were cultured in medium alone or IL-15 supplemented medium (10ng/ml) at a 2:1 E:T ratio with Hela cells. On days 7, 14 and 21, cells were harvested, and viable cell counts were performed using the NC250 cell counter (FIG. 1B). These data demonstrate that that genetically engineering GDX012 to express a mesothelin-CAR significantly enhanced the inherent killing capacity of Vδ1 T cells against Hela tumour targets, and that IL-15 is required in addition to CAR signaling for the survival and proliferation of mesothelin CAR-modified blood Vδ1 T-cells.

As a proof of concept to target solid tumours, GDX012 was genetically engineered with a YP218 anti-mesothelin CAR construct (FIG. 2). Mesothelin was chosen as a proof-of-concept solid tumour target as this protein is overexpressed across various cancer indications and mesothelin is an established target in various solid tumour models and in clinical trials. There is very low expression of mesothelin by healthy tissues making it an ideal solid tumour target. The anti-MSLN Vδ1 product was functionally tested to understand phenotype, cytotoxicity, proliferation and in *in vivo* activity to demonstrate solid tumour targeting and establish a framework for future gene engineering products.

GDX012, unlike skin resident γδ T cells show modest inherent capacity to target solid tumour targets but can be improved through gene engineering. GDX012 (n=3) and skin resident γδ T cells (n=3) were examined for functionality against a mesothelin+ target cell line, Hela cells. Cryopreserved GDX012 and skin resident γδ T cells were thawed and immediately cultured with target cells in a 5:1, 2.5:1, 1.25:1, E:T ratio for 18-24h in medium and without cytokine supplementation. Specific cell lysis was detected via a luciferase-based fluorescence cell counting assay (FIG. 3A). These data demonstrate that skin resident γδ T cells have inherent killing capacity against solid tumour targets, with >80% lysis of tumour targets at 5:1 E:T ratio, >60% at 2.5 E:T ratio and >50% at 1.25 E:T. Target cell lysis by GDX012 did not exceed >20% lysis across all E:T ratios profiled against Hela cells. Blood derived $\gamma\delta$ T cells transduced with the YP218 anti-mesothelin CAR construct and GDX012 were examined for functionality against OVCAR3 cells. Cryopreserved CAR-modified blood V$\delta$1 T-cells were thawed and immediately cultured with target cells in a 5:1, 2.5:1, 1.25:1, 0.625:1 and 0.3125:1 E:T ratio for 18-24h in medium and without cytokine supplementation. Specific cell lysis was detected via a luciferase-based fluorescence cell counting assay (FIG. 3B). These data demonstrate that genetically engineering GDX012 to express a mesothelin-CAR significantly enhanced the inherent killing capacity of V$\delta$1 T cells against OVCAR3 tumour targets, with >90% lysis of tumour targets at 5:1, >75% at 2.5:1, >50% at 1.1 and >20% at 1:2.5 E:T ratio, while target cell lysis by GDX012 did not exceed 30% across all E:T ratios profiled against OVCAR3 cells.

Mesothelin CAR-modified blood V$\delta$1 T-cells show robust cytotoxicity against HeLa and OVCAR3 cells, and this cytotoxicity was comparable to CAR-modified $\alpha\beta$ T cells. Blood derived $\gamma\delta$ T cells transduced with the YP218 anti-mesothelin CAR construct (n=3) and blood-derived $\alpha\beta$ T cells transduced with the YP218 anti-mesothelin construct (n=1) were examined for functionality against Hela (FIG. 4A) and OVCAR3 (FIG. 4B) tumour cells. Cryopreserved CAR-modified blood V$\delta$1 T-cells were thawed and immediately cultured with target cells in a 5:1, 2.5:1, 1.25:1, 0.625:1 and 0.3125 E:T ratio for 18-24h in medium and without cytokine supplementation. Specific cell lysis was detected via a luciferase-based fluorescence cell counting assay.

Mesothelin CAR-modified blood V$\delta$1 T-cells show robust cytotoxicity against HT29 (FIG. 5A) and A549 (FIG. 5B) modified to over express mesothelin, this cytotoxicity was comparable to CAR-modified $\alpha\beta$ T cells. Blood derived $\gamma\delta$ T cells transduced with the YP218 anti-mesothelin CAR construct (n=2) and blood derived $\alpha\beta$ T cells transduced with the YP218 anti-mesothelin construct (n=1) were examined for functionality against HT29.MSLN. Blood derived $\gamma\delta$ T cells transduced with the YP218 anti-mesothelin CAR construct (n=3) and blood derived $\alpha\beta$ T cells transduced with the YP218 anti-mesothelin construct (n=1) were tested for functionality against and A549.MSLN cells. Cryopreserved CAR-modified blood V$\delta$1 T-cells were thawed and immediately cultured with target cells in a 5:1, 2.5:1, 1.25:1, 0.625:1 and 0.3125 E:T ratio for 18-24h in medium and without cytokine supplementation. Specific cell lysis was detected via a luciferase-based fluorescence cell counting assay.

Repeat antigen stimulation (RAS) assay against solid tumour targets.

Effector cells are thawed and co-cultured in IL-15 supplemented medium (10ng/ml) with solid tumour target cancer cell lines at an E:T ratio of 2:1 (FIG. 6). Day +2: Cultures are replenished with cytokines through a 50% media exchange. Day +4: Targets and cytokines are replenished through a 50% media exchange. Day +7: Cultures are harvested. Effectors are counted and the equivalent number of effectors seeded at D0 are reseeded in IL-15 supplemented medium (10mg/ml) with target cells as an E:T ratio of 2:1. Additional effectors are used for phenotypic analysis by flow cytometry or seeded in 24hr killing assays with target cells. Overall, effector cells are subject to 6 rounds of in-assay target cell killing and 21 days in culture with the final 24hr killing assay demonstrating a 7$^{th}$ round of serial killing.

Mesothelin CAR-modified blood V$\delta$1 T-cells expand and enrich for CAR expression following 6 rounds of tumor challenge with Hela cells. Blood derived $\gamma\delta$ T cells transduced with the YP218 anti-mesothelin CAR construct (n=2) were cultured in IL-15 supplemented medium (10ng/ml) at a 2:1 E:T ratio with Hela cells. On days 7, 14 and 21, cells were harvested, and viable cell counts were performed using the NC250 cell counter. The fold proliferation of effector cells at each time point was calculated by dividing the cell counts from the harvested effector cells by the number of cells seeded 7 days previously. The total or cumulative fold proliferation was calculated at each time point by multiplying the fold proliferation by the previous fold proliferation. Cells expanded 60-fold over the course of 21 days (FIG. 7A). CAR expression was measured on V$\delta$1 T cells on day 0, 7, 14 and 21 by flow cytometry. CAR expressing V$\delta$1 T cells were enriched over the course 21 days increasing from <20% expression to >90% expression at the end of the assay (FIG. 7B).

Mesothelin CAR-modified blood V$\delta$1 T-cells expand and enrich in response to serial killing of OVCAR3 and A549.MSLN cells in a RAS assay. Blood derived $\gamma\delta$ T cells transduced with the YP218 anti-mesothelin CAR construct (n=3) were cultured in IL-15 supplemented medium (10ng/ml) at a 2:1 E:T ratio with OVCAR3 and A549.MSLN cells. On day 7, 14 and 21, cells were harvested, and viable cell counts were performed using the NC250 cell counter. The fold proliferation of effector cells at each time point was calculated by dividing the cell counts from the harvested effector cells by the number of cells seeded 7 days previously. The total (cumulative) fold proliferation was calculated at each time point by multiplying the fold proliferation by the previous fold proliferation. Cells cultured with OVCAR3 cells expanded 20-fold over the course of 21 days, and cells cultured with A549.MSLN cells expanded 400-fold over the course of 21 days (FIG. 8A). CAR expression was measured on V$\delta$1 T cells on day 0, 7, 14 and 21 by flow cytometry. CAR expressing V$\delta$1 T cells cultured with OVCAR3 and A549.MSLN cells were enriched over the course 21 days increasing to >95% expression at day 21 (FIG. 8B).

Blood derived $\gamma\delta$ T cells transduced with the YP218 anti-mesothelin CAR construct (n=2) were cultured in a 2:1 ratio with Hela cells in IL-15 supplemented medium (10ng/ml) for 21 days. On days 7, 14 and 21, fresh target cells were harvested, and viable cell counts were obtained using the NC250 cell counter. Target cells were seeded at $2\times10^5$ cells/well and allowed to adhere for 4h. Effectors were harvested, counted and cultured with adherent target cells in a 5:1, 2.5:1, 1.25:1, 0.625:1 and 0.3125 E:T ratio for 18-24h in medium and without cytokine supplementation. Specific cell lysis was detected via a luciferase-based fluorescence cell counting assay and data is representative of 7 rounds of serial killing (FIG. 9A). Flow cytometric analysis of live CAR$^+$ $\gamma\delta$ T cells after day 7, 14 and 21 of RAS assay against Hela cells was conducted. Phenotypic analysis of V$\delta$1 T cells demonstrated that NKG2D, CD56, NKp30, NKG2C and PD1 expression was upregulated from day 0 to 21, while CD27, representing effector state was downregulated by V$\delta$1 T cells from day 0 to 21 (FIG. 9B).

Mesothelin CAR-modified blood V$\delta$1 T-cells remain cytotoxic even after 21 days of RAS against OVCAR3 and A549.MSLN cells. Blood derived $\gamma\delta$ T cells transduced with the YP218 anti-mesothelin CAR construct (n=3) were cultured in a 2:1 ratio with OVCAR3 and A549.MSLN cells in IL-15 supplemented medium (10ng/ml) for 21 days. On day 7, 14 and 21, fresh target cells were harvested, and viable cell counts were obtained using the NC250 cell counter. Target cells were seeded at $2\times10^5$ cells/well and allowed to adhere for 4h. Effectors were harvested, counted and cultured with adherent target cells in a 5:1, 2.5:1, 1.25:1, 0.625:1 and 0.3125 E:T ratio for 18-24h in medium and without cytokine supplementation. Specific cell lysis was detected via a luciferase-based fluorescence cell counting assay (FIGs. 10A-10F).

Mesothelin CAR-modified blood V$\delta$1 T-cells have greater cytotoxicity against MSLN expressing A549 (FIG. 11B) than unmodified A549 (FIG. 11A) tumour cells. Blood derived $\gamma\delta$ T cells transduced with the YP218 anti-mesothelin CAR construct (n=4) and blood derived $\alpha\beta$ T cells transduced with the YP218 anti-mesothelin construct (n=1) were tested for functionality against A549 tumour cells and A549 tumour cells modified to overexpress MSLN. Cryopreserved CAR-modified blood V$\delta$1 T-cells were thawed and immediately cultured with target cells in a 5:1, 2.5:1, 1.25:1, 0.625:1 and 0.3125 E:T ratio for 18-24h in medium and without cytokine supplementation. Specific cell lysis was detected via a luciferase-based fluorescence cell counting assay.

Skin derived Vd1+ $\gamma\delta$ T cells can be efficiently transduced with Meso-CAR. Skin resident cells were defrosted and immediately processed via MACS selection to remove $\alpha\beta$ T cells. Resultant negatively selected $\gamma\delta$ T cells were then transduced with vector encoding either P4 or YP218 anti-mesothelin CAR constructs. Transduced cells were then expanded for 14 days in the presence of IL-15 (80ng/ml) and IL21 (11.25ng/ml) before harvest and cryopreservation. The $\gamma\delta$ T cell fold rate of expansion was recorded at D14 of expansion culture (FIG. 12A). The percentage of cells positive for each mesothelin specific CAR was measured at D14 via flow cytometry (FIG. 12B).

Skin derived $\gamma\delta$ T cells are cytotoxic against mesothelin positive target cells, HeLa cell line (FIGs. 13A-13B). Skin resident $\gamma\delta$ T cells were transduced and expanded as stated in FIGs. 12A-12B. Cryopreserved cells were then defrosted and immediately tested for functionality vs the mesothelin$^+$ Hela cell line. Cells were cultured at varying effector to target ratios for 17-20h with no cytokine supplementation. Specific cell lysis was detected using a CellTitreGLO cell counting assay. White circles denote untransduced skin resident $\gamma\delta$ t cells, and grey dots denote mesothelin-specific CAR transduced skin resident $\gamma\delta$ t cells.

Skin derived $\gamma\delta$ T cells are cytotoxic against mesothelin positive target cells, A459 (FIG. 14). Skin resident $\gamma\delta$ T cells transduced with the YP218 anti-mesothelin CAR construct were tested for functionality versus a mesothelin$^+$A549 target cell line. Cryopreserved YP218+ expanded skin resident $\gamma\delta$ T cells were defrosted and immediately placed in culture with target cells for 17-20h with no cytokine supplementation. Specific cell lysis was detected via a luciferase-based fluorescence cell counting assay.

Mesothelin CAR-modified skin V$\delta$1 T-cells are cytotoxic against HT29.MSLN (FIG. 15A) and A549.MSLN (FIG. 15B) tumor cells. Skin resident $\gamma\delta$ and $\alpha\beta$ T cells transduced with the YP218 anti mesothelin CAR construct (n=1) were tested for functionality versus mesothelin$^+$ A549 target cell line. Cryopreserved YP218+ expanded skin resident $\gamma\delta$ T cells were defrosted and immediately placed in culture with target HT29.MSLN and A549.MSLN cells for 18-24h with no cytokine supplementation. Specific cell lysis was detected via a luciferase-based fluorescence cell counting assay.

## EXAMPLE 2

Blood-derived Vd1+ $\gamma\delta$ are transduced with a transgene encoding an anti-mesothelin CAR comprising a P4 antigen-binding domain (*e.g.*, wherein the antigen-binding domain comprises a sequence of any of SEQ ID NOs: 1 - 8). Optionally the cells can also be transduced to express a 4-1BB and CD3 zeta domain. Optionally, the cells can also be transduced to express a CD8 transmembrane domain and hinge region. The cells are further modified to express (a) an IL-15R-beta or (b)(i) IL-15R-alpha tethered with an IL-15 or variant thereof and (ii) IL-15R-beta. CAR-expressing cells will be assayed for target cell binding and lysis.

## EXAMPLE 3

Blood derived $\gamma\delta$ T cells transduced with either a YP218 or P4 anti-mesothelin CAR constructs (comprising the anti-mesothelin binder, a CD8 hinge region, a CD8 transmembrane domain, a 4-1BB costimularoty domain, and a CD3$\zeta$ instracellular signaling domain) were assessed for functionality in a disseminated A549 tumour model. FIG. 16A shows that $\gamma\delta$ T cells transduced with either binder demonstrate effective tumor control. FIG. 16B shows that anti-MSLN CAR expressing $\gamma\delta$ T cells were able to control tumor growth when dosed with as few as 1 million total cells. On day 18 post drug product administration, all mice were culled and bone marrow aspirates from both femurs, spleens, lungs and peripheral blood (collected in lithium-heparin tubes) were harvested. FIG. 16C-16F shows that $\gamma\delta$ T cells expressing either anti-MSLN binder persisted in assessed niches to equivalent levels.

## EXAMPLE 4

In order to enhance the survival of V$\delta$1 $\gamma\delta$ CAR T cells in either the presence of low IL-15 concentrations, or in its absence, a set of novel armored constructs were designed and evaluated utilising both YP218 and P4 binders. Multicistronic gammaretroviral vectors were generated in which the chimeric antigen receptor (CAR) encoding sequences were fused in frame with IL-15 receptor chain components. FIG. 17A shows the schematics of the armoring strategies used to co-express the CAR and armoring moiety(s). FIG. 17B shows domain structure of the conventional armored CAR encoding gammaretroviral vectors. Cells transduced with IL-15R$\beta$ alone will be referred to as "$\beta$" while cells transduced with IL-15R$\beta$ + IL-15R$\alpha$ tethered to IL-15 will be referred to as "$\alpha$.15.$\beta$".

## EXAMPLE 5

For each of the P4 and YP218 binders armored with $\alpha$.15.$\beta$, two different codon optimized sequences generated using different optimization algorithms were explored to determine whether this might impact the frequency and relative protein expression of each component of the armoring cassette. Drug product was generated from 2 leukapheresis donors, donor 1 in squares, and donor 2 in circles utilising each of 4 different vectors (P4 $\alpha$.15.$\beta$ codon sequence 1, P4 $\alpha$.15.$\beta$ codon sequence 2, YP218 $\alpha$.15.$\beta$ codon sequence 1, YP218 $\alpha$.15.$\beta$ codon sequence 2). Drug product transduced with vector encoding P4 $\alpha$.15.$\beta$ codon sequence 1 and 2 had approximately equivalent expression of each armoring component, both in terms of percentage expression (FIG. 18A) and relative level of protein (FIG. 18B). In contrast, drug product transduced with YP218 $\alpha$.15.$\beta$ codon sequence 2 demonstrated a greater proportion of cells expressing each armoring component than YP218 $\alpha$.15.$\beta$ codon sequence 1 (FIG. 18C). The relative level of protein expression was also increased for both IL15R$\beta$ and IL15R$\alpha$ with codon sequence 2 as compared to 1 (FIG. 18D).

Significant enhanced cytotoxicity by the tethered IL-15 and IL-15R $\beta$ co-expressing cells was immediately observed when targeting antigen-expressing tumour targets (FIG. 18E).

## EXAMPLE 6

Response to repeated antigen challenge was explored for $\beta$ armored binder P compared its unarmoured counterpart in the presence of 1ng/mL IL-15. Tumour confluence was studied by measuring GFP fluorescence over time. The $\beta$ armored binder P4 construct demonstrated superior tumour control over the course of the 14-day repeated challenge assay. These results demonstrate that enhanced sensitivity to exogenous IL-15 provided by upregulation of IL-2R$\beta$ results in $\gamma\delta$ CAR T drug product with superior functionality (FIG. 19A). This empowered functionality, significantly correlates greater proliferation potential in response to low levels of exogenous IL-15 (1ng/mL) as compared to the unarmored counterpart, reflecting enhanced sensitivity to soluble exogenous IL-15 (FIG. 19B). Enrichment of CAR modified cells increases throughout the assay demonstrating that the CAR transduced cells represent the main population driving tumour control (FIG. 19C).

Blood derived $\gamma\delta$ T cells transduced with P4 $\alpha$.15.$\beta$ and P4 $\beta$ were assessed for functionality in a disseminated A549 tumour model (FIG. 20A). Data in FIG. 20B shows that $\alpha$.15.$\beta$ armored $\gamma\delta$ CAR T cells provide greater tumor control than unarmored counterparts in the absence of IL-15 support, with P4 $\alpha$.15.$\beta$ treated mice having greater than 10-fold lower BLI signal on average than mice dosed with unarmored cells at study end (day 19 post-dosing). In line with the in vitro experiments, in the presence of IL-15, $\beta$ armored $\gamma\delta$ CAR T cells demonstrate an initial faster tumour eliminataion kinetics, but overall comparable tumour control to the $\alpha$.15.$\beta$ armored (non IL-15 supported) $\gamma\delta$ CAR T treated arm.

## EXAMPLE 7

IL-15R$\alpha$ tethered to IL-15 and IL-15R $\beta$ co-expressing mesothelin-targeting blood-derived CAR $\gamma\delta$ T cells survived 14 days post-cryopreservation whereas unarmoured construct rapidly declines. This was true when investigating among two different Meso-CAR binders used, YP218 CAR (FIG. 21A) and P4 CAR (FIG. 21B). FIG. 21C demonstrates that IL-15R$\alpha$ tethered to IL-15 and IL-15R $\beta$ chain armoured Meso-CAR T cells can superiorly control tumour over the course of a repeated antigen stimulation assay in the absence of exogenous IL-15. Also, this functionality is observed regardless the CAR binder used (YP218 or P4). In addition, the enhanced tumour control exhibit by the IL-15R$\alpha$ tethered to IL-15 and IL-15R $\beta$ chain armoured CAR T cells do not necessarily correlate with proliferation potential. Instead, proliferation potential seems to be a functionality driven by the CAR molecule and specific to the binder used. Proliferation was observed when YP218 binder was used but not with P4 binder (FIG. 21D).

**LIST OF EMBODIMENTS**

1. An engineered innate lymphoid cell comprising a heterologous targeting construct specific for mesothelin.

2. The engineered innate lymphoid cell of Embodiment 1, wherein the lymphoid cell is a natural killer (NK) cell or a $\gamma\delta$ T cell.

3. The engineered innate lymphoid cell of Embodiment 2, wherein the $\gamma\delta$ T cell is a Vd1+ $\gamma\delta$ cell.

4. The engineered innate lymphoid cell of any of Embodiments 1 to 3, wherein the heterologous targeting construct is a chimeric antigen receptor (CAR).

5. The engineered innate lymphoid cell of Embodiment 4, wherein the CAR is a full-length CAR or a non-signaling CAR.

6. The engineered innate lymphoid cell of Embodiment 4 or Embodiment 5, wherein the CAR is an armoured CAR.

7. A pharmaceutical composition comprising the engineered innate lymphoid cell of any of Embodiments 1 to 6, in combination with one or more pharmaceutically or physiologically acceptable carriers, diluents, or excipients.

8. A method of treating a patient in need thereof using the engineered innate lymphoid cell of any of Embodiments 1 to 6 or the pharmaceutical composition of Embodiment 7.

9. Use of the engineered innate lymphoid cell of any of Embodiments 1 to 6 or the pharmaceutical composition of Embodiment 7 for treating a condition in a subject.

10. The engineered innate lymphoid cell of any of Embodiments 1 to 6 or the pharmaceutical composition of Embodiment 7 for use in therapy.

11. A method of making the engineered innate lymphoid cell of any of Embodiments 1 to 6.

12. An isolated cell population, the population comprising a plurality of engineered innate lymphoid cells of any one of Embodiments 1 to 6.

13. The engineered innate lymphoid cell of any of Embodiments 1-6, wherein the cell expresses: (a) IL-15R-beta or (b) (i) IL-15R-alpha tethered to an IL-15 or variant thereof and (ii) IL-15R-beta.

14. The engineered innate lymphoid cell of Embodiment 13, wherein the cell expresses IL-15R-beta.

15. The engineered innate lymphoid cell of Embodiment 13, wherein the cell expresses (i) IL-15R-alpha tethered to an IL-15 or variant thereof and (ii) IL-15R-beta.

16. A polynucleotide comprising a nucleotide sequence encoding a chimeric antigen receptor (CAR), wherein the CAR comprises an antigen-binding domain that specifically binds human mesothelin, wherein the antigen-binding domain comprises a variable heavy (VH) domain and a variable light (VL) domain, wherein the VH comprises a VH-complementarity determining region 3 (CDR3) comprising the amino acid sequence set forth in SEQ ID NO: 3.

17. The polynucleotide of Embodiment 16, wherein the CAR further comprises a VH-CDR2 comprising the amino acid sequence set forth in SEQ ID NO: 2.

18. The polynucleotide of Embodiment 16 or 17, wherein the CAR further comprises a VH-CDR3 comprising the amino acid sequence set forth in SEQ ID NO: 3.

19. The polynucleotide of any one of Embodiments 16 to 18, wherein the CAR further comprises a VL-CDR1 comprising the amino acid sequence set forth in SEQ ID NO: 4.

20. The polynucleotide of any one of Embodiments 16 to 19, wherein the CAR further comprises a VL-CDR2 comprising the amino acid sequence set forth in SEQ ID NO: 5.

21. The polynucleotide of any one of Embodiments 16 to 20, wherein the CAR further comprises a VL-CDR3 comprising the amino acid sequence set forth in SEQ ID NO: 6.

22. The polynucleotide of any one of Embodiments 16 to 21, wherein the CAR comprises a VH-CDR1 comprising the amino acid sequence set forth in SEQ ID NO: 1; a VH-CDR2 comprising the amino acid sequence set forth in SEQ ID NO: 2; a VH-CDR3 comprising the amino acid sequence set forth in SEQ ID NO: 3; a VL-CDR1 comprising the amino acid sequence set forth in SEQ ID NO: 4; a VL-CDR2 comprising the amino acid sequence set forth in SEQ ID NO: 5; and a VL-CDR3 comprising the amino acid sequence set forth in SEQ ID NO: 6.

23. The polynucleotide of any one of Embodiments 16 to 22, wherein the VH comprises an amino acid sequence having at least about 75%, at least about 80%, at least about 85%, at least about 90%, at least about 95%, at least about 96%, at least about 97%, at least about 98%, or at least about 99% sequence identity with the amino acid sequence set forth in SEQ ID NO: 7.

24. The polynucleotide of any one of Embodiments 16 to 23, wherein the VH comprises the amino acid sequence set forth in SEQ ID NO: 7.

25. The polynucleotide of any one of Embodiments 16 to 24, wherein the VL comprises an amino acid sequence having at least about 75%, at least about 80%, at least about 85%, at least about 90%, at least about 95%, at least about 96%, at least about 97%, at least about 98%, or at least about 99% sequence identity with the amino acid sequence set forth in SEQ ID NO: 8.

26. The polynucleotide of any one of Embodiments 16 to 25, wherein the VL comprises the amino acid sequence set forth in SEQ ID NO: 8.

27. The polynucleotide of any one of Embodiments 16 to 26, wherein the VH comprises the amino acid sequence set forth in SEQ ID NO: 7, and wherein the VL comprises the amino acid sequence set forth in SEQ ID NO: 8.

28. The polynucleotide of any one of Embodiments 16 to 27, wherein the CAR further comprises (i) a hinge region, (ii) a transmembrane domain, (iii) a costimulatory domain, (iv) an intracellular signaling domain, or (v) any combination thereof.

29. The polynucleotide of any one of Embodiments 16 to 28, wherein the CAR further comprises (i) a hinge region, (ii) a transmembrane domain, (iii) a costimulatory domain, and (iv) an intracellular signaling domain.

30. The polynucleotide of Embodiment 28 or 29, wherein the hinge region comprises a hinge region derived from CD8, CD28, or an immunoglobulin.

31. The polynucleotide of Embodiment 30, wherein the immunoglobulin is selected from the group consisting of IgA1, IgA2, IgG1, IgG2, IgG3, IgG4, IgD, IgE, IgM, and any combination thereof.

32. The polynucleotide of any one of Embodiments 28 to 31, wherein the hinge region is derived from a CD8 hinge region.

33. The polynucleotide of any one of Embodiments 28 to 32, wherein the hinge region comprises an amino acid sequence having at least about 75%, at least about 80%, at least about 85%, at least about 90%, at least about 95%, at least about 96%, at least about 97%, at least about 98%, or at least about 99% sequence identity with the amino acid sequence set forth in SEQ ID NO: 9.

34. The polynucleotide of any one of Embodiments 28 to 33, wherein the hinge region comprises the amino acid sequence set forth in SEQ ID NO: 9.

35. The polynucleotide of any one of Embodiments 28 to 34, wherein the transmembrane domain comprises a transmembrane domain derived from CD8, KIRDS2, OX40, CD2, CD4, CD28, CD45, PD1, CD152, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKG2D, NKG2C, CD19, or any combination thereof.

36. The polynucleotide of any one of Embodiments 28 to 35, wherein the transmembrane domain is derived from a CD8 transmembrane domain.

37. The polynucleotide of any one of Embodiments 28 to 36, wherein the transmembrane domain comprises an amino acid sequence having at least about 75%, at least about 80%, at least about 85%, at least about 90%, at least about 95%, at least about 96%, at least about 97%, at least about 98%, or at least about 99% sequence identity with the amino acid sequence set forth in SEQ ID NO: 10.

38. The polynucleotide of any one of Embodiments 28 to 37, wherein the transmembrane domain comprises the amino acid sequence set forth in SEQ ID NO: 10.

39. The polynucleotide of any one of Embodiments 28 to 38, wherein the costimulatory domain comprises a costimulatory domain derived from a costimulatory domain of 4-1BB/CD137, interleukin-2 receptor (IL-2R), interleukin-12 receptor (IL-12R), IL-7, IL-21, IL-23, IL-15, CD2, CD3, CD4, CD7, CD8, CD27, CD28, CD30, CD40, ICOS, lymphocyte function-associated antigen-1 (LFA-1), LIGHT, NKG2C, OX40, DAP10, B7-H3, CD28 deleted for Lck binding (ICA), BTLA, GITR, HVEM, LFA-1, LIGHT, NKG2C, PD-1, TILR2, TILR4, TILR7, TILR9, Fc receptor gamma chain, Fc receptor ε chain, a ligand that specifically binds with CD83, or any combination thereof.

40. The polynucleotide of any one of Embodiments 28 to 39, wherein the costimulatory domain is derived from a 4-1BB costimulatory domain.

41. The polynucleotide of any one of Embodiments 28 to 40, wherein the costimulatory domain comprises an amino acid sequence having at least about 75%, at least about 80%, at least about 85%, at least about 90%, at least about 95%, at least about 96%, at least about 97%, at least about 98%, or at least about 99% sequence identity with the amino acid sequence set forth in SEQ ID NO: 11.

42. The polynucleotide of any one of Embodiments 28 to 41, wherein the costimulatory domain comprises the amino acid sequence set forth in SEQ ID NO: 11.

43. The polynucleotide of any one of Embodiments 28 to 42, wherein the intracellular signaling domain comprises a CD3 ζ activating domain, a CD3δ activating domain, a CD3ε activating domain, a CD3η activating domain, a CD79A activating domain, a DAP 12 activating domain, a FCER1G activating domain, a DAP10/CD28 activating domain, a ZAP70 activating domain, or any combination thereof.

44. The polynucleotide of any one of Embodiments 28 to 43, wherein the intracellular signaling domain comprises a CD3 ζ activating domain.

45. The polynucleotide of any one of Embodiments 28 to 44, wherein the intracellular signaling domain comprises an amino acid sequence having at least about 75%, at least about 80%, at least about 85%, at least about 90%, at least about 95%, at least about 96%, at least about 97%, at least about 98%, or at least about 99% sequence identity with the amino acid sequence set forth in SEQ ID NO: 12.

46. The polynucleotide of any one of Embodiments 28 to 45, wherein the costimulatory domain comprises the amino acid sequence set forth in SEQ ID NO: 12.

47. The polynucleotide of any one of Embodiments 16 to 46, comprising a nucleotide sequence encoding a chimeric antigen receptor (CAR), wherein the CAR comprises

(a) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; and

(b)(i) a hinge region comprising an amino acid sequence having at least about 90%, at least about 95%, at least about 96%, at least about 98%, at least about 98%, or at least about 99% sequence identity to the sequence set forth in SEQ ID NO: 9;

(b)(ii) a transmembrane domain comprising an amino acid sequence having at least about 90%, at least about 95%, at least about 96%, at least about 98%, at least about 98%, or at least about 99% sequence identity to the sequence set forth in SEQ ID NO: 10;

(b)(iii) a costimulatory domain comprising an amino acid sequence having at least about 90%, at least about 95%, at least about 96%, at least about 98%, at least about 98%, or at least about 99% sequence identity to the sequence set forth in SEQ ID NO: 11;

(b)(iv) an intracellular signaling domain comprising an amino acid sequence having at least about 90%, at least about 95%, at least about 96%, at least about 98%, at least about 98%, or at least about 99% sequence identity to the sequence set forth in SEQ ID NO: 12; or

(b)(v) any combination of (b)(i) to (b)(iv).

48. The polynucleotide of any one of Embodiments 16 to 47, comprising a nucleotide sequence encoding a chimeric antigen receptor (CAR), wherein the CAR comprises

(i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6;

(ii) a hinge region comprising an amino acid sequence having at least about 90%, at least about 95%, at least about 96%, at least about 98%, at least about 98%, or at least about 99% sequence identity to the sequence set forth in SEQ ID NO: 9;

(iii) a transmembrane domain comprising an amino acid sequence having at least about 90%, at least about 95%, at least about 96%, at least about 98%, at least about 98%, or at least about 99% sequence identity to the sequence set forth in SEQ ID NO: 10;

(iv) a costimulatory domain comprising an amino acid sequence having at least about 90%, at least about 95%, at least about 96%, at least about 98%, at least about 98%, or at least about 99% sequence identity to the sequence set forth in SEQ ID NO: 11; and

(v) an intracellular signaling domain comprising an amino acid sequence having at least about 90%, at least about 95%, at least about 96%, at least about 98%, at least about 98%, or at least about 99% sequence identity to the sequence set forth in SEQ ID NO: 12.

49. The polynucleotide of any one of Embodiments 16 to 48, comprising a nucleotide sequence encoding a chimeric antigen receptor (CAR), wherein the CAR comprises

(i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6;

(ii) a hinge region comprising an amino acid sequence having at least about 90%, at least about 95%, at least about 96%, at least about 98%, at least about 98%, or at least about 99% sequence identity to the sequence set forth in SEQ ID NO: 9;

(iii) a transmembrane domain comprising an amino acid sequence having at least about 90%, at least about 95%, at least about 96%, at least about 98%, at least about 98%, or at least about 99% sequence identity to the sequence set forth in SEQ ID NO: 10;

(iv) a costimulatory domain comprising an amino acid sequence having at least about 90%, at least about 95%, at least about 96%, at least about 98%, at least about 98%, or at least about 99% sequence identity to the sequence set forth in SEQ ID NO: 11; and

(v) an intracellular signaling domain comprising an amino acid sequence having at least about 90%, at least about 95%, at least about 96%, at least about 98%, at least about 98%, or at least about 99% sequence identity to the sequence set forth in SEQ ID NO: 12.

50. The polynucleotide of any one of Embodiments 16 to 48, comprising a nucleotide sequence encoding a chimeric antigen receptor (CAR), wherein the CAR comprises

(i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6;

(ii) a hinge region comprising the amino acid sequence set forth in SEQ ID NO: 9;

(iii) a transmembrane domain comprising the amino acid sequence set forth in SEQ ID NO: 10;

(iv) a costimulatory domain comprising the amino acid sequence set forth in SEQ ID NO: 11; and

(v) an intracellular signaling domain comprising the amino acid sequence set forth in SEQ ID NO: 12.

51. The polynucleotide of any one of Embodiments 16 to 50, further comprising a second nucleotide sequence encoding an armor protein.

52. The polynucleotide of Embodiment 51, wherein the armor protein comprises an IL-15R$\beta$ polypeptide, an IL-15R$\alpha$ polypeptide, an IL-2R$\beta$ polypeptide, an IL-15 polypeptide, or any combination thereof.

53. The polynucleotide of Embodiment 51 or 52, wherein the armor protein comprises an IL-2R$\beta$ polypeptide.

54. The polynucleotide of any one of Embodiments 51 to 53, wherein the armor protein comprises an amino acid sequence having at least about 70%, at least about 75%, at least about 80%, at least about 85%, at least about 90%, at least about 95%, at least about 96%, at least about 97%, at least about 98%, at least about 99% sequence identity to the amino acid sequence set forth in SEQ ID NO: 15.

55. The polynucleotide of any one of Embodiments 51 to 54, wherein the armor protein comprises the amino acid sequence set forth in SEQ ID NO: 15.

56. The polynucleotide of any one of Embodiments 51 to 55, wherein the armor protein comprises an IL-2R$\beta$ polypeptide, and wherein the armor protein does not comprise an IL-15R$\alpha$ polypeptide or an IL-15 polypeptide.

57. The polynucleotide of any one of Embodiments 51 to 55, wherein the armor protein comprises (i) an IL-15R$\alpha$ polypeptide or (ii) an IL-15R$\alpha$ polypeptide tethered to an IL-15 polypeptide.

58. The polynucleotide of any one of Embodiments 51 to 55 and 57, wherein the armor protein comprises an IL-15R$\alpha$ polypeptide comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 17.

59. The polynucleotide of any one of Embodiments 51 to 55, 57, and 58, wherein the armor protein comprises an IL-15Rα polypeptide comprising the amino acid sequence set forth in SEQ ID NO: 17.

60. The polynucleotide of any one of Embodiments 51 to 55 and 57 to 59, wherein the armor protein comprises an IL-15 polypeptide.

61. The polynucleotide of any one of Embodiments 51 to 55 and 57 to 60, wherein the armor protein comprises an IL-15 polypeptide comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 16.

62. The polynucleotide of any one of Embodiments 51 to 55 and 57 to 61, wherein the armor protein comprises an IL-15 polypeptide comprising the amino acid sequence set forth in SEQ ID NO: 16.

63. The polynucleotide of any one of Embodiments 51 to 55 and 57 to 62, wherein the armor protein comprises an IL-15Rα polypeptide tethered to an IL-15 polypeptide by a linker.

64. The polynucleotide of Embodiment 63, wherein the linker comprises a peptide bond.

65. The polynucleotide of Embodiment 63 or 64, wherein the linker comprises a Gly-Ser linker.

66. The polynucleotide of any one of Embodiments 63 to 65, wherein the linker comprises an amino acid sequence selected from SEQ ID NOs: 20-25 or any combination thereof.

67. The polynucleotide of any one of Embodiments 51 to 52 and 57 to 60, wherein the armor protein comprises (i) an IL-2Rβ polypeptide; (ii) a cleavable linker; and (iii) an IL-15Rα polypeptide tethered to an IL-15 polypeptide.

68. The polynucleotide of Embodiment 67, wherein the cleavable linker comprises a P2A sequence.

69. The polynucleotide of Embodiment 67 or 68, wherein the cleavable linker comprises an amino acid sequence having at least about 80%, at least about 85%, at least about 90%, at least about 95%, at least about 96%, at least about 97%, at least about 98%, or at least about 99% sequence identity to the amino acid sequence set forth in SEQ ID NO: 18.

70. The polynucleotide of any one of Embodiments 67 to 69, wherein the cleavable linker comprises the amino acid sequence set forth in SEQ ID NO: 18.

71. The polynucleotide of any one of Embodiments 16 to 70, comprising a nucleotide sequence encoding:

(a) a chimeric antigen receptor (CAR), wherein the CAR comprises

(i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6;

(ii) a hinge region comprising the amino acid sequence set forth in SEQ ID NO: 9;

(iii) a transmembrane domain comprising the amino acid sequence set forth in SEQ ID NO: 10;

(iv) a costimulatory domain comprising the amino acid sequence set forth in SEQ ID NO: 11; and

(v) an intracellular signaling domain comprising the amino acid sequence set forth in SEQ ID NO: 12; and

(b) an armor protein, wherein the armor protein comprises an IL-2Rβ polypeptide comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 15.

72. The polynucleotide of any one of Embodiments 16 to 71, comprising a nucleotide sequence encoding:

(a) a chimeric antigen receptor (CAR), wherein the CAR comprises

(i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6;

(ii) a hinge region comprising the amino acid sequence set forth in SEQ ID NO: 9;

(iii) a transmembrane domain comprising the amino acid sequence set forth in SEQ ID NO: 10;

(iv) a costimulatory domain comprising the amino acid sequence set forth in SEQ ID NO: 11; and

(v) an intracellular signaling domain comprising the amino acid sequence set forth in SEQ ID NO: 12; and

(b) an armor protein, wherein the armor protein comprises an IL-2Rβ polypeptide comprising the amino acid sequence set forth in SEQ ID NO: 15.

73. The polynucleotide of Embodiment 71 or 72, which does not comprise a nucleotide sequence encoding an IL-15Rα polypeptide or an IL-15 polypeptide.

74. The polynucleotide of any one of Embodiments 16 to 55 and 57 to 72, comprising a nucleotide sequence encoding:

(a) a chimeric antigen receptor (CAR), wherein the CAR comprises

(i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6;

(ii) a hinge region comprising the amino acid sequence set forth in SEQ ID NO: 9;

(iii) a transmembrane domain comprising the amino acid sequence set forth in SEQ ID NO: 10;

(iv) a costimulatory domain comprising the amino acid sequence set forth in SEQ ID NO: 11; and

(v) an intracellular signaling domain comprising the amino acid sequence set forth in SEQ ID NO: 12; and

(b) an armor protein, wherein the armor protein comprises:

(i) an IL-2Rβ polypeptide comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 15;

(ii) a linker comprising an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 18; and

(iii) an IL-15Rα polypeptide tethered to an IL-15 polypeptide;

wherein:

a. the IL-15Rα polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 17; and

b. the IL-15 polypeptide comprises an amino acid sequence having at least about 70%, at least about 75%, at least about 80%, at least about 85%, at least about 90%, at least about 95%, at least about 96%, at least about 97%, at least about 98%, or at least about 99% sequence identity to the amino acid sequence set forth in SEQ ID NO: 16.

75. The polynucleotide of any one of Embodiments 16 to 55 and 57 to 72, comprising a nucleotide sequence encoding:

(a) a chimeric antigen receptor (CAR), wherein the CAR comprises

(i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6;

(ii) a hinge region comprising the amino acid sequence set forth in SEQ ID NO: 9;

(iii) a transmembrane domain comprising the amino acid sequence set forth in SEQ ID NO: 10;

(iv) a costimulatory domain comprising the amino acid sequence set forth in SEQ ID NO: 11; and

(v) an intracellular signaling domain comprising the amino acid sequence set forth in SEQ ID NO: 12; and

(b) an armor protein, wherein the armor protein comprises:

(i) an IL-2Rβ polypeptide comprising the amino acid sequence set forth in SEQ ID NO: 15;

(ii) a linker comprising the amino acid sequence set forth in SEQ ID NO: 18; and

(iii) an IL-15Rα polypeptide tethered to an IL-15 polypeptide;

wherein:

a. the IL-15Rα polypeptide comprises the amino acid sequence set forth in SEQ ID NO: 17; and

b. the IL-15 polypeptide comprises the amino acid sequence set forth in SEQ ID NO: 16.

76. A vector or a set of vectors comprising the polynucleotide of any one of Embodiments 16 to 75.

77. The vector or the set of vectors of Embodiment 76, comprising one or more promoter.

78. The vector or the set of vectors of Embodiment 76 or 77, which is a retroviral vector, a DNA vector, a murine leukemia virus vector, an SFG vector, a plasmid, a RNA vector, an adenoviral vector, a baculoviral vector, an Epstein Barr viral vector, a papovaviral vector, a vaccinia viral vector, a herpes simplex viral vector, an adenovirus associated vector (AAV), a lentiviral vector, or any combination thereof.

79. A polypeptide encoded by the polynucleotide of any one of Embodiments 16 to 75.

80. A host cell comprising the polynucleotide of any one of Embodiments 16 to 75, the vector or the set of vectors of any one of Embodiments 76 to 78, or the polypeptide of Embodiment 79.

81. The host cell of Embodiment 80, which is an immune cell.

82. The host cell of Embodiment 81, wherein the immune cell is an innate lymphoid cell.

83. The host cell of Embodiment 81 or 82, wherein the immune cell is selected from a T cell, an NK cell, a B cell, or any combination thereof.

84. The host cell of Embodiment 83, wherein the T cell is selected from a $\gamma\delta$ T cell, an $\alpha\beta$ T cell, or any combination thereof.

85. The host cell of Embodiment 83 or 84, wherein the T cell is selected from a V$\delta$1 T cell and a V$\delta$2 T cell.

86. The host cell of any one of Embodiments 80 to 85, which is obtained from a human subject.

87. The host cell of any one of Embodiments 80 to 86, which is obtained from a blood sample obtained from a human subject, a skin sample obtained from a human subject, a tumor sample obtained from a human subject, a gut tissue sample obtained from a human subject, or any combination thereof.

88. The host cell of any one of Embodiments 80 to 86, which is derived from an induced pluripotent stem cell (iPSC).

89. A population of cells comprising the host cell of any one of Embodiments 80 to 88.

90. The population of cells of Embodiment 89, wherein at least about 10%, at least about 20%, at least about 30%, at least about 40%, at least about 50%, at least about 60%, at least about 70%, at least about 80%, at least about 90%, at least about 95%, at least about 96%, at least about 97%, at least about 98%, or at least about 99% of the total number of cells in the population of cells comprises the host cell.

91. A pharmaceutical composition, comprising the polynucleotide of any one of Embodiments 16 to 75, the vector or the set of vectors of any one of Embodiments 76 to 78, the polypeptide of Embodiment 79, the host cell of any one of Embodiments 80 to 88, or the population of cells of Embodiment 89 or 90 and a pharmaceutically acceptable excipient.

92. A method of treating a disease or condition in a subject in need thereof, comprising administering to the subject the polynucleotide of any one of Embodiments 16 to 75, the vector or the set of vectors of any one of Embodiments 76 to 78, the polypeptide of Embodiment 79, the host cell of any one of Embodiments 80 to 88, the population of cells of Embodiment 89 or 90, or the pharmaceutical composition of Embodiment 91.

93. The method of Embodiment 92, wherein the disease or condition comprises a cancer.

94. The method of Embodiment 93, wherein the cancer comprises a lung cancer, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemia, acute myeloid leukemia, chronic myeloid leukemia, acute lymphoblastic leukemia, chronic lymphocytic leukemia, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, or any combination thereof.

95. The method of Embodiment 93 or 94, wherein the cancer comprises small cell lung cancer, non-small cell lung cancer, lung adenocarcinoma, mesothelioma, or any combination thereof.

96. The method of any one of Embodiments 93 to 94, wherein the cancer is locally advanced.

97. The method of any one of Embodiments 93 to 95, wherein the cancer is metastatic.

98. The method of any one of Embodiments 92 to 97, wherein the cancer is refractory or relapsed.

99. The method of any one of Embodiments 92 to 98, further comprising administering an additional anti-cancer agent.

100. The method of Embodiment 99, wherein the additional anti-cancer therapy comprises a chemotherapy, an immunotherapy, a radiotherapy, a surgery, or any combination thereof.

101. The method of Embodiment 99 or 100, wherein the additional anti-cancer therapy comprises a chemotherapy.

102. The method of any one of Embodiments 99 to 101, wherein the additional anti-cancer therapy comprises an immune-checkpoint inhibitor.

103. The method of any one of Embodiments 98 to 101, wherein the additional anti-cancer therapy comprises a PD-1 antagonist, a PD-L1 antagonist, a CTLA-4 antagonist, a LAG-3 antagonist, a GITR antagonist, or any combination thereof.

104. The method of any one of Embodiments 99 to 103, wherein the anti-cancer therapy comprises an antibody or antigen-binding portion thereof the specifically binds and inhibits PD-1.

105. The method of any one of Embodiments 99 to 104, wherein the anti-cancer therapy comprises an antibody or antigen-binding portion thereof the specifically binds and inhibits PD-L1.

**Claims**

1. An engineered γδ T cell for use in treating a subject afflicted with a cancer, wherein the engineered γδ T cell comprises:

   (i) a chimeric antigen receptor (CAR), wherein the CAR comprises an antigen-binding domain that specifically binds mesothelin, wherein the antigen-binding domain comprises a variable heavy (VH) domain and a variable light (VL) domain, wherein the VH comprises a VH-complementarity determining region 1 (VH-CDR1) comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3; and wherein the VL comprises a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6; and
   (ii) a transgene encoding an armor protein comprising an IL-15Rβ polypeptide.

2. The engineered γδ T cell for use of claim 1, which is a Vd1+ γδ cell.

3. The engineered γδ T cell for use of any one of claim 1 or 2, wherein:

   (i) the VH comprises an amino acid sequence having at least 75% sequence identity with the amino acid sequence set forth in SEQ ID NO: 7,
   (ii) the VL comprises an amino acid sequence having at least 75% sequence identity with the amino acid sequence set forth in SEQ ID NO: 8, or
   (iii) both (i) and (ii).

4. The engineered γδ T cell for use of any one of claims 1 to 3, wherein:

   (i) the VH comprises the amino acid sequence set forth in SEQ ID NO: 7;
   (ii) the VL comprises the amino acid sequence set forth in SEQ ID NO: 8; or
   (iii) both (i) and (ii).

5. The engineered γδ T cell for use of any one of claims 1 to 4, wherein the CAR further comprises (i) a hinge region, (ii) a transmembrane domain, (iii) a costimulatory domain, (iv) an intracellular signaling domain, or (v) any combination thereof.

**6.** The engineered γδ T cell for use of claim 5, wherein:

(i) the hinge region comprises a hinge region derived from CD8, CD28, or an immunoglobulin;
(ii) the transmembrane domain comprises a transmembrane domain derived from CD8, KIRDS2, OX40, CD2, CD4, CD28, CD45, PD1, CD152, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKG2D, NKG2C, CD19, or any combination thereof;
(iii) the costimulatory domain comprises a costimulatory domain derived from a costimulatory domain of 4-1BB/CD137, interleukin-2 receptor (IL-2R), interleukin-12 receptor (IL-12R), IL-7, IL-21, IL-23, IL-15, CD2, CD3, CD4, CD7, CD8, CD27, CD28, CD30, CD40, ICOS, lymphocyte function-associated antigen-1 (LFA-1), LIGHT, NKG2C, OX40, DAP10, B7-H3, CD28 deleted for Lck binding (ICA), BTLA, GITR, HVEM, LFA-1, LIGHT, NKG2C, PD-1, TILR2, TILR4, TILR7, TILR9, Fc receptor gamma chain, Fc receptor ε chain, a ligand that specifically binds with CD83, or any combination thereof;
(iv) the intracellular signalling domain comprises a CD3 ζ activating domain, a CD3δ activating domain, a CD3ε activating domain, a CD3η activating domain, a CD79A activating domain, a DAP 12 activating domain, a FCER1G activating domain, a DAP10/CD28 activating domain, a ZAP70 activating domain, or any combination thereof; or
(v) any combination of (i)-(iv).

**7.** The engineered γδ T cell for use of claim 5 or 6, wherein

(i) the hinge region comprises an immunoglobulin selected from the group consisting of IgA1, IgA2, IgG1, IgG2, IgG3, IgG4, IgD, IgE, IgM, and any combination thereof, or the hinge region is derived from a CD8 hinge region;
(ii) the transmembrane domain is derived from a CD8 transmembrane domain;
(iii) the costimulatory domain is derived from a 4-1BB costimulatory domain;
(iv) the intracellular signalling domain comprises a CD3 ζ activating domain; or
(v) any combination of (i)-(iv).

**8.** The engineered γδ T cell for use of any one of claims 1 to 7, wherein the CAR comprises:

(i) a hinge region comprising an amino acid sequence having at least 90% sequence identity to the sequence set forth in SEQ ID NO: 9,
(ii) a transmembrane domain comprising an amino acid sequence having at least 90% sequence identity to the sequence set forth in SEQ ID NO: 10,
(iii) a costimulatory domain comprising an amino acid sequence having at least 90% sequence identity to the sequence set forth in SEQ ID NO: 11,
(iv) an intracellular signalling domain comprising an amino acid sequence having at least 90% sequence identity to the sequence set forth in SEQ ID NO: 12, or
(v) any combination of (i) to (iv).

**9.** The engineered γδ T cell for use of any one of claims 1 to 8, wherein the CAR comprises:

(i) a hinge region comprising the amino acid sequence set forth in SEQ ID NO: 9;
(ii) a transmembrane domain comprising the amino acid sequence set forth in SEQ ID NO: 10;
(iii) a costimulatory domain comprising the amino acid sequence set forth in SEQ ID NO: 11; and
(iv) an intracellular signalling domain comprising the amino acid sequence set forth in SEQ ID NO: 12.

**10.** The engineered γδ T cell for use of any one of claims 1 to 9, wherein the armor protein comprises an amino acid sequence having at least 70% sequence identity to the amino acid sequence set forth in SEQ ID NO: 15.

**11.** The engineered γδ T cell for use of any one of claims 1 to 10, wherein the armor protein does not comprise an IL-15Rα polypeptide or an IL-15 polypeptide.

12. The engineered γδ T cell for use of any one of claims 1 to 11, comprising:

(a) a chimeric antigen receptor (CAR), wherein the CAR comprises:

(i) an antigen-binding domain that specifically binds human mesothelin, wherein the antigen-binding domain comprises a VH-CDR1 comprising the amino acid sequence set forth in SEQ ID NO: 1, a VH-CDR2 comprising the amino acid sequence set forth in SEQ ID NO: 2, a VH-CDR3 comprising the amino acid sequence set forth in SEQ ID NO: 3, a VL-CDR1 comprising the amino acid sequence set forth in SEQ ID NO: 4, a VL-CDR2 comprising the amino acid sequence set forth in SEQ ID NO: 5, and a VL-CDR3 comprising the amino acid sequence set forth in SEQ ID NO: 6;
(ii) a hinge region comprising the amino acid sequence set forth in SEQ ID NO: 9;
(iii) a transmembrane domain comprising the amino acid sequence set forth in SEQ ID NO: 10;
(iv) a costimulatory domain comprising the amino acid sequence set forth in SEQ ID NO: 11; and
(v) an intracellular signaling domain comprising the amino acid sequence set forth in SEQ ID NO: 12; and

(b) an armor protein, wherein the armor protein comprises:

(i) an IL-15Rβ polypeptide comprising the amino acid sequence set forth in SEQ ID NO: 15.

13. The engineered γδ T cell for use of any one of claims 1 to 12, wherein the cancer comprises a lung cancer, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemia, acute myeloid leukemia, chronic myeloid leukemia, acute lymphoblastic leukemia, chronic lymphocytic leukemia, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, or any combination thereof.

14. The engineered γδ T cell for use of any one of claims 1 to 13, wherein the cancer comprises small cell lung cancer, non-small cell lung cancer, lung adenocarcinoma, mesothelioma, or any combination thereof.

15. The engineered γδ T cell for use of any one of claims 1 to 14, wherein the cancer is:

(a) locally advanced or metastatic;
(b) refractory or relapsed; or
(c) any combination of (a) and (b).

**Solid Tumor Targeting**
Driven solely by CAR

**Serial Killing Capacity**
Supported by IL-15 supplementation

FIG. 1A

FIG. 1B

EP 4 628 504 A2

# FIG. 2

FIG. 4A                                    FIG. 4B

EP 4 628 504 A2

**HT29-MSLN**
(Colon cancer overexpressing MSLN)

**A549-MSLN**
(Lung cancer overexpressing MSLN)

Anti-MSLN Vδ1
Anti-MSLN αβ

FIG. 5A

FIG. 5B

# FIG. 6

FIG. 7B

FIG. 7A

**Total Cell Fold Expansion**

OVCAR3

A549.MSLN

FIG. 8A

**CAR T cell Enrichment**

OVCAR3

A549.MSLN

FIG. 8B

**Vδ1 frequency maintained**

OVCAR3

A549.MSLN

FIG. 8C

**Cytotoxicity post-assay**

FIG. 9A

**Phenotype post-assay**

FIG. 9B

Anti-MSLN Vδ1

Ovcar D7

Ovcar D14

Ovcar D21

A549 D7

A549 D14

A549 D21

FIG. 10D   FIG. 10E   FIG. 10F

EP 4 628 504 A2

FIG. 11A

FIG. 11B

EP 4 628 504 A2

FIG. 12A

FIG. 12B

P4 Meso Binder

YP218 Meso Binder

FIG. 13A

FIG. 13B

FIG. 14

FIG. 15A

FIG. 15B

EP 4 628 504 A2

FIG. 16A

FIG. 16B

Day post implantation

BLI

Dose 1

- Untreated
- Binder Y
- Binder P

- Untreated
- Binder Y - 10 Million cells
- Binder Y - 3 Million cells
- Binder Y - 1 Million cells

## FIG. 16C

## FIG. 16D

○ Y binder 10M
● P binder 10M

## FIG. 16E

## FIG. 16F

FIG. 17A

Novel methods

Autonomous
IL-15 signaling loop

Increase Sensitivity
towards
exogenous IL-15

α.15.β

β

FIG. 17B

EP 4 628 504 A2

FIG. 18A

FIG. 18B

codon opt #1
codon opt #2

FIG. 18C

FIG. 18D

codon opt #1
codon opt #2

EP 4 628 504 A2

FIG. 18E

FIG. 19A

EP 4 628 504 A2

FIG. 19B

FIG. 19C

# FIG. 20A

A549-meso
implanted I.V

IV, $1\times10^7$
MesoCAR cells

Day  -X          0                                    20+

Termination – BM, spleen, blood, lungs

| Group | Treatment | N | Mouse | Dosing schedule |
|-------|-----------|---|-------|-----------------|
| 1 | Untreated | 7 | | N/A |
| 2 | MesoCAR(1) | | NSG | **Single CAR Dose** |
| 3 | MesoCAR(1).α.15. β | 7 | | **No IL-15 supplementation** |
| 4 | MesoCAR(1).β | 7 | NSG | **Single CAR Dose** **Daily IL-15 supplementation** |

## FIG. 20B

FIG. 21A

FIG. 21B

EP 4 628 504 A2

FIG. 21C

FIG. 21D

EP 4 628 504 A2

## REFERENCES CITED IN THE DESCRIPTION

### Patent documents cited in the description

- GB 2204927 A
- US 63482752
- WO 2007046006 A
- US 4683195 A, Mullis

### Non-patent literature cited in the description

- **JUO, PEI-SHOW**. Concise Dictionary of Biomedicine and Molecular Biology. CRC Press
- The Dictionary of Cell and Molecular Biology. Academic Press, 1999
- Oxford Dictionary Of Biochemistry And Molecular Biology, Revised. Oxford University Press, 2000
- **KABAT et al.** Sequences of Proteins of Immunological Interest. Public Health Service, National Institutes of Health, 1991
- **AL-LAZIKANI et al.** *JMB*, 1997, vol. 273, 927-948
- **GRABSTEIN et al.** *Science*, 1994, vol. 264 (5161), 965-969
- **YANG et al.** *Cancer*, 1995, vol. 76, 687-694
- **CLARK-LEWIS et al.** *PNAS*, 1993, vol. 90, 3574-3577
- **PREUSS et al.** *Hum Gene Ther.*, August 2010, vol. 21 (8), 929-41
- Molecular Cloning A Laboratory Manual. Cold Spring Harbor Laboratory Press, 1989
- Molecular Cloning: A Laboratory Manual. Cold Springs Harbor Laboratory, 1992
- DNA Cloning. 1985, vol. I and II
- Oligonucleotide Synthesis. 1984
- Nucleic Acid Hybridization. 1984
- Transcription And Translation. 1984
- **FRESHNEY**. Culture Of Animal Cells. Alan R. Liss, Inc., 1987
- Immobilized Cells And Enzymes. IRL Press, 1986
- A Practical Guide To Molecular Cloning; the treatise. **PERBAL**. Methods In Enzymology. Academic Press, Inc., 1984
- Gene Transfer Vectors For Mammalian Cells. Cold Spring Harbor Laboratory, 1987
- Methods In Enzymology, vol. 154 and 155
- Immunochemical Methods In Cell And Molecular Biology. Academic Press, 1987
- Handbook Of Experimental Immunology. 1986, vol. I-IV
- Manipulating the Mouse Embryo. Cold Spring Harbor Laboratory Press, 1986
- **CROOKE**. Antisense drug Technology: Principles, Strategies and Applications. CRC Press, 2007
- **AUSUBEL et al.** Current Protocols in Molecular Biology. John Wiley and Sons, 1989